# EUROPEAN PATENT APPLICATION

(11) **EP 1 875 926 A1**
(43) Date of publication of application: **09.01.2008**
(21) Application number: 06746045.1
(22) Date of filing: 27.04.2006
(51) Int. Cl.: A61K 47/20, A61K 31/216, A61K 45/00, A61K 47/22, A61K 47/24, A61K 47/44, A61K 47/46

(54) **STABLE EMULSION COMPOSITION**

(30) Priority: 28.04.2005 JP 2005131807
(71) Applicant: Takeda Pharmaceutical Company Limited, Osaka-shi, Osaka 541-0045 (JP)
(72) Inventor: ASAKAWA, Naoki, c/o TAKEDA PHARMACEUTICAL CO. LTD., Osaka-shi, Osaka 532-8686 (JP); DOEN, Takayuki, c/o TAKEDA PHARMACEUTICAL CO., LTD, Osaka-shi, Osaka 532-8686 (JP)
(74) Representative: Keller, Günter
(86) International application number: PCT/JP2006/309213
(87) International publication number: WO 2006/118329

(57) **Abstract**

The present invention provides an emulsion composition comprising (A) a compound stable in an acidic range, and (B) a buffer, wherein the pH is adjusted from about 3.7 to about 5.5.

## Description

### Technical Field

The present invention relates to an emulsion composition having improved stability.

### Background Art

WO 99/46242 describes that a compound represented by the formula (i): 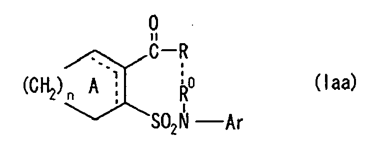 [wherein R represents an aliphatic hydrocarbon group optionally having substituents, an aromatic hydrocarbon group optionally having substituents, a heterocyclic group optionally having substituents, a group represented by the formula: -OR¹ (wherein R¹ represents a hydrogen atom or an aliphatic hydrocarbon group optionally having substituents) or a group represented by the formula: 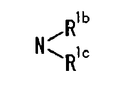 (wherein R^{1b} represents a hydrogen atom or an aliphatic hydrocarbon group optionally having substituents, R^{1c} is same or different from R^{1b}, and is a hydrogen atom or an aliphatic hydrocarbon group optionally having substituents), R⁰ represents a hydrogen atom or an aliphatic hydrocarbon group, or R and R° are joined to form a bond, ring A is a cycloalkene substituted by 1 to 4 substituent(s) selected from (1) an aliphatic hydrocarbon group optionally having substituents, (2) an aromatic hydrocarbon group optionally having substituents, (3) a group represented by the formula: -OR¹ (wherein R¹ represents the same meaning as mentioned above) and (4) a halogen atom, Ar represents an aromatic hydrocarbon group optionally having substituents, a group represented by the formula: 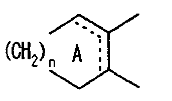 can be a group represented by the formula: 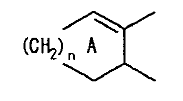 or a group represented by the formula: 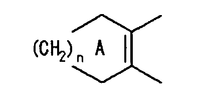 and n represents an integer of 1 to 4], and a compound represented by the formula (ii): 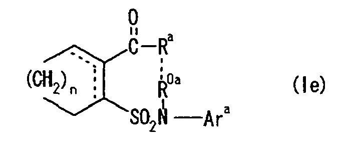 wherein R^{a} represents an aliphatic hydrocarbon group optionally having substituents, an aromatic hydrocarbon group optionally having substituents, a heterocyclic group optionally having substituents, a group represented by the formula -OR^{1a} (wherein R^{1a} represents a hydrogen atom or an aliphatic hydrocarbon group optionally having substituents) or a group represented by the formula: 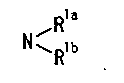 (wherein R^{1a} represents the same meaning as defined above, R^{1b} is same with or different from R^{1a}, a hydrogen atom or an aliphatic hydrocarbon group optionally having substituents), R^{0a} represents a hydrogen atom or an aliphatic hydrocarbon group, or R^{a} and R^{0a} are joined to form a bond, Ar^{a} represents an aromatic hydrocarbon group optionally having substituents, a group represented by the formula: 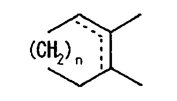 can be a group represented by the formula: 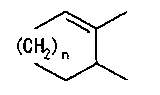 or a group represented by the formula: 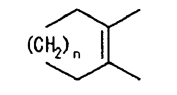 and n represents an integer of 1 to 4], or a salt thereof and a prodrug thereof have nitric oxide (NO) production-inhibiting effect and an inhibitory effect on the production of inflammatory cytokines such as TNF-α, IL-1, IL-6 and the like, and are useful as a prophylactic and therapeutic agent against the diseases including cardiac diseases, autoimmune diseases, inflammatory diseases, central nervous system diseases, infectious diseases, sepsis, septic shock and the like.

This publication also describes that an oily injection can be produced by dissolving, suspending or emulsifying this compound in a vegetable oil or propylene glycol (Patent Document 1).

Also, WO 01/10826 describes that a compound represented by the formula: 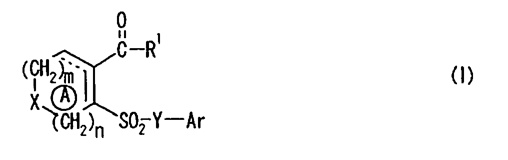 [wherein R¹ represents an aliphatic hydrocarbon group optionally having substituents, an aromatic hydrocarbon group optionally having substituents, a heterocyclic group optionally having substituents, a group represented by the formula: OR^{1a} (wherein R^{1a} represents a hydrogen atom or an aliphatic hydrocarbon group optionally having substituents) or a group represented by the formula: 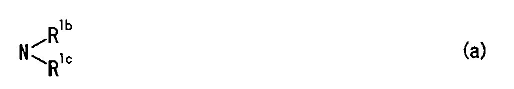 (wherein R^{1b} and R^{1c} are same or different from each other and represent a hydrogen atom or an aliphatic hydrocarbon group optionally having substituents), X represents a methylene group, a nitrogen atom, a sulfur atom, or an oxygen atom, Y represents an optionally substituted methylene group or an optionally substituted nitrogen atom, a ring A is a 5 to 8 membered ring optionally further substituted by 1 to 4 substituent(s) selected from (1) an aliphatic hydrocarbon group optionally having substituents, (2) an aromatic hydrocarbon group optionally having substituents, (3) a group represented by the formula: OR² (wherein R² represents a hydrogen atom or an aliphatic hydrocarbon group optionally having substituents) and (4) a halogen atom, Ar represents an aromatic hydrocarbon group optionally having substituents, a group represented by the formula: 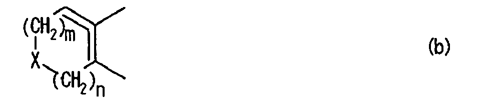 can be a group represented by the formula: 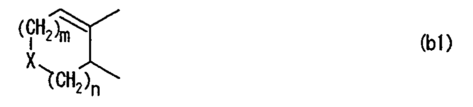 or a group represented by the formula: 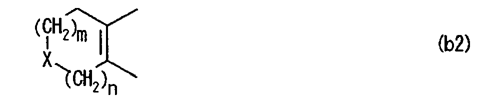 m represents an integer of 0 to 2, n represents an integer of 1 to 3, and sum of m and n is 4 or less. Where X is a methylene group, Y represents an optionally substituted methylene group], or a salt thereof and a prodrug thereof have nitric oxide (NO) production-inhibiting effect and an inhibitory effect on the production of inflammatory cytokines such as TNF-α, IL-1, IL-6 and the like, and are useful as a prophylactic and therapeutic agent against the diseases including cardiac diseases, autoimmune diseases, inflammatory diseases, central nervous system diseases, infectious diseases, sepsis, septic shock and the like (Patent Document 2).
[Patent Document 1] International Patent Publication No. 1999-46242 pamphlet
[Patent Document 2] International Patent Publication No. 2001-10826 pamphlet

### Disclosure of the Invention

The object of the present invention is to provide an emulsion composition containing a compound stable in an acidic range, which is represented by the aforementioned compound having improved stability.

A term stable compound in acidic range according to the specification referred to a group of compounds easily decompose or generate analogs in neutral of above about pH 6 or in basic ranges, and less decompose or generate analogs and highly stable in acidic range (about pH 6 or less). In specific, the compound stable in acidic range is a compound that less decomposes or generates analogs in acidic range when the compound in acidic range and the compound in neutral or in basic ranges are stored in same storing conditions (temperature, humidity, period or the like) and examined their quality. Although these compounds or compositions containing the compounds are stable when stored under acidic range condition, it was difficult to be stored or to be used in neutral or basic range conditions as these are unstable under those ranges. For example, in a case where the aforementioned compound stable under acidic range is made to an emulsion composition, since an emulsion composition is generally stable in a neutral range, a range for the compound to be stable does not match to the range for the emulsion composition to be stable and thus it was difficult to obtain a composition excellent in stability. A stable composition is not obtained due to decomposition of the compound when emulsion composition of neutral range is prepared by emulsifying a compound stable under acidic range, when an emulsion composition of acidic range is prepared, phospholipid or the like contained in the emulsion composition as an emulsifier mainly decomposes at the time of high pressure sterilization by steam or a long term storage, and generates free fatty acid due to the decomposition and lowers pH of the emulsion composition, and thus reduces a stability of the emulsion composition. In addition, problem arises in that a decomposition of the emulsion composition is encouraged as pH is lowered.

Also, Washington and colleagues disclose in 'Advanced Drug Delivery Reviews 20, 131-145, 1996' that a stability of emulsion composition reduces if electrolytic substance is added to the emulsion composition, and so it is known that electrolytic substance such as buffer is not supposed to be added.

The present inventors discovered by carrying out a keen investigation considering the aforementioned problems, that an emulsion composition in which pH is adjusted to about 3.7 to about 5.5 and a compound stable in the acidic range and buffer are included, unexpectedly gives no pH variation at the time of high pressure sterilization by steam or a long term storage and shows an extremely favorable stability, and thus superior efficacy has been provided. On the basis of this finding, further investigation is carried out and the present invention is achieved.

Accordingly, the present invention provides
[1] An emulsion composition containing (A) a compound stable in acidic range and (B) a buffering agent in which pH is adjusted to about 3.7 to about 5.5.
[2] The composition of [1], which further comprises an anionic synthetic phospholipids as an emulsifier.
[3] The composition of any of [1] or [2], wherein (A) the compound stable in acidic range is (a) a compound represented by the formula: 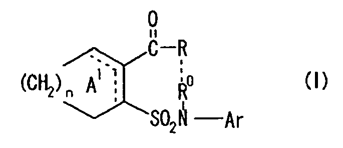 [wherein R represents an aliphatic hydrocarbon group optionally having substituents, an aromatic hydrocarbon group optionally having substituents, a heterocyclic group optionally having substituents, a group represented by the formula: OR¹ (wherein R¹ represents a hydrogen atom or an aliphatic hydrocarbon group optionally having substituents) or a group represented by the formula: 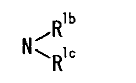 (wherein R^{1b} represents a hydrogen atom or an aliphatic hydrocarbon group optionally having substituents, R^{1c} is, same with or different from R^{1b}, a hydrogen atom or an aliphatic hydrocarbon group optionally having substituents, R⁰ represents a hydrogen atom or an aliphatic hydrocarbon group, or R and R° are joined to form a bond, ring A¹ is a cycloalkene optionally substituted by 1 to 4 substituent(s) selected from the group consisting of (1) an aliphatic hydrocarbon group optionally having substituents, (2) an aromatic hydrocarbon group optionally having substituents, (3) a group represented by the formula -OR¹ (wherein R¹ represents the same meaning as mentioned above) and (4) a halogen atom, Ar represents an aromatic hydrocarbon group optionally having substituents, a group represented by the formula: 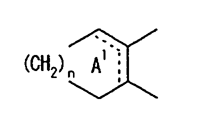 can be a group represented by the formula: 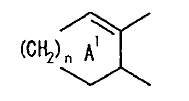 or a group represented by the formula: 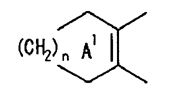 and n is an integer of 1 to 4], or (b) a compound represented by the formula: 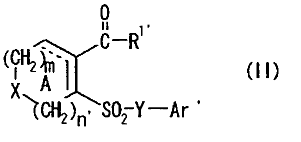 [wherein R^{1'} represents an aliphatic hydrocarbon group optionally having substituents, an aromatic hydrocarbon group optionally having substituents, a heterocyclic group optionally having substituents, a group represented by the formula: OR^{1a}' (wherein R^{1a}' represents a hydrogen atom or an aliphatic hydrocarbon group optionally having substituents) or a group represented by the formula: 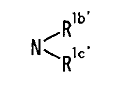 (wherein R^{1b'} and R^{1c'} are same or different from each other and represent a hydrogen atom or an aliphatic hydrocarbon group optionally having substituents), X represents a methylene group, a nitrogen atom, a sulfur atom, or an oxygen atom, Y represents an optionally substituted methylene group or an optionally substituted nitrogen atom, a ring A is a 5 to 8 membered ring optionally further substituted by 1 to 4 substituent(s) selected from the group consisting of (1) an aliphatic hydrocarbon group optionally having substituents, (2) an aromatic hydrocarbon group optionally having substituents, (3) a group represented by the formula: OR^{2'} (wherein R^{2'} represents a hydrogen atom or an aliphatic hydrocarbon group optionally having substituents) and (4) a halogen atom, Ar' represents an aromatic hydrocarbon group optionally having substituents, a group represented by the formula: 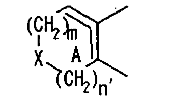 can be a group represented by the formula: 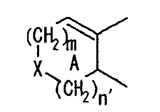 or a group represented by the formula: 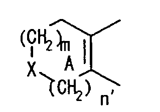 m represents an integer of 0 to 2, n' represents an integer of 1 to 3, and sum of m and n' is 4 or less, provided that when X is a methylene group, Y represents an optionally substituted methylene group], or a salt thereof or a prodrug thereof.
[4] The composition of [3], wherein the compound represented by the formula (I) is (6R)-6-[N-(2-chloro-4-fluorophenyl)sulfamoyl]cyclohex-1-en-1-carboxylate, d-ethyl 6-[N-(2,4-difluorophenyl)sulfamoyl]cyclohex-1-en-1-carboxylate, ethyl 6-[N-(2-chlorophenyl)sulfamoyl]cyclohex-1-en-1-carboxylate or ethyl 6-[N-(2-chloro-4-methylphenyl)sulfamoyl]cyclohex-1-en-1-carboxylate.
[5] The composition of any of [1] or [2], wherein the buffer is 1, 2 or more buffers selected from the group consisting of acetate buffer, lactate buffer, citrate buffer, and phosphate buffer.
[6] The composition of [5], wherein the acetate buffer is acetic acid and sodium acetate.
[7] The emulsion composition of any of [1] or [2], wherein the buffer concentration is 100mM or less.
[8] The composition of [1], which further comprises a component selected from the group consisting of oil, emulsifier, water and a combination thereof.
[9] The composition of [8], which is an oil-in-water type.
[10] The composition of any of [1] or [2], which comprises a disperse phase particle comprising a compound stable in an acidic range, oil, and emulsifier, and water wherein the disperse phase particle is dispersed.
[11] The composition of [10], wherein the disperse phase particle has an average particle size of about 0.025 to about 0.7 µm.
[12] The composition of [10], wherein the disperse phase particle and water wherein the disperse phase particle is dispersed are not phase separated and are stable.
[13] The composition of [9], which does not contain visibly identifiable free oil-droplet.
[14] The composition of [8], wherein the oil is a vegetable oil.
[15] The composition of [14], wherein the vegetable oil is a soybean oil.
[16] The composition of [8], wherein the emulsifier is phospholipid.
[17] The composition of [16], wherein the phospholipid is egg yolk lecithin or phosphatidylglycerol.
[18] The composition of [2], wherein the anionic synthetic phospholipids is phosphatidylglycerol.
[19] The composition of [8], wherein the oil is in a proportion of about 1 to about 30 weight percent of the composition in total.
[20] The composition of [8], wherein the emulsifier is in a proportion of about 0.1 to 10 % (W/V) of the composition in total.
[21] The composition of any of [17] or [18], wherein phosphatidylglycerol is dimyristoylphosphatidylglycerol.
[22] The composition of [8], which further comprises a soybean oil, egg yolk lecithin, glycerine, and water.
[23] The composition of any of [1] or [2], which is for an injection.
[24] The composition of any of [1] or [2], wherein the compound stable in an acidic region is in a proportion of about 0.001 to about 95 weight percent of the composition in total.
[25] A preparation method of an emulsion composition comprising (A) a compound stable in an acidic range and (B) a buffer, which comprises the step of adjusting pH of the emulsion composition to the range of about 3.7 to about 5.5.
[26] The preparation method of [25], wherein the emulsion composition further comprises an anionic synthetic phospholipids as an emulsifier.
[27] The preparation method of [25], whereby stability of pH of the emulsion composition and particle size of the disperse phase particle during autoclave sterilization is improved.
[28] A method for long-term stabilizing an emulsion composition, wherein the emulsion composition comprises (A) a compound stable in an acidic range, and (B) a buffer, the method comprises the step of adjusting pH of the emulsion composition to the range of about 3.7 to about 5.5.
[29] The method of [28], which further comprises the step of adding an anionic synthetic phospholipids as an emulsifier to the emulsion composition.
   The present invention further provides the following.
[30] The composition of any of [1] or [2], which is a nitric oxide or/and cytokine production inhibitor, or a TLR signal inhibitor.
[31] The composition of any of [1] or [2], which is an agent for treating cardiac disease, autoimmune disease, sepsis, septic shock, severe sepsis, organopathy, ichorrhemia, endotoxin shock, shock, inflammatory disease, central nervous system disease or infectious disease.
[32] A method for treating cardiac disease, autoimmune disease, sepsis, septic shock, severe sepsis, organopathy, ichorrhemia, endotoxin shock, shock, inflammatory disease, central nervous system disease or infectious disease, comprising administrating to a mammal in need thereof a pharmaceutically effective amount of the composition of any of [1] or [2].
[33] Use of the composition of any of [1] or [2] for manufacturing an agent for preventing or treating cardiac disease, autoimmune disease, sepsis, septic shock, severe sepsis, organopathy, ichorrhemia, endotoxin shock, shock, inflammatory disease, central nervous system disease or infectious disease.
[34] The composition of [3] wherein in the formula (I), R is
   (1) (1) linear or branched C₁₋₂₀ alkyl group, (2) C₃₋₁₀ cycloalkyl group, (3)C₄₋₁₂ cycloalkylalkyl group, (4)lower (C₃-₆) alkenyl group or (5)lower (C₃₋₆) alkynyl group (wherein the substituent selected from substituent group A may form, together with (1) linear or branched (C₁₋₂₀) alkyl group, (2) C₃₋₁₀ cycloalkyl group, (3)C₄₋₁₂ cycloalkylalkyl group, (4)lower (C₃₋₆) alkenyl group or (5)lower (C₃₋₆) alkynyl group, indanyl group or 1,2,3,4-tetrahydronaphthyl group optionally having 1 to 4 substituents selected from the substituent group A), optionally having 1 to 4 substituents selected from a group (hereinafter substituent group A) consisting of (i) a 5- to 8-membered ring group or a condensed ring group containing 1 to 4 hetero atoms selected from nitrogen atom (optionally oxidized), oxygen atom and sulfur atom, which is optionally substituted by 1 to 3 substituent(s) selected from C₁₋₄ alkyl, hydroxy, oxo and C₁₋₄ alkoxy, (ii) oxo group, (iii) hydroxyl group, (iv) C₁₋₆ alkoxy group, (v) C₃₋₁₀ cycloalkyloxy group, (vi) C₆₋₁₀ aryloxy group, (vii) C₇₋₁₉ aralkyloxy group, (viii) a 5- to 8-membered ring group or a condensed ring-oxy group containing 1 to 4 hetero atoms selected from nitrogen atom (optionally oxidized), oxygen atom and sulfur atom, which is optionally substituted by 1 to 3 substituent(s) selected from C₁₋₄ alkyl, hydroxy, oxo and C₁₋₄ alkoxy, (ix) C₁₋₆ alkylthio group (the sulfur atom may be oxidized), (x) C₃₋₁₀ cycloalkylthio group (the sulfur atom may be oxidized), (xi) C₆₋₁₀ arylthio group (the sulfur atom may be oxidized), (xii) C₇₋₁₉ aralkylthio group (the sulfur atom may be oxidized), (xiii) a 5- to 8-membered ring group or a condensed ring-thio group containing 1 to 4 hetero atoms selected from nitrogen atom (optionally oxidized), oxygen atom and sulfur atom, which is optionally substituted by 1 to 3 substituent(s) selected from C₁₋₄ alkyl, hydroxy, oxo and C₁₋₄ alkoxy, (xiv) a 5- to 8-membered ring group or a condensed ring-sulfinyl group containing 1 to 4 hetero atoms selected from nitrogen atom (optionally oxidized), oxygen atom and sulfur atom, which is optionally substituted by 1 to 3 substituent(s) selected from C₁₋₄ alkyl, hydroxy, oxo and C₁₋₄ alkoxy, (xv) a 5- to 8-membered ring group or a condensed ring-sulfonyl group containing 1 to 4 hetero atoms selected from nitrogen atom (optionally oxidized), oxygen atom and sulfur atom, which is optionally substituted by 1 to 3 substituent(s) selected from C₁₋₄ alkyl, hydroxy, oxo and C₁₋₄ alkoxy, (xvi) nitro group, (xvii) halogen atom, (xviii) cyano group, (xix) carboxyl group, (xx) C₁₋₁₀ alkoxy-carbonyl group, (xxi) C₃₋₆ cycloalkyloxy-carbonyl group, (xxii) C₆₋₁₀ aryloxy-carbonyl group, (xxiii) C₇₋₁₉ aralkyloxy-carbonyl group, (xxiv) a 5- to 8-membered ring group or a condensed ring-oxycarbonyl group containing 1 to 4 hetero atoms selected from nitrogen atom (optionally oxidized), oxygen atom and sulfur atom, which is optionally substituted by 1 to 3 substituent(s) selected from C₁₋₄ alkyl, hydroxy, oxo and C₁₋₄ alkoxy, (xxv) C₆₋₁₀ arylcarbonyl group, (xxvi) C₁₋₆ alkanoyl group, (xxvii) C₃₋₅ alkenoyl group, (xxviii) C₆₋₁₀ aryl-carbonyloxy group, (xxix) C₂₋₆ alkanoyloxy group, (xxx) C₃₋₅ alkenoyloxy group, (xxxi) carbamoyl group or cyclic aminocarbonyl group optionally substituted by 1 or 2 substituent(s) selected from C₁₋₄ alkyl, phenyl, C₁₋₇ acyl and C₁₋₄ alkoxy-phenyl, (xxxii) thiocarbamoyl group optionally substituted by 1 or 2 substituent(s) selected from C₁₋₄ alkyl and phenyl, (xxxiii) carbamoyloxy group optionally substituted by 1 or 2 substituent(s) selected from C₁₋₄ alkyl and phenyl, (xxxiv) C₁₋₆ alkanoylamino group, (xxxv) C₆₋₁₀ aryl-carbonylamino group, (xxxvi) C₁₋₁₀ alkoxy-carboxamide group, (xxxvii) C₆₋₁₀ aryloxy-carboxamide group, (xxxviii) C₇₋₁₉ aralkyloxy-carboxamide group, (xxxix) C₁₋₁₀ alkoxy-carbonyloxy group, (xxxx) C₆₋₁₀ aryloxy-carbonyloxy group, (xxxxi) C₇₋₁₉ aralkyloxy-carbonyloxy group, (xxxxii) C₃₋₁₀ cycloalkyloxy-carbonyloxy group, (xxxxiii) ureido group optionally substituted by 1 to 3 substituent(s) selected from C₁₋₄ alkyl group and phenyl group, and (xxxxiv) C₆₋₁₀ aryl group optionally having 1 to 4 substituents selected from a group consisting of the above-mentioned (i) - (xxxxiii),
   (2) C₆₋₁₄ aromatic hydrocarbon group optionally having 1 to 5 substituents selected from the group consisting of halogen atom, C₁₋₄ alkyl group, C₁₋₄ alkoxy group, C₁₋₄ alkoxy-carbonyl group, carboxyl group, nitro group, cyano group, hydroxyl group, C₁₋₄ alkanoylamino group, C₃₋₆ cycloalkyl group, C₆₋₁₀ aryl group, halogeno C₁₋₄ alkyl group, halogeno C₁₋₄ alkoxy group, C₁₋₄ alkylthio group, C₁₋₄ alkylsulfonyl group, C₁₋₄ alkanoyl group, 5-membered aromatic heterocyclic group, carbamoyl group, C₁₋₄ alkyl-carbamoyl group, C₁₋₄ alkoxy-carbonyl-C₁₋₄ alkyl-carbamoyl group and 1,3-diacylguanidino-C₁₋₄ alkyl group,
   (3) a 5- to 8-membered ring group or a condensed ring group containing 1 to 4 hetero atom selected from nitrogen atom (optionally oxidized), oxygen atom and sulfur atom, which optionally has 1 to 3 substituents selected from the group consisting of C₁₋₄ alkyl, hydroxy, oxo and C₁₋₄ alkoxy,
   (4) a group of the formula -OR¹ (wherein R¹ is (i) hydrogen atom or (ii) <1>linear or branched C₁₋₂₀ alkyl group, <2>C₃₋₁₀ cycloalkyl group, <3>C₄₋₁₂ cycloalkylalkyl group, <4>lower (C₃₋₆) alkenyl group or <5> lower (C₃₋₆) alkynyl group (wherein the substituent selected from substituent group A may form, together with <1>linear or branched C₁₋₂₀ alkyl group, <2>C₃₋₁₀ cycloalkyl group, <3>C₄₋₁₂ cycloalkylalkyl group, <4>lower (C₃₋₆) alkenyl group or <5>lower (C₃₋₆) alkynyl group, indanyl group or 1,2,3,4-tetrahydronaphthyl group optionally having 1 to 4 substituents selected from the substituent group A), which optionally has 1 to 4 substituents selected from the substituent group A), or
   (5) a group of the formula 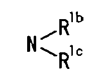 (wherein R^{1b} is (i) hydrogen atom or (ii) <1>linear or branched C₁₋₂₀ alkyl group, <2>C₃₋₁₀ cycloalkyl group, <3>C₄₋₁₂ cycloalkylalkyl group, <4>lower (C₃₋₆) alkenyl group or <5>lower (C₃₋₆) alkynyl group (wherein the substituent selected from substituent group A may form, together with <1>linear or branched C₁₋₂₀ alkyl group, <2>C₃₋₁₀ cycloalkyl group, <3>C₄₋₁₂ cycloalkylalkyl group, <4>lower (C₃₋₆) alkenyl group or <5>lower (C₃₋₆) alkynyl group, indanyl group or 1,2,3,4-tetrahydronaphthyl group optionally having 1 to 4 substituents selected from the substituent group A), which optionally has 1 to 4 substituents selected from the substituent group A), and R^{1c} is the same or different from R^{1b} and is (i) hydrogen atom or (ii) <1>linear or branched C₁₋₂₀ alkyl group, <2>C₃₋₁₀ cycloalkyl group, <3>C₄₋₁₂ cycloalkylalkyl group, <4>lower (C₃-6) alkenyl group or <5> lower (C₃₋₆) alkynyl group (wherein the substituent selected from substituent group A may form, together with <1>linear or branched C₁₋₂₀ alkyl group, <2>C₃₋₁₀ cycloalkyl group, <3>C₄₋₁₂ cycloalkylalkyl group, <4>lower (C₃₋₆) alkenyl group or <5>lower (C₃₋₆) alkynyl group, indanyl group or 1,2,3,4-tetrahydronaphthyl group optionally having 1 to 4 substituents selected from the substituent group A) optionally having 1 to 4 substituents selected from substituent group A),
R⁰ represents a hydrogen atom, a linear or branched C₁₋₂₀ alkyl group, a C₃₋₁₀ cycloalkyl group, a C₄₋₁₂ cycloalkylalkyl group, a lower (C₃₋₆) alkenyl group or a lower (C₃₋₆) alkynyl group,
or R and R° are joined to form a bond,
ring A¹ represents
(1) <1>linear or branched C₁₋₂₀ alkyl group, <2>C₃₋₁₀ cycloalkyl group, <3>C₄₋₁₂ cycloalkylalkyl group, <4>lower (C₃-₆) alkenyl group or <5>lower (C₃₋₆) alkynyl group, which optionally has 1 to 4 substituents selected from substituent group A (wherein the substituents selected from the substituent group A may form, together with <1>linear or branched C₁₋₂₀ alkyl group, <2>C₃₋₁₀ cycloalkyl group, <3>C₄₋₁₂ cycloalkylalkyl group, <4>lower (C₃₋₆) alkenyl group or <5>lower (C₃₋₆) alkynyl group, indanyl group or 1,2,3,4-tetrahydronaphthyl group optionally having 1 to 4 substituents selected from the substituent group A),
(2) a C₆₋₁₄ aromatic hydrocarbon group optionally having 1 to 5 substituents selected from the group consisting of halogen atom, C₁₋₄ alkyl group, C₁₋₄ alkoxy group, C₁₋₄ alkoxy-carbonyl group, carboxyl group, nitro group, cyano group, hydroxyl group, C₁₋₄ alkanoylamino group, C₃₋₆ cycloalkyl group, C₆₋₁₀ aryl group, halogeno C₁₋₄ alkyl group, halogeno C₁₋₄ alkoxy group, C₁₋₄ alkylthio group, C₁₋₄ alkylsulfonyl group, C₁₋₄ alkanoyl group, 5-membered aromatic heterocyclic group, carbamoyl group, C₁₋₄ alkyl-carbamoyl group, C₁₋₄ alkoxy-carbonyl-C₁₋₄ alkyl-carbamoyl group and 1,3-diacylguanidino-C₁₋₄ alkyl group,
(3) a group of the formula -OR¹ (wherein R¹ is as defined above) or
(4) cycloalkene optionally substituted by 1 to 4 substituent(s) selected from halogen atoms, and
   Ar represents a C₆₋₁₄ aromatic hydrocarbon group optionally having 1 to 5 substituents selected from the group consisting of halogen atom, C₁₋₄ alkyl group, C₁₋₄ alkoxy group, C₁₋₄ alkoxycarbonyl group, carboxyl group, nitro group, cyano group, hydroxyl group, C₁₋₄ alkanoylamino group, C₃₋₆ cycloalkyl group, C₆₋₁₀ aryl group, halogeno C₁₋₄ alkyl group, halogeno C₁₋₄ alkoxy group, C₁₋₄ alkylthio group, C₁₋₄ alkylsulfonyl group, C₁-₄ alkanoyl group, 5-membered aromatic heterocyclic group, carbamoyl group, C₁₋₄ alkyl-carbamoyl group, C₁₋₄ alkoxy-carbonyl-C₁₋₄ alkyl-carbamoyl group and 1,3-diacylguanidino-C₁₋₄ alkyl group.
[35] The composition of [3], wherein the compound represented by the formula (I) is (i) a compound represented by the formula: 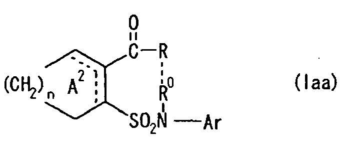 [wherein R represents an aliphatic hydrocarbon group optionally having substituents, an aromatic hydrocarbon group optionally having substituents, a heterocyclic group optionally having substituents, a group represented by the formula -OR¹ (wherein R¹ represents a hydrogen atom or an aliphatic hydrocarbon group optionally having substituents) or a group represented by the formula: 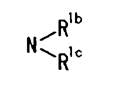 (wherein R^{1b} represents a hydrogen atom or an aliphatic hydrocarbon group optionally having substituents, R^{1c} is, same with or different from R^{1b}, a hydrogen atom or an aliphatic hydrocarbon group optionally having substituents), R° represents a hydrogen atom or an aliphatic hydrocarbon group, or R and R° are joined to form a bond, ring A² is a cycloalkene substituted by 1 to 4 substituent(s) selected from (1) an aliphatic hydrocarbon group optionally having substituents, (2) an aromatic hydrocarbon group optionally having substituents, (3) a group represented by the formula -OR¹ (wherein R¹ represents the same meaning as mentioned above) and (4) a halogen atom, Ar represents an aromatic hydrocarbon group optionally having substituents, a group represented by the formula: 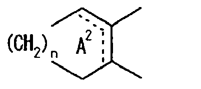 can be a group represented by the formula: 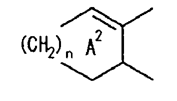 or a group represented by the formula: 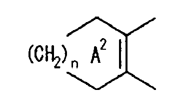 and n represents an integer of 1 to 4], or (ii) a compound represented by the formula: 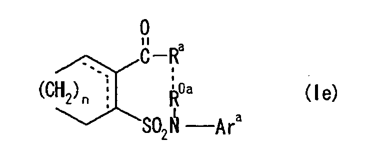 [wherein R^{a} represents an aliphatic hydrocarbon group optionally having substituents, an aromatic hydrocarbon group optionally having substituents, a heterocyclic group optionally having substituents, a group represented by the formula -OR^{1a} (wherein R^{1a} represents a hydrogen atom or an aliphatic hydrocarbon group optionally having substituents) or a group represented by the formula: 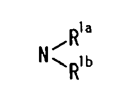 (wherein R^{1a} represents the same meaning as defined above, R^{1b} is, same with or different from R^{1a}, a hydrogen atom or an aliphatic hydrocarbon group optionally having substituents, R^{0a} represents a hydrogen atom or an aliphatic hydrocarbon group, or R^{a} and R^{0a} are joined to form a bond, Ar^{a} represents an aromatic hydrocarbon group optionally having substituents, a group represented by the formula: 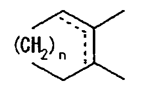 can be a group represented by the formula: 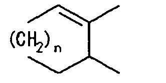 or a group represented by the formula: 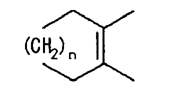 and n represents an integer of 1 to 4].
[36] The composition of [35], wherein the compound represented by the formula (Iaa) is a compound represented by the formula: 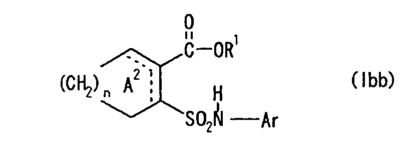 [wherein each symbols represents the same meaning as defined in [35]].
[37] The composition of [35], wherein the ring A² is a cycloalkene substituted by lower alkyl, phenyl or halogen, R¹ is a lower alkyl group, Ar is a phenyl group optionally having substituents, and n is 2.
[38] The composition of [35], wherein the compound represented by the formula (Ie) is a compound of the formula: 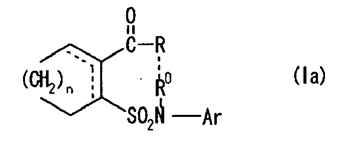 [wherein R represents an aliphatic hydrocarbon group optionally having substituents, an aromatic hydrocarbon group optionally having substituents, a heterocyclic group optionally having substituents, a group represented by the formula -OR¹ (wherein R¹ represents a hydrogen atom or an aliphatic hydrocarbon group optionally having substituents) or a group represented by the formula: 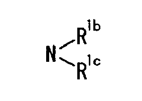 (wherein R^{1b} represents a hydrogen atom or an aliphatic hydrocarbon group optionally having substituents, R^{1c} is, same with or different from R^{1b}, a hydrogen atom or an aliphatic hydrocarbon group optionally having substituents), R° represents a hydrogen atom or an aliphatic hydrocarbon group, or R and R° are joined to form a bond, Ar represents an aromatic hydrocarbon group optionally having substituents, a group represented by the formula: 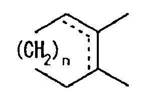 can be a group represented by the formula: 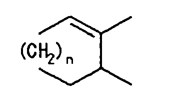 or a group represented by the formula: 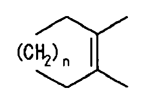 and n represents an integer of 1 to 4, provided that when n is 1 or 2 and (i) R¹ is a hydrogen atom or an ethyl group, R° is a methyl group and Ar is a phenyl group or (ii) R and R° are joined to form a bond and Ar is a phenyl group, a 2-methylphenyl group, a 4-bromophenyl group, a 4-methoxyphenyl group or a 2,6-dimethylphenyl group, a group represented by the formula: 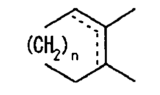 can be a group represented by the formula: 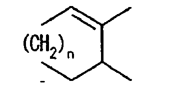
[39] The composition of [38], wherein the compound represented by the formula (Ia) is a compound represented by the formula: 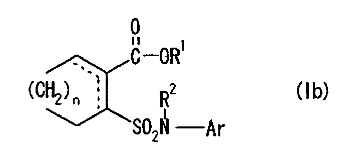 [wherein R² represents a hydrogen atom or an aliphatic hydrocarbon group, R¹, Ar, n and the group represented by the formula: 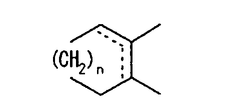 represent the same meanings as defined in [38], provided that when n is 1 or 2, Ar is a phenyl group, R¹ is a hydrogen atom or an ethyl group and R² is a methyl group, the group represented by the formula: 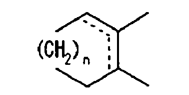 can be a group represented by the formula: 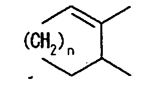
[40] The composition of [39], wherein R¹ is a lower alkyl group optionally having substituents.
[41] The composition of [39], wherein R¹ is an ethyl group.
[42] The composition of [39], wherein R² is a hydrogen atom or a lower alkyl group.
[43] The composition of [39], wherein R² is a hydrogen atom.
[44] The composition of [39], wherein Ar is a phenyl group optionally having substituents.
[45] The composition of [39], wherein Ar is a phenyl group substituted by halogen or/and lower alkyl.
[46] The composition of [39], wherein Ar is a group represented by the formula: 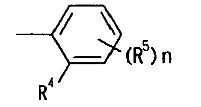 [wherein R⁴ and R⁵ are same or different from each other and represent a halogen atom or a lower alkyl group, and n represents an integer of 0 to 2].
[47] The composition of [39], wherein the halogen atom is a fluorine atom or a chlorine atom.
[48] The composition of [39], wherein the group represented by the formula: 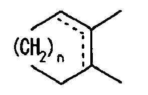 can be a group represented by the formula: 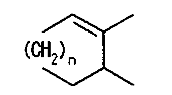 [wherein n represents the same meaning as defined in [39]].
[49] The composition of [39], wherein n is 1 to 3.
[50] The composition of [39], wherein R¹ is a lower alkyl group optionally having substituents, R² is a hydrogen atom or a lower alkyl group, Ar is a phenyl group optionally having substituents, and n is 1, 2 or 3.
[51] The composition of [39], wherein R¹ is a lower alkyl group optionally having substituents, R² is a hydrogen atom, Ar is a phenyl group substituted by a halogen atom, and n is 2.
[52] The composition of [38], wherein the compound represented by the formula (Ia) is a compound represented by the formula: 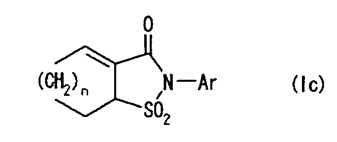 [wherein Ar and n represent the same meanings as defined in [38]].
[53] The composition of [52], wherein Ar is a phenyl group optionally having substituents, and n is 2.
[54] The composition of [38], wherein the compound represented by the formula (Ia) is a compound represented by the formula: 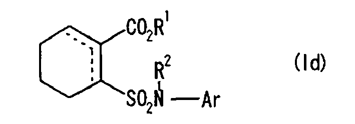 [wherein R¹, R² and Ar represent the same meanings as defined in [39], the group represented by the formula: 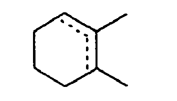 can be a group represented by the formula: 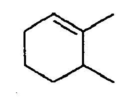 or a group represented by the formula: 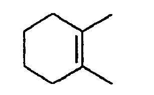 provided that when Ar is a phenyl group, R¹ is a hydrogen atom or an ethyl group and R² is a methyl group, the group represented by the formula: 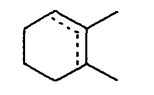 can be a group represented by the formula: 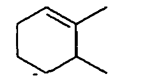
[55] The composition of [35], wherein the compound represented by the formula (Ie) is a compound represented by the formula: 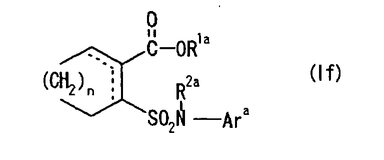 [wherein R^{2a} represents a hydrogen atom or an aliphatic hydrocarbon group, R^{1a}, Ar^{a}, n and the group represented by the formula: 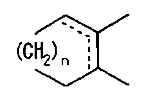 represent the same meanings as defined in [35]].
[56] The composition of [35], wherein the compound represented by the formula (Ie) is a compound represented by the formula: 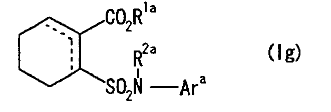 [wherein R^{1a}, R^{2a} and Ar^{a} represent the same meanings as defined in [55] and the group represented by the formula: 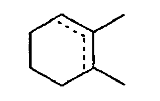 can be a group represented by the formula: 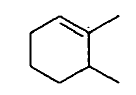 or a group represented by the formula: 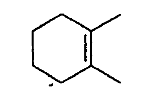
[57] The composition of [8], wherein the oil component is selected from the group consisting of vegetable oil, a partially hydrogenated vegetable oil, mono-acid glyceride, mixed acid glyceride and medium-size chain fatty acid glycerine ester.
[58] The composition of [8], wherein the vegetable oil is selected from the group consisting of soybean oil, cottonseed oil, rapeseed oil, peanut oil, safflower oil, sesame oil, rice bran oil, corn germ oil, sunflower oil, poppy oil and olive oil.
[59] The composition of [8], wherein the emulsifier is phospholipids or a non-ionic surfactant.
[60] The composition of [8], wherein the phospholipid is egg yolk lecithin, soybean lecithin, hydrogenation product thereof, phosphatidylcholine, phosphatidylethanolamine, phosphatidic acid, phosphatidylserine, phosphatidylinositol, or phosphatidylglycerol.
[61] The composition of [8], wherein the anionic phospholipid is further contained in the emulsifier in a proportion of about 0.0001 to about 5% (W/V) of the composition in total.
[62] The composition of [61], wherein the anionic phospholipid is dimyristoylphosphatidylglycerol.
[63] The composition of [8], wherein the emulsifier is contained in a proportion of about 0.1 to about 150 wt% of the oil component.

### Effects of the Invention

The emulsion composition of the present invention is adjusted to about pH 3.7 - about pH 5.5 by adding a buffering agent. Therefore, the pH of the emulsion composition and the average particle size of the disperse phase particles therein scarcely vary even after sterilization in an autoclave etc. or after a long-term preservation, and the composition is stable. Consequently, the emulsion composition of the present invention and a compound, a salt thereof or a prodrug thereof, which is the active ingredient of the emulsion composition, exhibit superior stability. Moreover, sterilization of the emulsion composition of the present invention in an autoclave etc. and a long-term preservation thereof do not result in the production of visually observable free oil drops. In other words, the disperse phase particle and water, in which the disperse phase particle has been dispersed, do not show phase separation and are stable.

In addition, since the emulsion composition of the present invention can be adjusted to pH about 3.7 - about 5.5 by adding a buffering agent to give a stable emulsion composition, an optimal pH can be determined depending on various other conditions such as the characteristics of the emulsifier and stability of the compound and the like. Therefore, even when other conditions that can be overcome by adjusting pH to about 3.7 - about 5.5 are present, such as the case where the stability of the emulsion composition is influenced, and the like, an emulsion composition satisfying various conditions can be afforded by adopting the adjustment of pH.

The emulsion composition of the present invention moreover shows long-term preservation stability for 24 months. This exceeds a general period of use of 18 months of emulsion preparations.

### Best Mode for Carrying Out the Invention

Hereinafter, embodiment of the present invention will be described.

A compound to be used in an emulsion composition of the present invention is the aforementioned compound stable in an acidic range and specifically is a compound which can be represented by the formula (I) or (II), or a salt thereof or a prodrug thereof.

### (A) About a formula (I) compound

In the specification, R represents an aliphatic hydrocarbon group optionally having substituents, an aromatic hydrocarbon group optionally having substituents, a heterocyclic group optionally having substituents, a group represented by the formula -OR¹ (wherein R¹ represents a hydrogen atom or an aliphatic hydrocarbon group optionally having substituents) or a group represented by the formula: 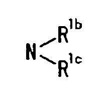 (wherein R^{1b} represents a hydrogen atom or an aliphatic hydrocarbon group optionally having substituents, R^{1c} is same with or different from R^{1b} and represents a hydrogen atom or an aliphatic hydrocarbon group optionally having substituents), or R forms a bond with R°, and among them the group represented by the formula -OR¹ [wherein R¹ represents the same meaning as defined above] is preferred.

And, R^{a} represents an aliphatic hydrocarbon group optionally having substituents, an aromatic hydrocarbon group optionally having substituents, a heterocyclic group optionally having substituents, a group represented by the formula -OR^{1a} (wherein R^{1a} represents a hydrogen atom or an aliphatic hydrocarbon group optionally having substituents) or a group represented by the formula: 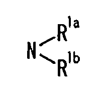 (wherein R^{1a} represents the same meaning as defined above, R^{1b} is same with or different from R^{1a} and represents a hydrogen atom or an aliphatic hydrocarbon group optionally having substituents), or form a bond with R^{0a}, and among them the group represented by the formula -OR^{1a} [wherein R^{1a} represents the same meaning as defined above] is preferred.

When R and R° are joined to form a bond, the compound represented by the formula (Iaa) can be represented by the formula: 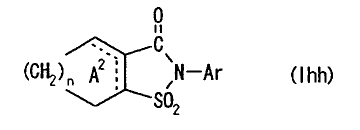 [wherein each symbol represents the same meaning as defined above], and specifically can be represented by the formula: 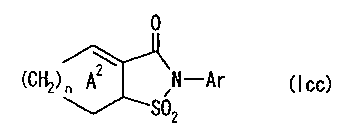 [wherein each symbol represents the same meaning as defined above], or the formula: 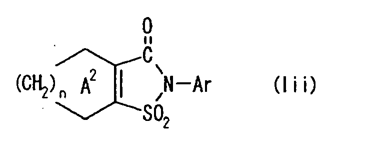 [wherein each symbol represents the same meaning as defined above].

When R and R⁰ are joined to form a bond, the compound represented by the formula (Ia) can be represented by the formula: 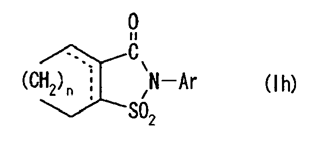 [wherein each symbol represents the same meaning as defined above], and specifically can be represented by the formula: 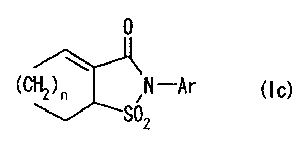 [wherein each symbol represents the same meaning as defined above], or the formula: 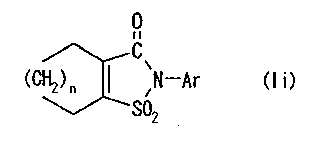 [wherein each symbol represents the same meaning as defined above].

When R^{a} and R^{0a} are joined to form a bond, the compound represented by the formula (Ie) can be represented by the formula: 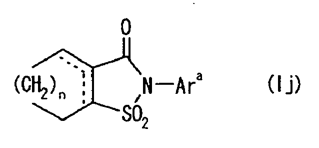 [wherein each symbol represents the same meaning as defined above], and specifically can be represented by the formula: 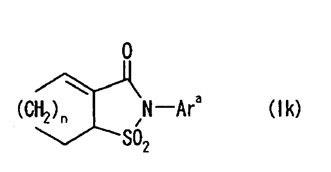 [wherein each symbol represents the same meaning as defined above], or the formula: 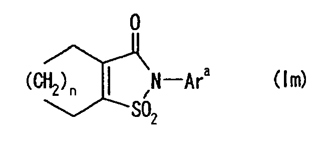 [wherein each symbol represents the same meaning as defined above].

When R is a group represented by the formula -OR¹ [wherein R¹ represents the same meaning as defined above], the compound represented by the formula (Iaa) can be represented by the formula: 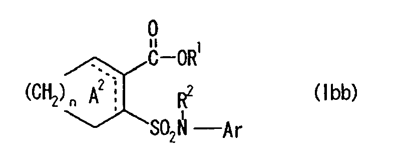 [wherein each symbol represents the same meaning as defined above], and specifically can be represented by the formula: 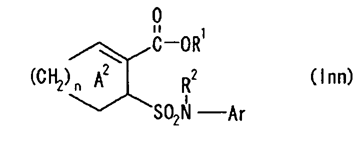 [wherein each symbol represents the same meaning as defined above], or the formula: 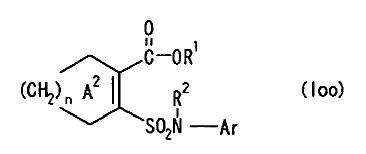 [wherein each symbol represents the same meaning as defined above].

When R is a group represented by the formula - OR¹ [wherein R¹ represents the same meaning as defined above], the compound represented by the formula (Ia) can be represented by the formula: 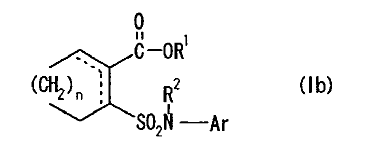 [wherein each symbol represents the same meaning as defined above], and specifically can be represented by the formula: 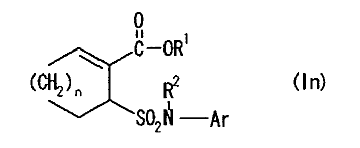 [wherein each symbol represents the same meaning as defined above], or the formula: 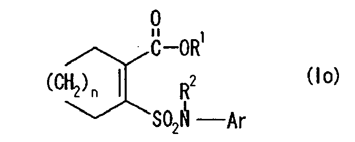 [wherein each symbol represents the same meaning as defined above].

When R^{a} is a group represented by the formula - OR^{1a} [wherein R^{1a} represents the same meaning as defined above], the compound represented by the formula (Ie) can be represented by the formula: 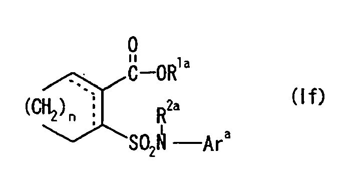 [wherein each symbol represents the same meaning as defined above], and specifically can be represented by the formula: 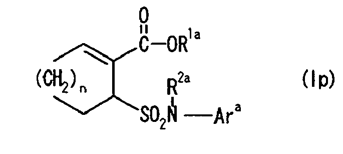 [wherein each symbol represents the same meaning as defined above], or the formula: 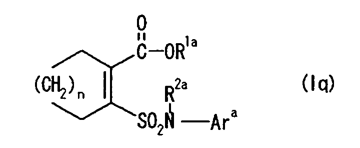 [wherein each symbol represents the same meaning as defined above].
As the compound represented by the formula (Iaa), the compound represented by the formula (Icc) or the formula (Inn) is preferred, as the compound represented by the formula (Ia), the compound represented by the formula (Ic) or the formula (In) are preferred, and as the compound represented by the formula (Ie), the compound represented by the formula (Ik) or the formula (Ip) are preferred.

Similarly, the compound represented by the formula (Id) can be represented by the formula: 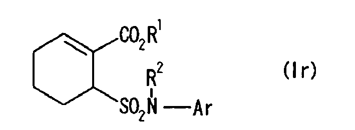 [wherein each symbol represents the same meaning as defined above], or the formula: 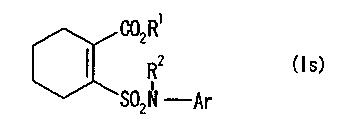 [wherein each symbol represents the same meaning as defined above], and the compound represented by the formula (Ig) can be represented by the formula: 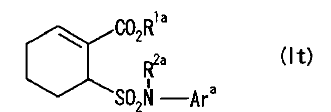 [wherein each symbol represents the same meaning as defined above], or the formula: 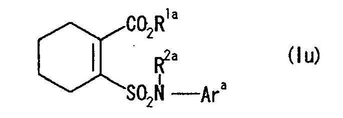 [wherein each symbol represents the same meaning as defined above].

As the compound represented by the formula (Id), the compound represented by the formula (Ir) is preferred, as the compound represented by the formula (Ig), the compound represented by the formula (It) is preferred.

In the compound represented by the formula (Ia), when n is 1 or 2, and (i) R¹ is a hydrogen atom or an ethyl group, R° is a methyl group and Ar is a phenyl group, or (ii) R and R° are joined to form a bond and Ar is a phenyl group, a 2-methylphenyl group, a 4-bromophenyl group, a 4-methoxyphenyl group or a 2,6-dimethylphenyl group, a group represented by the formula: 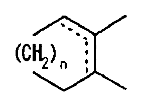 can be a group represented by the formula:

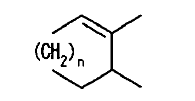

Furthermore, when n represents 1 to 4, and (i) R¹ is a hydrogen atom or a lower alkyl group optionally having substituents, R° is a lower alkyl group optionally having substituents, and Ar is a phenyl group optionally having substituents, or (ii) R and R° are joined to form a bond and Ar is a phenyl group optionally having substituents, a group represented by the formula: 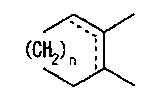 may be a group represented by the formula:

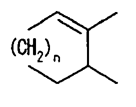

In the compound represented by the formula (Ib), when n is 1 or 2, R¹ is a hydrogen atom or an ethyl group, R° is a methyl group, and Ar is a phenyl group, a group represented by the formula: 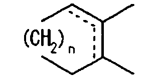 is a group represented by the formula:

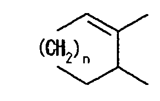

Furthermore, when n is 1 to 4, and R¹ is a hydrogen atom or a lower alkyl group optionally having substituents, R° is a lower alkyl group optionally having substituents, and Ar is a phenyl group optionally having substituents, a group represented by the formula: 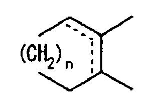 may be a group represented by the formula:

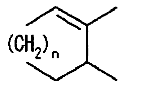

As the "aliphatic hydrocarbon group" of the "aliphatic hydrocarbon group optionally having substituents" represented by R, R^{a}, R¹, R^{1a}, R^{1b}, R^{1c}, and the "aliphatic hydrocarbon group" represented by R°, R^{0a}, R², R^{2a}, for example, an alkyl group, a cycloalkyl group, a cycloalkylalkyl group, an alkenyl group, an alkynyl group, etc. are preferred.

As the alkyl group, for example, a linear or branched alkyl group having 1 to 20 carbons (e.g., a methyl group, an ethyl group, an n-propyl group, a isopropyl group, an n-butyl group, a isobutyl group, a sec-butyl group, a tert-butyl group, a pentyl group, a hexyl group, a heptyl group, an octyl group, a nonyl group, a decyl group, a dodecyl group, etc.), etc. are preferred, and particularly, for example, a lower alkyl group having 1 to 6 carbons (e.g., a methyl group, an ethyl group, an n-propyl group, a isopropyl group, an n-butyl group, a isobutyl group, a sec-butyl group, a tert-butyl group, etc.), etc. are preferred.

As the cycloalkyl group, for example, a cycloalkyl group having 3 to 10 carbons (e.g., a cyclopropyl group, a cyclobutyl group, a cyclopentyl group, a cyclohexyl group, a cycloheptyl group, a cyclooctyl group, etc.), etc. are preferred, and particularly, for example, a cycloalkyl group having 3 to 6 carbons (e.g., a cyclopropyl group, a cyclobutyl group, a cyclopentyl group, a cyclohexyl group, etc.), etc. are preferred.

As the cycloalkylalkyl group, for example, a cycloalkylalkyl group having 4 to 12 carbons (e.g., a cyclopropylmethyl group, a cyclopentylmethyl group, a cyclohexylmethyl group, a cycloheptylmethyl group, etc.), etc. are preferred, and particularly, for example, a cycloalkylalkyl group having 4 to 8 (particularly, 4 to 7) carbons (e.g., a cyclopropylmethyl group, a cyclopentylmethyl group, a cyclohexylmethyl group, etc.), etc. are preferred.

As the alkenyl group, for example, a lower alkenyl group having 3 to 6 carbons (e.g., a propenyl group, a butenyl group, a pentenyl group, etc.), and particularly, for example, a lower alkenyl group having 3 or 4 carbons (e.g., a propenyl group, a butenyl group, etc.), etc. are preferred.

As the alkynyl group,for example, a lower alkynyl group having 3 to 6 carbons (e.g., a propynyl group, a butynyl group, a pentynyl group, etc.), and particularly, for example, a lower alkenyl group having 3 or 4 carbons (e.g., a propynyl group, a butynyl group, etc.), etc. are preferred.

As the "substituents" of the above mentioned "aliphatic hydrocarbon group optionally having substituents", for example, a heterocyclic group, an oxo group, a hydroxy group, a C₁₋₆ alkoxy group, a C₃₋₁₀ (particularly, C₃₋₆) cycloalkyloxy group, a C₆₋₁₀ aryloxy group, a C₇₋₁₉ (particularly, C₇₋₁₂) aralkyloxy group, a heterocyclic oxy group, a C₁₋₆ alkylthio group (the sulfur atom may be oxidized), a C₃₋₁₀ (particularly, C₃₋₆) cycloalkylthio group (the sulfur atom may be oxidized), a C₆₋₁₀ arylthio group (the sulfur atom may be oxidized), a C₇₋₁₉ (particularly, C₇₋₁₂) aralkylthio group (the sulfur atom may be oxidized), a heterocyclic thio group, a heterocyclic sulfinyl group, a heterocyclic sulfonyl group, a nitro group, a halogen atom, a cyano group, a carboxyl group, a C₁₋₁₀ (particularly, C₁₋₆) alkoxy-carbonyl group, a C₃₋₆ cycloalkyloxy-carbonyl group, a C₆₋₁₀ aryloxy-carbonyl group, a C₇₋₁₉ (particularly, C₇₋₁₂) aralkyloxy-carbonyl group, a heterocyclic oxycarbonyl group, a C₆₋₁₀ aryl-carbonyl group, C₁₋₆ alkanoyl group, C₃₋₅ alkenoyl group, a C₆₋₁₀ aryl-carbonyloxy group, a C₂₋₆ alkanoyloxy group, a C₃₋₅ alkenoyloxy group, a carbamoyl group optionally having substituents, a thiocarbamoyl group optionally having substituents, a carbamoyloxy group optionally having substituents, a C₁₋₆ alkanoylamino group, a C₆₋₁₀ aryl-carbonylamino group, a C₁₋₁₀ (particularly, C₁₋₆) alkoxy-carboxamide group, a C₆₋₁₀ aryloxy-carboxamide group, a C₇₋₁₉ (particularly, C₇₋₁₂) aralkyloxy-carboxamide group, a C₁₋₁₀ (particularly, C₁₋₆) alkoxy-carbonyloxy group, a C₆₋₁₀ aryloxy-carbonyloxy group, a C₇₋₁₉ (particularly, C₇₋₁₂) aralkyloxy-carbonyloxy group, a C₃₋₁₀ (particularly, C₃₋₆) cycloalkyloxy-carbonyloxy group, an ureido group optionally having substituents, a C₆₋₁₀ aryl group optionally having substituents, etc. are used.

These substituents are substituted at substitutable positions in the above mentioned "aliphatic hydrocarbon group", and the substituents are not limited to one and may be same or different and a few numbers (2 to 4).

As the "C₁₋₆ alkoxy group", for example, a methoxy group, an ethoxy group, an n-propoxy group, a isopropoxy group, an n-butoxy group, a tert-butoxy group, an n-pentyloxy group, an n-hexyloxy group, etc. are used, as the "C₃₋₁₀ cycloalkyloxy group", for example, a cyclopropyloxy group, a cyclohexyloxy group, etc. are used, as the "C₆₋₁₀ aryloxy group", for example, a phenoxy group, a naphthyloxy group, etc. are used, as the "C₇₋₁₉ aralkyloxy group", for example, a benzyloxy group, a 1-phenylethyloxy group, a 2-phenylethyloxy group, a benzhydryloxy group, a 1-naphthylmethyloxy group, etc. are used, as the "C₁₋₆ alkylthio group (the sulfur atom may be oxidized)", for example, a methylthio group, an ethylthio group, an n-propylthio group, an n-butylthio group, a methylsulfinyl group, a methylsulfonyl group, etc. are used, as the "C₃₋₁₀ cycloalkylthio group (the sulfur atom may be oxidized)", for example, a cyclopropylthio group, a cyclohexylthio group, a cyclopentylsulfinyl group, a cyclohexylsulfonyl group, etc. are used, as the "C₆₋₁₀ arylthio group (the sulfur atom may be oxidized)", for example, a phenylthio group, a naphthylthio group, a phenylsulfinyl group, a phenylsulfonyl group, etc. are used, as the "C₇₋₁₉ aralkylthio group (the sulfur atom may be oxidized)", for example, a benzylthio group, a phenylethylthio group, a benzhydrylthio group, a benzylsulfinyl group, a benzylsulfonyl group, etc. are used, as the "halogen atom", for example, a fluorine atom, a chlorine atom, a bromine atom, an iodine atom, ets. are used, as the "C₁₋₁₀ alkoxy-carbonyl group", for example, a methoxycarbonyl group, an ethoxycarbonyl group, an n-propoxycarbonyl group, a isopropoxycarbonyl group, an n-butoxycarbonyl group, a isobutoxycarbonyl group, a tert-butoxycarbonyl group, etc. are used, as the "C₃₋₆ cycloalkyloxycarbonyl group", for example, a cyclopropyloxycarbonyl group, a cyclopentyloxycarbonyl group,a cyclohexyloxycarbonyl group, a norbornyloxycarbonyl group, etc. are used, as the "C₆₋₁₀ aryloxy-carbonyl group", for example, a phenoxycarbonyl group, a naphthyloxycarbonyl group, etc. are used, as the "C₇₋₁₉ aralkyl-oxycarbonyl group", for example, a benzyloxycarbonyl group, a benzhydryloxycarbonyl group, a 2-phenethyloxycarbonyl group, etc. are used, as the "C₆₋₁₀ aryl-carbonyl group", for example, a benzoyl group, a naphthoyl group, a phenylacetyl group, etc. are used, as the "C₁₋₆ alkanoyl group", for example, a formyl group, an acetyl group, a propionyl group, a butyryl group, a valeryl group, a pivaloyl group, etc. are used, as the "C₃₋₅ alkenoyl group", for example, an acryloyl group, a crotnoyl group, etc. are used, as the "C₆₋₁₀ aryl-carbonyloxy group", for example, a benzoyloxy group, a naphthoyloxy group, a phenylacetoxy group, etc. are used, as the "C₂₋₆ alkanoyloxy group", for example, an acetoxy group, a propionyloxy group, a butyryloxy group, a valeryloxy group, a pivaloyloxy group, etc. are used, as the "C₃₋₅ alkenoyloxy group", for example, an acryloyloxy group, a crotnoyloxy group, etc. are used.

As the "carbamoyl group optionally having substituents", for example, a carbamoyl group or a cyclic aminocarbonyl group, which may be substituted by 1 or 2 substituents selected from C₁₋₄ alkyl (e.g., methyl, ethyl, etc.), phenyl, C₁₋₇ acyl (e.g., acetyl, propionyl, benzoyl, etc.), C₁₋₄ alkoxy-phenyl (e.g., methoxyphenyl, etc.), etc. and specifically for example a carbamoyl group, an N-methylcarbamoyl group, an N-ethylcarbamoyl group, an N,N-dimethylcarbamoyl group, an N,N-diethylcarbamoyl group, an N-phenylcarbamoyl group, an N-acetylcarbamoyl group, an N-benzoylcarbamoyl group, an N-(p-methoxyphenyl)carbamoyl group, a 1-pyrrolidinylcarbonyl group, a piperidinocarbonyl group, a 1-piperazinylcarbonyl group, a morpholinocarbonyl group, etc. are used. As the "thiocarbamoyl group optionally having substituents", for example, a thiocarbamoyl group which may be substituted by 1 or 2 substituents selected from C₁₋₄ alkyl (e.g., methyl, ethyl, etc.), phenyl, etc., and specifically for example a thiocarbamoyl group, an N-methylthiocarbamoyl group, an N-phenylthiocarbamoyl group, etc. are used. As the "carbamoyloxy group optionally having substituents", for example, a carbamoyloxy group which may be substituted by 1 or 2 substituents selected from C₁₋₄ alkyl (e.g., methyl, ethyl, etc.), phenyl, etc. and specifically for example a carbamoyloxy group, an N-methylcarbamoyloxy group, an N,N-dimethylcarbamoyloxy group, an N-ethylcarbamoyloxy group, an N-phenylcarbamoyloxy group, etc. are used.

As the "C₁₋₆ alkanoylamino group", for example, an acetoamide group, a propionamide group, a butyroamide group, a valeroamide group, a pivaroamide group, etc. are used, as the "C₆₋₁₀ aryl-carbonylamino group", for example, a benzamide group, a naphthoamide group, a phthalimide group, etc. are used, as the "C₁₋₁₀ alkoxy-carboxamide group", for example, a methoxycarboxamide (CH₃OCONH-) group, an ethoxycarboxamide group, a tert-butoxycarboxamide group, etc. are used, as the "C₆₋₁₀ aryloxy-carboxamide group", for example, a phenoxycarboxamide (C₆H₅OCONH-) group, etc. are used, as the "C₇₋₁₉ aralkyloxy-carboxamide group", for example, a benzyloxycarboxamide (C₆H₅CH₂OCONH-) group, a benzhydryloxycarboxamide group, etc. are used, as the "C₁₋₁₀ alkoxy-carbonyloxy group", for example, a methoxycarbonyloxy group, an ethoxycarbonyloxy group, an n-propoxycarbonyloxy group, a isopropoxycarbonyloxy group, an n-butoxycarbonyloxy group, a tert-butoxycarbonyloxy group, an n-pentyloxycarbonyloxy group, an n-hexyloxycarbonyloxy group, etc. are used, as the "C₆₋₁₀ aryloxy-carbonyloxy group", for example, a phenoxycarbonyloxy group, a naphthyloxycarbonyloxy group, etc. are used, as the "'C₇₋₁₉ aralkyloxy-carbonyloxy group", for example, a benzyloxycarbonyloxy group, a 1-phenylethyloxycarbonyloxy group, a 2-phenylethyloxycarbonyloxy group, a benzhydryloxycarbonyloxy group, etc. are used, and as the "C₃₋₁₀ cycloalkyloxy-carbonyloxy group", for example, a cyclopropyloxycarbonyloxy group, a cyclohexyloxycarbonyloxy group, etc. are used.

As the "ureido group optionally having substituents", for example, an ureido group optionally substituted by 1 to 3 (particularly, 1 or 2) substituents selected from a C₁₋₄ alkyl group (e.g., a methyl group, an ethyl group, etc.), a phenyl group, etc. are used, and for example an ureido group, a 1-methylureido group, a 3-methylureido group, a 3,3-dimethylureido group, a 1,3-dimethylureido group, a 3-phenylureido group, etc. used.

When a heterocyclic group, a heterocyclic oxy group, a heterocyclic thio group, a heterocyclic sulfinyl group, a heterocyclic sulfonyl group or a heterocyclic oxycarbonyl group is used as the "substituents" of the "aliphatic hydrocarbon group optionally having substituents", the heterocyclic group represents a group formed by excluding one hydrogen atom which binds to the heterocycle, and it represents, for example, a 5- to 8-membered cyclic (preferably 5- to 6-membered cyclic) group containing 1 to a few, preferably 1 to 4 hetero atoms such as a nitrogen atom (optionally oxidized), an oxygen atom, a sulfur atom, etc., or a condensed cyclic group thereof. As these heterocyclic group, for example, a pyrrolyl group, a pyrazolyl group, an imidazolyl group, a 1,2,3-triazolyl group, a 1,2,4-triazolyl group, a tetrazolyl group, a furyl group, a thienyl group, an oxazolyl group, an isoxazolyl group, a 1,2,3-oxadiazolyl group, a 1,2,4-oxadiazolyl group, a 1,2,5-oxadiazolyl group, a 1,3,4-oxadiazolyl group, a thiazolyl group, an isothiazolyl group, a 1,2,3-thiadiazolyl group, a 1,2,4-thiadiazolyl group, a 1,2,5-thiadiazolyl group, a 1,3,4-thiadiazolyl group, a pyridyl group, a pyridazinyl group, a pyrimidinyl group, a pyrazinyl group, an indolyl group, a pyranyl group, a thiopyranyl group, a dioxynyl group, a dioxolyl group, a quinolyl group, a pyrido[2,3-d]pyrimidyl group, 1,5-, 1,6-, 1,7-, 1,8-, 2,6- or 2,7-naphthyridyl group, a thieno[2,3-d]pyridyl group, a benzpyranyl group, a tetrahydrofuryl group, a tetrahydropyranyl group, a dioxolanyl group, a dioxanyl group, etc. are used.

These heterocyclic groups may be substituted at possible positions by 1 to 3 substituents selected from C₁₋₄ alkyl (e.g., methyl, ethyl, etc.), hydroxy, oxo, C₁₋₄ alkoxy (e.g., methoxy, ethoxy, etc.), etc.

As the "C₆₋₁₀ aryl group" of the "C₆₋₁₀ aryl group optionally having substituents", for example, a phenyl group, a naphthyl group, etc. are used. The C₆₋₁₀ aryl group may be substituted at a substitutable position by a substituent selected from the those listed as a "substituent" (except for an optionally substituted C₆₋₁₀ aryl group) of the "aliphatic hydrocarbon group optionally having substituents" described above. Such a substituent is substituted at a substitutable position in the C₆₋₁₀ aryl group, and the number of such substituents is not limited to one, and, the same or different, more than one (2 to 4) substituents may exist.

Additionally, in the "aliphatic hydrocarbon group optionally having substituents", the substituent together with the aliphatic hydrocarbon group may form an optionally substituted fused ring group, and as these condensed ring groups, an indanyl group, a 1,2,3,4-tetrahydronaphthyl group, etc. are used. This condensed ring group may be substituted at a substitutable position by a substituent selected from the those listed as a "substituent" of the "aliphatic hydrocarbon group optionally having substituents" described above. Such a substituent is substituted at a substitutable position in a fused ring group, and the number of such substituents is not limited to one, and, the same or different, more than one (2 to 4) substituents may exist.

As R, R^{a}, R¹, R^{1a}, R^{1b}, R^{1c}, for example, a lower alkyl group having 1 to 6 carbon atoms (e.g., a methyl group, an ethyl group, an n-propyl group, an isopropyl group, an n-butyl group, an isobutyl group, a tert-butoxycarbonylmethyl group, a hydroxyethyl group and the like) optionally having substituents, and of them a methyl group, an ethyl group, an n-propyl group, an isopropyl group, an n-butyl group, an isobutyl group, etc. are preferably used. Particularly, a methyl group, an ethyl group, an n-propyl group and the like, etc. are preferred, and an ethyl group etc. is preferred particularly.

As R², R^{2a}, for example, a hydrogen atom, a lower alkyl group having 1 to 6 carbon atoms (e.g., a methyl group, an ethyl group, an n-propyl group, an isopropyl group, an n-butyl group, an isobutyl group, a t-butoxycarbonylmethyl group, a hydroxyethyl group and the like), etc. are preferably used, and a hydrogen atom, a methyl group, etc. are preferably used and particularly a hydrogen atom, etc. are preferably used.

As R⁰, R^{0a}, for example, a hydrogen atom, a lower alkyl group having 1 to 6 carbon atoms (e.g., a methyl group, an ethyl group, an n-propyl group, an isopropyl group, an n-butyl group, an isobutyl group, a t-butoxycarbonylmethyl group, a hydroxyethyl group and the like), etc. are preferably used, and a hydrogen atom, a methyl group, etc. are preferably used.

As the "aromatic hydrocarbon group" of the "aromatic hydrocarbon group optionally having substituents" represented by R and R^{a}, an aromatic hydrocarbon group having 6 to 14 carbon atoms (e.g., a phenyl group, a naphthyl group, a biphenyl group, an anthryl group, an indenyl group and the like) and the like, and particularly an aryl group having 6 to 10 carbon atoms and the like (e.g., phenyl and naphthyl groups) are preferred and a phenyl group and the like are particularly preferred.

As the "substituent" of the "aromatic hydrocarbon group optionally having substituents" represented by R and R^{a}, for example, a halogen atom (e.g., fluorine, chlorine, bromine, iodine and the like), a lower (C₁₋₄) alkyl group (e.g., a methyl group, an ethyl group, a propyl group, a butyl group and the like), a lower (C₁₋₄) alkoxy group (e.g., a methoxy group, an ethoxy group, a propoxy group, a butoxy group and the like), a lower (C₁₋₄) alkoxycarbonyl group (e.g., a methoxycarbonyl group, an ethoxycarbonyl group, a propoxycarbonyl group, a butoxycarbonyl group and the like), a carboxyl group, a nitro group, a cyano group, a hydroxyl group, an acylamino group (e.g., an alkanoylamino group having 1 to 4 carbon atoms such as an acetylamino group, a propyonylamino group, a butyrylamino group and the like), a cycloalkyl group having 3 to 6 carbon atoms (e.g., a cyclopropyl group, a cyclopentyl group and the like), an aryl group having 6 to 10 carbon atoms (e.g., a phenyl group, a naphthyl group, an indenyl group and the like), a halogeno-lower (C₁₋₄) alkyl group (e.g., a trifluoromethyl group, a trifluoroethyl group and the like), a halogeno-lower (C₁₋₄) alkoxy group (e.g., a trifluoromethoxy group, a 1,1,2,2-tetrafluoroethoxy group, a 2,2,3,3,3-pentafluoropropoxy group and the like), a lower (C₁-₄) alkylthio group (e.g., a methylthio group, an ethylthio group, a propionylthio group and the like), a lower (C₁₋₄) alkylsulfonyl group (e.g., a methanesulfonyl group, an ethanesulfonyl group, a propanesulfonyl group and the like), a lower (C₁₋₄) alkanoyl group (e.g., a formyl group, an acetyl group, a propionyl group and the like), a 5-membered aromatic heterocyclic group (e.g., a 1,2,3-triazolyl group, a 1,2,4-triazolyl group, a tetrazolyl group, a thiazolyl group, an isothiazolyl group, an oxazolyl group, an isooxazolyl group, a thiadiazolyl group, a thienyl group, a furyl group and the like), a carbamoyl group, a lower (C₁₋₄) alkyl-carbamoyl group (e.g., a methylcarbamoyl group, a dimethylcarbamoyl group, a propionylcarbamoyl group and the like), a lower (C₁₋₄) alkoxy-carbonyl-lower (C₁₋₄) alkyl-carbamoyl group (e.g., a butoxycarbonylmethylcarbamoyl group, an ethoxycarbonylmethylcarbamoyl group and the like), a 1,3-diacylguanidino-lower (C₁₋₄) alkyl group (e.g., 1,3-diacetylguanidinomethyl, 1,3-bis-tert-butoxycarbonylguanidinomethyl and the like) and the like are used, and a halogen atom (e.g., fluorine, chlorine, bromine, iodine atoms and the like), a lower (C₁₋₄) alkyl group (e.g., a methyl group, an ethyl group, a propyl group, a butyl group and the like) and the like are preferably used, and a fluorine atom, a chlorine atom and a methyl group are more preferably used.

These substituents are substituted at substitutable positions in the aromatic hydrocarbon group, and the number of the substituents is preferably 1 to 5, more preferably 1 to 3, most preferably 1 to 2. When two or more of such substituents are present, they may be the same or different.

The "heterocyclic group" in the "heterocyclic group optionally having substituents" represented by R and R^{a} represent a 5- to 8-membered (preferably 5- to 6-membered) ring group having 1 to several, preferably 1 to 4, hetero atoms such as a nitrogen atom (optionally oxidized), an oxygen atom, a sulfur atom and the like, or a condensed ring group thereof. As these heterocyclic group, for example, a pyrrolyl group, a pyrazolyl group, an imidazolyl group, a 1,2,3-triazolyl group, a 1,2,4-triazolyl group, a tetrazolyl group, a furyl group, a thienyl group, an oxazolyl group, an isoxazolyl group, a 1,2,3-oxadiazolyl group, a 1,2,4-oxadiazolyl group, a 1,2,5-oxadiazolyl group, a 1,3,4-oxadiazolyl group, a thiazolyl group, an isothiazolyl group, a 1,2,3-thiadiazolyl group, a 1,2,4-thiadiazolyl group, a 1,2,5-thiadiazolyl group, a 1,3,4-thiadiazolyl group, a pyridyl group, a pyridazinyl group, a pyrimidinyl group, a pyrazinyl group, an indolyl group, a pyranyl group, a thiopyranyl group, a dioxynyl group, a dioxolyl group, a quinolyl group, a pyrido[2,3-d]pyrimidyl group, 1,5-, 1,6-, 1,7-, 1,8-, 2,6- or 2,7-naphthyridyl group, a thieno[2,3-d]pyridyl group, a benzpyranyl group, a tetrahydrofuryl group, a tetrahydropyranyl group, a dioxolanyl group, a dioxanyl group, etc. are used.

These heterocyclic groups may be substituted at possible positions by 1 to 3 substituents selected from C₁₋₄ alkyl (e.g., methyl, ethyl, etc.), hydroxy, oxo, C₁₋₄ alkoxy (e.g., methoxy, ethoxy, etc.), etc.

As the "aromatic hydrocarbon group" of the "aromatic hydrocarbon group optionally having substituents" represented by Ar, Ar^{a}, an aromatic hydrocarbon group having 6 to 14 carbon atoms (e.g., a phenyl group, a naphthyl group, a biphenyl group, an anthryl group, an indenyl group and the like) and the like, and particularly, for example, an aryl group having 6 to 10 carbon atoms (e.g., phenyl and naphthyl groups) and the like are preferred and a phenyl group and the like are particularly preferred.

As the "substituent" of the "aromatic hydrocarbon group optionally having substituents" represented by Ar, Ar^{a}, for example, a halogen atom (e.g., fluorine, chlorine, bromine, iodine and the like), a lower (C₁₋₄) alkyl group (e.g., a methyl group, an ethyl group, a propyl group, a butyl group and the like), a lower (C₁₋₄) alkoxy group (e.g., a methoxy group, an ethoxy group, a propoxy group, a butoxy group and the like), a lower (C₁₋₄) alkoxycarbonyl group (e.g., a methoxycarbonyl group, an ethoxycarbonyl group, a propoxycarbonyl group, a butoxycarbonyl group and the like), a carboxyl group, a nitro group, a cyano group, a hydroxyl group, an acylamino group (e.g., an alkanoylamino group having 1 to 4 carbon atoms such as an acetylamino group, a propionylamino group, a butyrylamino group and the like), a cycloalkyl group having 3 to 6 carbon atoms (e.g., a cyclopropyl group, a cyclopentyl group and the like), an aryl group having 6 to 10 carbon atoms (e.g., a phenyl group, a naphthyl group, an indenyl group and the like), a halogeno-lower (C₁₋₄) alkyl group (e.g., a trifluoromethyl group, a trifluoroethyl group and the like), a halogeno-lower (C₁₋₄) alkoxy group (e.g., a trifluoromethoxy group, a 1,1,2,2-tetrafluoroethoxy group, a 2,2,3,3,3-pentafluoropropoxy group and the like), a lower (C₁-₄) alkylthio group (e.g., a methylthio group, an ethylthio group, a propionylthio group and the like), a lower (C₁₋₄) alkylsulfonyl group (e.g., a methanesulfonyl group, an ethanesulfonyl group, a propanesulfonyl group and the like), a lower (C₁₋₄) alkanoyl group (e.g., a formyl group, an acetyl group, a propionyl group and the like), a 5-membered aromatic heterocyclic group (e.g., a 1,2,3-triazolyl group, a 1,2,4-triazolyl group, a tetrazolyl group, a thiazolyl group, an isothiazolyl group, an oxazolyl group, an isooxazolyl group, a thiadiazolyl group, a thienyl group, a furyl group and the like), a carbamoyl group, a lower (C₁₋₄) alkyl-carbamoyl group (e.g., a methylcarbamoyl group, a dimethylcarbamoyl group, a propionylcarbamoyl group and the like), a lower (C₁₋₄) alkoxy-carbonyl-lower (C₁₋₄) alkyl-carbamoyl group (e.g., a butoxycarbonylmethylcarbamoyl group, an ethoxycarbonylmethylcarbamoyl group and the like), a 1,3-diacylguanidino-lower (C₁₋₄) alkyl group (e.g., 1,3-diacetylguanidinomethyl, 1,3-bis-tert-butoxycarbonylguanidinomethyl and the like) and the like are used, and a halogen atom (e.g., fluorine, chlorine, bromine, iodine atoms and the like), a lower (C₁₋₄) alkyl group (e.g., a methyl group, an ethyl group, a propyl group, a butyl group and the like) and the like are preferably used, and a fluorine atom, a chlorine atom and a methyl group are more preferably used.

These substituents are substituted at substitutable positions in the aromatic hydrocarbon group, and the number of the substituents is preferably 1 to 5, more preferably 1 to 3, most preferably 1 to 2. When two or more of such substituents are present, they may be the same or different.

Specifically, as Ar or Ar^{a}, for example, a phenyl group, a halogenophenyl group, a lower (C₁₋₄) alkylphenyl group, a lower (C₁₋₄) alkoxyphenyl group, a lower (C₁₋₄) alkoxycarbonylphenyl group, a carboxylphenyl group, a nitrophenyl group, a cyanophenyl group, a halogeno-lower (C₁₋₄) alkylphenyl group, a halogeno-lower (C₁₋₄) alkoxyphenyl group, a lower (C₁₋₄) alkanoylphenyl group, a 5-membered aromatic heterocycle-substituted phenyl group, a lower (C₁₋₄) alkoxy-carbonyl-lower (C₁₋₄) alkyl-carbamoylphenyl group, 1,3-diacylguanidino-lower (C₁₋₄) alkylphenyl group, a halogen- and lower (C₁₋₄) alkyl-substituted phenyl group, a halogen- and lower (C₁₋₄) alkoxycarbonyl-substituted phenyl group, a halogen- and cyano-substituted phenyl group, a halogen- and 5-membered aromatic heterocycle-substituted phenyl group, a halogen- and lower (C₁₋₄) alkoxycarbonyl-lower (C₁₋₄) alkyl-carbamoyl-substituted phenyl group and the like are used.

As Ar or Ar^{a}, a halogenophenyl group, a lower (C₁₋₄) alkylphenyl group, a halogen- and lower (C₁₋₄) alkoxycarbonyl-substituted phenyl and the like are preferably used.

As Ar or Ar^{a}, a group represented by formula: 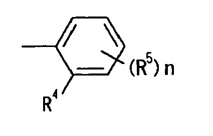 [wherein R⁴ and R⁵ is the same or different and each represents a halogen atom or a lower alkyl group, and n represents an integer of 0 to 2] is particularly preferred, and one in which at least one of R⁴ and R⁵ is a halogen atom is further preferred.

As the halogen atom represented by R⁴ and R⁵, a fluorine atom or a chlorine atom is preferred.

As the halogenophenyl group, for example, a 2,3-difluorophenyl group, a 2,3-dichlorophenyl group, a 2,4-difluorophenyl group, a 2,4-dichlorophenyl group, a 2,5-difluorophenyl group, a 2,5-dichlorophenyl group, a 2,6-difluorophenyl group, a 2,6-dichlorophenyl group, a 3,4-difluorophenyl group, a 3,4-dichlorophenyl group, a 3,5-difluorophenyl group, a 3,5-dichlorphenyl group, a 2-fluorophenyl group, a 2-chlorophenyl group, a 3-fluorophenyl group, a 3-chlorophenyl group, a 4-fluorophenyl group, a 4-chlorophenyl group, a 2-fluoro-4-chlorophenyl group, a 2-chloro-4-fluorophenyl group, a 4-bromo-2-fluorophenyl group, a 2,3,4-trifluorophenyl group, a 2,4,5-trifluorophenyl group, a 2,4,6-trifluorohenyl group and the like are used.

As the lower (C₁₋₄) alkylphenyl group, a 2-ethylphenyl group, a 2,6-diisopropylphenyl group and the like are preferably used, and as the lower (C₁₋₄) alkoxyphenyl group, for example, a 4-methoxyphenyl group and the like are preferably used.

As the lower (C₁₋₄) alkoxy-carbonylphenyl group, a 2-ethoxycarbonylphenyl group, a 2-methoxycarbonylphenyl group, a 4-methoxycarbonylphenyl group and the like are preferably used, and as the halogeno-lower (C₁₋₄) alkylphenyl group, for example, a 2-trifluoromethylphenyl group and the like are preferably used, and as the halogeno-lower (C₁₋₄) alkoxyphenyl group, for example, a 2-trifluoromethoxyphenyl group, a 4-(2,2,3,3,3-pentafluoropropoxy)phenyl group and the like are preferably used.

As the lower (C₁₋₄) alkanoylphenyl group, for example, a 2-acetylphenyl group and the like are preferably used, and as the 5-membered aromatic heterocycle-substituted phenyl, for example, a 4-(2H-1,2,3-triazol-2-yl)phenyl group, a 4-(2H-tetrazol-2-yl)phenyl group, a 4-(1H-tetrazol-1-yl)phenyl group, a 4-(1H-1,2,3-triazol-1-yl)phenyl group and the like are preferably used, and as the lower (C₁₋₄) alkoxy-carbonyl-lower (C₁₋₄) alkyl-carbamoylphenyl group, for example, a 4-(N-ethoxycarbonylmethylcarbamoyl) phenyl group and the like are preferably used, and as the 1,3-diacylguanidino-lower (C₁₋₄) alkylphenyl group, for example, a 4-(1,3-bis-tert-butoxycarbonylguanidinomethyl)phenyl group and the like are preferably used.

As the phenyl group substituted by the halogen and lower (C₁₋₄) alkyl, for example, a 2-fluoro-4-methylphenyl group, a 2-chloro-4-methylphenyl group, a 4-fluoro-2-methylphenyl group and the like are preferably used, and as the phenyl group substituted by the halogen and lower (C₁₋₄) alkoxycarbonyl, for example, a 2-chloro-4-methoxycarbonylphenyl group and the like are preferably used, and the phenyl group substituted by halogen and cyano, for example, a 2-chloro-4-cyanophenyl group and the like are preferably used, and as the phenyl group substituted by halogen and 5-membered aromatic heterocycle, for example, a 2-fluoro-4-(1H-1,2,4-triazol-1-yl)phenyl group and the like are preferably used, and as the phenyl group substituted by halogen and lower (C₁₋₄) alkoxycarbonyl-lower (C₁₋₄) alkyl-carbamoyl, for example, a 2-chloro-4-(N-tert-butoxycarbonylmethylcarbamoyl)phenyl group, a 2-chloro-4-(N-ethoxycarbonylmethylcarbamoyl) phenyl group and the like are preferably used.

More specifically, as Ar or Ar^{a}, a phenyl group, a phenyl group substituted with 1 to 3 (particularly 1 to 2) halogen atoms (e.g., a 2,3-difluorophenyl group, a 2,3-dichlorophenyl group, a 2,4-difluorophenyl group, a 2,4-dichlorophenyl group, a 2,5-difluorophenyl group, a 2,5-dichlorophenyl group, a 2,6-difluorophenyl group, a 2,6-dichlorophenyl group, a 3,4-difluorophenyl group, a 3,4-dichlorophenyl group, a 3,5-difluorophenyl group, a 3,5-dichlorophenyl group, a 4-bromo-2-fluorophenyl group, a 2-fluorophenyl group, a 2-chlorophenyl group, a 3-fluorophenyl group, a 3-chlorophenyl group, a 4-fluorophenyl group, a 4-chlorophenyl group, a 2-fluoro-4-chlorophenyl group, a 2-chloro-4-fluorophenyl group, a 2,3,4-trifluorophenyl group, a 2,4,5-trifluorophenyl group and the like), a phenyl group substituted by halogen and lower (C₁₋₄) alkyl (e.g., a 2-chloro-4-methylphenyl group, a 4-fluoro-2-methylphenyl group and the like), etc. are preferred. Among them, a phenyl group substituted with 1 to 3 (particularly 1 to 2) halogen atoms (e.g., a 2,3-dichlorophenyl group, a 2,4-difluorophenyl group, a 2,4-dichlorophenyl group, a 2,6-diclorophenyl group, a 2-fluorophenyl group, a 2-chlorophenyl group, a 3-chlorophenyl group, a 2-chloro-4-fluorophenyl group, a 2,4,5-trifluorophenyl group and the like), a phenyl group substituted by halogen and lower (C₁₋₄) alkyl (e.g., a 2-chloro-4-methylphenyl group, a 4-fluoro-2-methylphenyl group and the like), etc. are preferred. Particularly, a 2,4-difluorophenyl group, a 2-chlorophenyl group, a 2-chloro-4-fluorophenyl group, a 2-chloro-4-methylphenyl group and the like are preferred, and a 2,4-difluorophenyl group, a 2-chloro-4-fluorophenyl group and the like are preferred.

In this specification, the ring A¹ represents a cycloalkene optionally substituted by 1 to 4 substituent(s) selected from the group consisting of (1) an aliphatic hydrocarbon group optionally having substituents, (2) an aromatic hydrocarbon group optionally having substituents, (3) a group represented by the formula -OR¹ (wherein R¹ represents the same meaning as mentioned above) and (4) a halogen atom, and a cycloalkene optionally substituted by 1 to 4 substituent(s) selected from the group consisting of (1) an aliphatic hydrocarbon group optionally having substituents, (2) an aromatic hydrocarbon group optionally having substituents, and (4) a halogen atom is preferred.

In this specification, the ring A² represents a cycloalkene substituted by 1 to 4 substituent(s) selected from the group consisting of (1) an aliphatic hydrocarbon group optionally having substituents, (2) an aromatic hydrocarbon group optionally having substituents, (3) a group represented by the formula -OR¹ (wherein R¹ represents the same meaning as mentioned above) and (4) a halogen atom, and a cycloalkene substituted by 1 to 4 substituent(s) selected from the group consisting of (1) an aliphatic hydrocarbon group optionally having substituents, (2) an aromatic hydrocarbon group optionally having substituents, and (4) a halogen atom is preferred.

These substituents are substituted on substitutable carbon atoms in a ring A¹ or ring A², and when the ring A¹ or A² is substituted by plural number of such substituents, the substituents may be the same or different. A single carbon atom may be substituted by two substituents and different carbon atoms may be substituted by two or more substituents.

As the "aliphatic hydrocarbon group optionally having substituents" as a substituent on the ring A¹ and ring A², for example, the same substituents as the "aliphatic hydrocarbon group optionally having substituents" represented by R, R^{a}, R¹, R^{1a}, R^{1b}, R^{1c} described above may be used.

As the "aromatic hydrocarbon group optionally having substituents" as a substituent on the ring A¹ and ring A², for example, the same substituents as the "aromatic hydrocarbon group optionally having substituents" represented by Ar or Ar^{a} described above may be used.

As the substituents for the ring A¹ and ring A², 1 or 2 C₁₋₆ alkyl group (e.g., a C₁₋₄ alkyl group such as a methyl group, a tert-butyl group, etc.), a phenyl group, a halogen atom (e.g., fluorine, chlorine, bromine, iodine, etc.), etc. are preferably used.

The group represented by the formula: 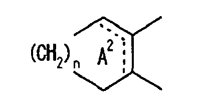 [wherein n represents the same meaning as defined above], can be a group represented by the formula: 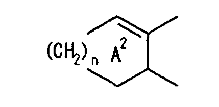 or a group represented by the formula: 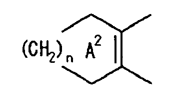 [wherein n represents the same meaning as defined above], and a group represented by the formula: 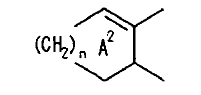 [wherein n represents the same meaning as defined above], is more preferred.
The group represented by the formula: 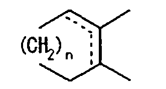 [wherein n represents the same meaning as defined above], can be a group represented by the formula: 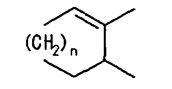 or a group represented by the formula: 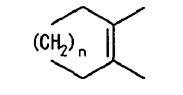 [wherein n represents the same meaning as defined above], and a group represented by the formula: 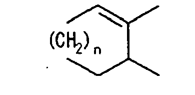 [wherein n represents the same meaning as defined above], is more preferred.
A group represented by the formula: 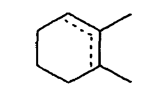 can be a group represented by the formula: 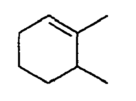 or a group represented by the formula: 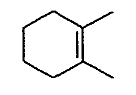 and a group represented by the formula: 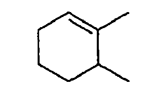 is more preferred.

As the integer of 1 to 4 represented by n, 1 to 3 is preferred and 2 is more preferred.

As the compound represented by the formula (Iaa), the compound represented by the formula (Ibb) is preferred, and as the compound represented by the formula (Ia), the compound represented by the formula (Ib) is preferred.

As the compound represented by the formula (Ibb), the compound represented by the formula (Inn) is preferred, and as the compound represented by the formula (Ib), the compound represented by the formula (In) is preferred.

As the compound (Ibb), (Ib), a compound wherein R¹ is a lower alkyl group optionally having substituents, R² is a hydrogen atom or a lower alkyl group, Ar is a phenyl group optionally having substituents, n is 1, 2 or 3 is preferred, and a compound wherein R¹ is a lower alkyl group optionally having substituents, R² is a hydrogen atom, Ar is a phenyl group substituted by a halogen atom, n is 2 is more preferred,

As the compound represented by the formula (Icc), (Ic), a compound wherein Ar is a phenyl group optionally having substituents, n is 2 is preferred.

As the leaving group represented by X¹, for example, a halogen atom (e.g., chlorine, bromine, iodine, etc.), etc. are preferred and a chlorine atom is more preferred.

When the compounds represented by formulae (I), (Iaa), (Ibb), (Icc), (Ia), (Ib), (Ic), (Id), (Ie), (If) and (Ig) have stereoisomers, any of such stereoisomers and mixtures thereof are included in the invention.

In addition, when a compound represented by formula (Iaa) is a compound represented by formula (Icc) or (Inn), when a compound represented by formula (Ia) is a compound represented by formula (Ic) or (In), when a compound represented by formula (Ie) is a compound represented by formula (Ik) or (Ip), when a compound represented by formula (Id) is a compound represented by formula (Ir), and when a compound represented by formula (Ig) is a compound represented by formula (It), then each compound can exist as an optical isomer with regard to the asymmetric carbon atom in a cycloalkene or cyclohexene ring, and any of such optical isomers and mixtures thereof are included in the invention.

As a compound represented by formula (I) or (Ia), specifically, the compounds obtained in Reference Example B described later and the like are used, and among them, <1>d-ethyl 6-[N-(2,4-difluorophenyl)sulfamoyl]-1-cyclohexene-1-carboxylate (d-ethyl6-[N-(2,4-difluorophenyl)sulfamoyl]cyclohex-1-en-1-carboxylate) <2>ethyl 6-[N-(2-chlorophenyl)sulfamoyl]-1-cyclohexene-1-carboxylate (ethyl 6-[N-(2-chlorophenyl)sulfamoyl]cyclohex-1-en-1-carboxylate) <3>ethyl 6-[N-(2-chloro-4-methylphenyl)sulfamoyl]-1-cyclohexene-1-carboxylate (ethyl 6-[N-(2-chloro-4-methylphenyl)sulfamoyl] cyclohex-1-en-1-carboxylate) or <4>d-ethyl 6-[N-(2-chloro-4-fluorophenyl)sulfamoyl]-1-cyclohexene-1-carboxylate (ethyl (6R)6-[N-(2-chloro-4-fluorophenyl)sulfamoyl] cyclohex-1-en-1-carboxylate), as well as a salt thereof are preferable.

The compounds (I), (Iaa), (Ia), (Ib), (Ic), (Id), (Ie), (If), (Ig), (Ibb) and (Icc) (hereinafter simply referred to as an inventive Compound) used in the composition of the present invention may, for example, be converted into a salt with an inorganic base, organic base, inorganic acid, organic acid, basic or acidic amino acid. A salt with an inorganic base may, for example, be an alkaline metal salt such as sodium and potassium salts, an alkaline earth metal salt such as calcium and magnesium salts, aluminum and ammonium salts, and a salt with an organic base may, for example, be a salt with trimethylamine, triethylamine, pyridine, picoline, ethanolamine, diethanolamine, triethanolamine, dicyclohexylamine or N,N'-dibenzylethylenediamine. A salt with an inorganic acid may, for example, be a salt with hydrochloric acid, hydrobromic acid, nitric acid, sulfuric acid or phosphoric acid, and a salt with an organic acid may, for example, be a salt with formic acid, acetic acid, trifluoroacetic acid, fumaric acid, oxalic acid, tartaric acid, maleic acid, citric acid, succinic acid, malic acid, methanesulfonic acid, benzenesulfonic acid or p-toluenesulfonic acid. A salt with a basic amino acid may, for example, be a salt with arginine, lysine or ornithine, and a salt with acidic amino acid may, for example, be a salt with aspartic acid or glutamic acid.

The inventive Compound can be produced according to a method known per se, for example, a production method described in WO99/46242.

### (B) About a formula (II) compound

In the specification, R^{1'} represents an aliphatic hydrocarbon group optionally having substituents, an aromatic hydrocarbon group optionally having substituents, a heterocyclic group optionally having substituents, a group represented by the formula OR^{1a'} or a group represented by the formula: 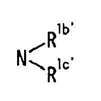 and among them, the group represented by the formula OR^{1a'} is preferred.

As the "aliphatic hydrocarbon group" of the "aliphatic hydrocarbon group optionally having substituents" represented by R¹', for example, an alkyl group, a cycloalkyl group, a cycloalkylalkyl group, an alkenyl group, an alkynyl group, etc. are preferred.

As the alkyl group, for example, a linear or branched alkyl group having 1 to 20 carbons (e.g., a methyl group, an ethyl group, an n-propyl group, a isopropyl group, an n-butyl group, a isobutyl group, a sec-butyl group, a tert-butyl group, a pentyl group, a hexyl group, a heptyl group, an octyl group, a nonyl group, a decyl group, a dodecyl group, etc.), etc. are preferred, and particularly, for example, a lower alkyl group having 1 to 6 carbons (e.g., a methyl group, an ethyl group, an n-propyl group, a isopropyl group, an n-butyl group, a isobutyl group, a sec-butyl group, a tert-butyl group, etc.), etc. are preferred.

As the cycloalkyl group, for example, a cycloalkyl group having 3 to 10 carbons (e.g., a cyclopropyl group, a cyclobutyl group, a cyclopentyl group, a cyclohexyl group, a cycloheptyl group, a cyclooctyl group, etc.), etc. are preferred, and particularly, for example, a cycloalkyl group having 3 to 6 carbons (e.g., a cyclopropyl group, a cyclobutyl group, a cyclopentyl group, a cyclohexyl group, etc.), etc. are preferred.

As the cycloalkylalkyl group, for example, a cycloalkylalkyl group having 4 to 12 carbons (e.g., a cyclopropylmethyl group, a cyclopentylmethyl group, a cyclohexylmethyl group, a cycloheptylmethyl group, etc.), etc. are preferred, and particularly, for example, a cycloalkylalkyl group having 4 to 8 (particularly, 4 to 7) carbons (e.g., a cyclopropylmethyl group, a cyclopentylmethyl group, a cyclohexylmethyl group, etc.), etc. are preferred.

As the alkenyl group, for example, a lower alkenyl group having 3 to 6 carbons (e.g., a propenyl group, a butenyl group, a pentenyl group, etc.), and particularly, for example, a lower alkenyl group having 3 or 4 carbons (e.g., a propenyl group, a butenyl group, etc.), etc. are preferred.

As the alkynyl group,for example, a lower alkynyl group having 3 to 6 carbons (e.g., a propynyl group, a butynyl group, a pentynyl group, etc.), and particularly, for example, a lower alkynyl group having 3 or 4 carbons (e.g., a propynyl group, a butynyl group, etc.), etc. are preferred.

As the "substituents" of the above mentioned "aliphatic hydrocarbon group optionally having substituents", for example, a heterocyclic group, an oxo group, a hydroxy group, a C₁₋₆ alkoxy group, a C₃₋₁₀ (particularly, C₃₋₆) cycloalkyloxy group, a C₆₋₁₀ aryloxy group, a C₇₋₁₉ (particularly, C₇₋₁₂) aralkyloxy group, a heterocyclic oxy group, a C₁₋₆ alkylthio group (the sulfur atom may be oxidized), a C₃₋₁₀ (particularly, C₃₋₆) cycloalkylthio group (the sulfur atom may be oxidized), a C₆₋₁₀ arylthio group (the sulfur atom may be oxidized), a C₇₋₁₉ (particularly, C₇₋₁₂) aralkylthio group (the sulfur atom may be oxidized), a heterocyclic thio group, a heterocyclic sulfinyl group, a heterocyclic sulfonyl group, a nitro group, a halogen atom, a cyano group, a carboxyl group, a C₁₋₁₀ (particularly, C₁₋₆) alkoxy-carbonyl group, a C₃₋₆ cycloalkyloxy-carbonyl group, a C₆₋₁₀ aryloxy-carbonyl group, a C₇₋₁₉ (particularly, C₇₋₁₂) aralkyloxy-carbonyl group, a heterocyclic oxycarbonyl group, a C₆₋₁₀ aryl-carbonyl group, C₁₋₆ alkanoyl group, C₃₋₅ alkenoyl group, a C₆₋₁₀ aryl-carbonyloxy group, a C₂₋₆ alkanoyloxy group, a C₃₋₅ alkenoyloxy group, a carbamoyl group optionally having substituents, a thiocarbamoyl group optionally having substituents, a carbamoyloxy group optionally having substituents, a C₁₋₆ alkanoylamino group, a C₆₋₁₀ aryl-carbonylamino group, a C₁₋₁₀ (particularly, C₁₋₆) alkoxy-carboxamide group, a C₆₋₁₀ aryloxy-carboxamide group, a C₇₋₁₉ (particularly, C₇₋₁₂) aralkyloxy-carboxamide group, a C₁₋₁₀ (particularly, C₁₋₆) alkoxy-carbonyloxy group, a C₆₋₁₀ aryloxy-carbonyloxy group, a C₇₋₁₉ (particularly, C₇₋₁₂) aralkyloxy-carbonyloxy group, a C₃₋₁₀ (particularly, C₃₋₆)cycloalkyloxy-carbonyloxy group, an ureido group optionally having substituents, a C₆₋₁₀ aryl group optionally having substituents, etc. are used.

These substituents are substituted at substitutable positions in the above mentioned "aliphatic hydrocarbon group", and the substituents are not limited to one and may be same or different and a few numbers (2 to 4).

As the "C₁₋₆ alkoxy group", for example, a methoxy group, an ethoxy group, an n-propoxy group, a isopropoxy group, an n-butoxy group, a tert-butoxy group, an n-pentyloxy group, an n-hexyloxy group, etc. are used, as the "C₃₋₁₀ cycloalkyloxy group", for example, a cyclopropyloxy group, a cyclohexyloxy group, etc. are used, as the "C₆₋₁₀ aryloxy group", for example, a phenoxy group, a naphthyloxy group, etc. are used, as the "C₇₋₁₉ aralkyloxy group", for example, a benzyloxy group, a 1-phenylethyloxy group, a 2-phenylethyloxy group, a benzhydryloxy group, a 1-naphthylmethyloxy group, etc. are used, as the "C₁₋₆ alkylthio group (the sulfur atom may be oxidized)", for example, a methylthio group, an ethylthio group, an n-propylthio group, an n-butylthio group, a methylsulfinyl group, a methylsulfonyl group, etc. are used, as the "C₃₋₁₀ cycloalkylthio group (the sulfur atom may be oxidized)", for example, a cyclopropylthio group, a cyclohexylthio group, a cyclopentylsulfinyl group, a cyclohexylsulfonyl group, etc. are used, as the "C₆₋₁₀ arylthio group (the sulfur atom may be oxidized)", for example, a phenylthio group, a naphthylthio group, a phenylsulfinyl group, a phenylsulfonyl group, etc. are used, as the "C₇₋₁₉ aralkylthio group (the sulfur atom may be oxidized)", for example, a benzylthio group, a phenylethylthio group, a benzhydrylthio group, a benzylsulfinyl group, a benzylsulfonyl group, etc. are used, as the "halogen atom", for example, a fluorine atom, a chlorine atom, a bromine atom, an iodine atom, ets. are used, as the "C₁₋₁₀ alkoxy-carbonyl group", for example, a methoxycarbonyl group, an ethoxycarbonyl group, an n-propoxycarbonyl group, an isopropoxycarbonyl group, an n-butoxycarbonyl group, an isobutoxycarbonyl group, a tert-butoxycarbonyl group, etc. are used, as the "C₃₋₆ cycloalkyloxycarbonyl group", for example, a cyclopropyloxycarbonyl group, a cyclopentyloxycarbonyl group,a cyclohexyloxycarbonyl group, a norbornyloxycarbonyl group, etc. are used, as the "C₆₋₁₀ aryloxy-carbonyl group", for example, a phenoxycarbonyl group, a naphthyloxycarbonyl group, etc. are used, as the "C₇₋₁₉ aralkyl-oxycarbonyl group", for example, a benzyloxycarbonyl group, a benzhydryloxycarbonyl group, a 2-phenethyloxycarbonyl group, etc. are used, as the "C₆₋₁₀ aryl-carbonyl group", for example, a benzoyl group, a naphthoyl group, a phenylacetyl group, etc. are used, as the "C₁₋₆ alkanoyl group", for example, a formyl group, an acetyl group, a propionyl group, a butyryl group, a valeryl group, a pivaloyl group, etc. are used, as the "C₃₋₅ alkenoyl group", for example, an acryloyl group, a crotnoyl group, etc. are used, as the "C₆₋₁₀ aryl-carbonyloxy group", for example, a benzoyloxy group, a naphthoyloxy group, a phenylacetoxy group, etc. are used, as the "C₂₋₆ alkanoyloxy group", for example, an acetoxy group, a propionyloxy group, a butyryloxy group, a valeryloxy group, a pivaloyloxy group, etc. are used, as the "C₃₋₅ alkenoyloxy group", for example, an acryloyloxy group, a crotnoyloxy group, etc. are used.

As the "carbamoyl group optionally having substituents", for example, a carbamoyl group or a cyclic aminocarbonyl group, which may be substituted by 1 or 2 substituents selected from C₁₋₄ alkyl (e.g., methyl, ethyl, etc.), phenyl, C₁₋₇ acyl (e.g., acetyl, propionyl, benzoyl, etc.), C₁₋₄ alkoxy-phenyl (e.g., methoxyphenyl, etc.), etc. and specifically for example a carbamoyl group, an N-methylcarbamoyl group, an N-ethylcarbamoyl group, an N,N-dimethylcarbamoyl group, an N,N-diethylcarbamoyl group, an N-phenylcarbamoyl group, an N-acetylcarbamoyl group, an N-benzoylcarbamoyl group, an N-(p-methoxyphenyl)carbamoyl group, a 1-pyrrolidinylcarbonyl group, a piperidinocarbonyl group, a 1-piperazinylcarbonyl group, a morpholinocarbamoyl group, etc. are used. As the "thiocarbamoyl group optionally having substituents", for example, a thiocarbamoyl group which may be substituted by 1 or 2 substituents selected from C₁₋₄ alkyl (e.g., methyl, ethyl, etc.), phenyl, etc., and specifically for example a thiocarbamoyl group, an N-methylthiocarbamoyl group, an N-phenylthiocarbamoyl group, etc. are used. As the "carbamoyloxy group optionally having substituents", for example, a carbamoyloxy group which may be substituted by 1 or 2 substituents selected from C₁₋₄ alkyl (e.g., methyl, ethyl, etc.), phenyl, etc. and specifically for example a carbamoyloxy group, an N-methylcarbamoyloxy group, an N,N-dimethylcarbamoyloxy group, an N-ethylcarbamoyloxy group, an N-phenylcarbamoyloxy group, etc. are used.

As the "C₁₋₆ alkanoylamino group", for example, an acetoamide group, a propionamide group, a butyroamide group, a valeroamide group, a pivaroamide group, etc. are used, as the "C₆₋₁₀ aryl-carbonylamino group", for example, a benzamide group, a naphthoamide group, a phthalimide group, etc. are used, as the "C₁₋₁₀ alkoxy-carboxamide group", for example, a methoxycarboxamide (CH₃OCONH-) group, an ethoxycarboxamide group, a tert-butoxycarboxamide group, etc. are used, as the "C₆₋₁₀ aryloxy-carboxamide group", for example, a phenoxycarboxamide (C₆H₅OCONH-) group, etc. are used, as the "C₇₋₁₉ aralkyloxy-carboxamide group", for example, a benzyloxycarboxamide (C₆H₅CH₂OCONH-) group, a benzhydryloxycarboxamide group, etc. are used, as the "C₁₋₁₀ alkoxy-carbonyloxy group", for example, a methoxycarbonyloxy group, an ethoxycarbonyloxy group, an n-propoxycarbonyloxy group, a isopropoxycarbonyloxy group, an n-butoxycarbonyloxy group, a tert-butoxycarbonyloxy group, an n-pentyloxycarbonyloxy group, an n-hexyloxycarbonyloxy group, etc. are used, as the "C₆₋₁₀ aryloxy-carbonyloxy group", for example, a phenoxycarbonyloxy group, a naphthyloxycarbonyloxy group, etc. are used, as the "C₇₋₁₉ aralkyloxy-carbonyloxy group", for example, a benzylnoxycarbonyloxy group, a 1-phenylethyloxycarbonyloxy group, a 2-phenylethyloxycarbonyloxy group, a benzhydryloxycarbonyloxy group, etc. are used, and as the "C₃₋₁₀ cycloalkyloxy-carbonyloxy group", for example, a cyclopropyloxycarbonyloxy group, a cyclohexyloxycarbonyloxy group, etc. are used.

As the "ureido group optionally having substituents", for example, an ureido group optionally substituted by 1 to 3 (particularly, 1 or 2) substituents selected from a C₁₋₄ alkyl group (e.g., a methyl group, an ethyl group, etc.), a phenyl group, etc. are used, and for example an ureido group, a 1-methylureido group, a 3-methylureido group, a 3,3-dimethylureido group, a 1,3-dimethylureido group, a 3-phenylureido group, etc. used.

When a heterocyclic group, a heterocyclic oxy group, a heterocyclic thio group, a heterocyclic sulfinyl group, a heterocyclic sulfonyl group or a heterocyclic oxycarbonyl group is used as the "substituents" of the "aliphatic hydrocarbon group optionally having substituents", the heterocyclic group represents a group formed by excluding one hydrogen atom which binds to the heterocycle, and it represents, for example, a 5- to 8-membered cyclic (preferably 5- to 6-membered cyclic) group containing 1 to a few, preferably 1 to 4 hetero atoms such as a nitrogen atom (optionally oxidized), an oxygen atom, a sulfur atom, etc., or a condensed cyclic group thereof. As these heterocyclic group, for example, a pyrrolyl group, a pyrazolyl group, an imidazolyl group, a 1,2,3-triazolyl group, a 1,2,4-triazolyl group, a tetrazolyl group, a furyl group, a thienyl group, an oxazolyl group, an isoxazolyl group, a 1,2,3-oxadiazolyl group, a 1,2,4-oxadiazolyl group, a 1,2,5-oxadiazolyl group, a 1,3,4-oxadiazolyl group, a thiazolyl group, an isothiazolyl group, a 1,2,3-thiadiazolyl group, a 1,2,4-thiadiazolyl group, a 1,2,5-thiadiazolyl group, a 1,3,4-thiadiazolyl group, a pyridyl group, a pyridazinyl group, a pyrimidinyl group, a pyrazinyl group, an indolyl group, a pyranyl group, a thiopyranyl group, a dioxynyl group, a dioxolyl group, a quinolyl group, a pyrido[2,3-d]pyrimidyl group, 1,5-, 1,6-, 1,7-, 1,8-, 2,6- or 2,7-naphthyridyl group, a thieno[2,3-d]pyridyl group, a benzpyranyl group, a tetrahydrofuryl group, a tetrahydropyranyl group, a dioxolanyl group, a dioxanyl group, etc. are used.

These heterocyclic groups may be substituted at possible positions by 1 to 3 substituents selected from C₁₋₄ alkyl (e.g., methyl, ethyl, etc.), hydroxy, oxo, C₁₋₄ alkoxy (e.g., methoxy, ethoxy, etc.), etc.

As the "C₆₋₁₀ aryl group" of the "C₆₋₁₀ aryl group optionally having substituents", for example, a phenyl group, a naphthyl group, etc. are used. The C₆₋₁₀ aryl group may be substituted at a substitutable position by a substituent selected from the those listed as a "substituent" (except for an optionally substituted C₆₋₁₀ aryl group) of the "aliphatic hydrocarbon group optionally having substituents" described above. Such a substituent is substituted at a substitutable position in the C₆₋₁₀ aryl group, and the number of such substituents is not limited to one, and, the same or different, more than one (2 to 4) substituents may exist.

Additionally, in the "aliphatic hydrocarbon group optionally having substituents", the substituent together with the aliphatic hydrocarbon group may form an optionally substituted condensed ring group, and as these condensed ring groups, an indanyl group, a 1,2,3,4-tetrahydronaphthyl group, etc. are used. This condensed ring group may be substituted at a substitutable position by a substituent selected from the those listed as a "substituent" of the "aliphatic hydrocarbon group optionally having substituents" described above. Such a substituent is substituted at a substitutable position in a fused ring group, and the number of such substituents is not limited to one, and, the same or different, more than one (2 to 4) substituents may exist.

As the "aromatic hydrocarbon group" of the "aromatic hydrocarbon group optionally having substituents" represented by R^{1'}, an aromatic hydrocarbon group having 6 to 14 carbon atoms (e.g., a phenyl group, a naphthyl group, a biphenyl group, an anthryl group, an indenyl group and the like) and the like, and particularly an aryl group having 6 to 10 carbon atoms (e.g., phenyl and naphthyl groups) and the like are preferred and a phenyl group and the like are particularly preferred.

As the "substituent" of the "aromatic hydrocarbon group optionally having substituents" represented by R¹', for example, a halogen atom (e.g., fluorine, chlorine, bromine, iodine and the like), a lower (C₁₋₄) alkyl group (e.g., a methyl group, an ethyl group, a propyl group, a butyl group and the like), a lower (C₁₋₄) alkoxy group (e.g., a methoxy group, an ethoxy group, a propoxy group, a butoxy group and the like), a lower (C₁₋₄) alkoxycarbonyl group (e.g., a methoxycarbonyl group, an ethoxycarbonyl group, a propoxycarbonyl group, a butoxycarbonyl group and the like), a carboxyl group, a nitro group, a cyano group, a hydroxyl group, an acylamino group (e.g., an alkanoylamino group having 1 to 4 carbon atoms such as an acetylamino group, a propionylamino group, a butyrylamino group and the like), a cycloalkyl group having 3 to 6 carbon atoms (e.g., a cyclopropyl group, a cyclopentyl group and the like), an aryl group having 6 to 10 carbon atoms (e.g., a phenyl group, a naphthyl group, an indenyl group and the like), a halogeno-lower (C₁₋₄) alkyl group (e.g., a trifluoromethyl group, a trifluoroethyl group and the like), a halogeno-lower (C₁₋₄) alkoxy group (e.g., a trifluoromethoxy group, a 1,1,2,2-tetrafluoroethoxy group, a 2,2,3,3,3-pentafluoropropoxy group and the like), a lower (C₁₋₄) alkylthio group (e.g., a methylthio group, an ethylthio group, a propionylthio group and the like), a lower (C₁₋₄) alkylsulfonyl group (e.g., a methanesulfonyl group, an ethanesulfonyl group, a propanesulfonyl group and the like), a lower (C₁₋₄) alkanoyl group (e.g., a formyl group, an acetyl group, a propionyl group and the like), a 5-membered aromatic heterocyclic group (e.g., a 1,2,3-triazolyl group, a 1,2,4-triazolyl group, a tetrazolyl group, a thiazolyl group, an isothiazolyl group, an oxazolyl group, an isooxazolyl group, a thiadiazolyl group, a thienyl group, a furyl group and the like), a carbamoyl group, a lower (C₁₋₄) alkyl-carbamoyl group (e.g., a methylcarbamoyl group, a dimethylcarbamoyl group, a propionylcarbamoyl group and the like), a lower (C₁₋₄) alkoxy-carbonyl-lower (C₁₋₄) alkyl-carbamoyl group (e.g., a butoxycarbonylmethylcarbamoyl group, an ethoxycarbonylmethylcarbamoyl group and the like), a 1,3-diacylguanidino-lower (C₁₋₄) alkyl group (e.g., 1,3-diacetylguanidinomethyl, 1,3-bis-tert-butoxycarbonylguanidinomethyl and the like) and the like are used, and a halogen atom (e.g., fluorine, chlorine, bromine, iodine atoms and the like), a lower (C₁₋₄) alkyl group and the like (e.g., a methyl group, an ethyl group, a propyl group, a butyl group and the like) are preferably used, and a fluorine atom, a chlorine atom and a methyl group are more preferably used.

These substituents are substituted at substitutable positions in the aromatic hydrocarbon group, and the number of the substituents is preferably 1 to 5, more preferably 1 to 3, most preferably 1 to 2. When two or more of such substituents are present, they may be the same or different.

The "heterocyclic group" in the "heterocyclic group optionally having substituents" represented by R¹' represent a 5- to 8-membered (preferably 5- to 6-membered) ring group having 1 to several, preferably 1 to 4, hetero atoms such as a nitrogen atom (optionally oxidized), an oxygen atom, a sulfur atom and the like, or a condensed ring group thereof. As these heterocyclic group, for example, a pyrrolyl group, a pyrazolyl group, an imidazolyl group, a 1,2,3-triazolyl group, a 1,2,4-triazolyl group, a tetrazolyl group, a furyl group, a thienyl group, an oxazolyl group, an isoxazolyl group, a 1,2,3-oxadiazolyl group, a 1,2,4-oxadiazolyl group, a 1,2,5-oxadiazolyl group, a 1,3,4-oxadiazolyl group, a thiazolyl group, an isothiazolyl group, a 1,2,3-thiadiazolyl group, a 1,2,4-thiadiazolyl group, a 1,2,5-thiadiazolyl group, a 1,3,4-thiadiazolyl group, a pyridyl group, a pyridazinyl group, a pyrimidinyl group, a pyrazinyl group, an indolyl group, a pyranyl group, a thiopyranyl group, a dioxynyl group, a dioxolyl group, a quinolyl group, a pyrido[2,3-d]pyrimidyl group, 1,5-, 1,6-, 1,7-, 1,8-, 2,6- or 2,7-naphthyridyl group, a thieno[2,3-d]pyridyl group, a benzpyranyl group, a tetrahydrofuryl group, a tetrahydropyranyl group, a dioxolanyl group, a dioxanyl group, etc. are used. These heterocyclic groups may be substituted at possible positions by 1 to 3 substituents selected from C₁₋₄ alkyl (e.g., methyl, ethyl, etc.), hydroxy, oxo, C₁₋₄ alkoxy (e.g., methoxy, ethoxy, etc.), etc.

As the "aliphatic hydrocarbon group optionally having substituents" represented by R^{1a'}, for example, same ones as the "aliphatic hydrocarbon group optionally having substituents" represented by R^{1'} can be used. As the R^{1a'}, for example, a lower alkyl group having 1 to 6 carbon atoms (e.g., a methyl group, an ethyl group, an n-propyl group, an isopropyl group, an n-butyl group, an isobutyl group, a tert-butoxycarbonylmethyl group, a hydroxyethyl group and the like) optionally having substituents, and among them, for example, a methyl group, an ethyl group, an n-propyl group, an isopropyl group, an n-butyl group, an isobutyl group, etc. are preferably used. Particularly, a methyl group, an ethyl group, an n-propyl group and the like, etc. are preferred, and an ethyl group is particularly preferred.

As the "aliphatic hydrocarbon group optionally having substituents" represented by R^{1b'}, R^{1c'}, for example, same ones as the "aliphatic hydrocarbon group optionally having substituents" represented by R^{1'} can be used. As the R^{1b'} and R^{1c'}, for example, a lower alkyl group having 1 to 6 carbon atoms (e.g., a methyl group, an ethyl group, an n-propyl group, an isopropyl group, an n-butyl group, an isobutyl group, a tert-butoxycarbonylmethyl group, a hydroxyethyl group and the like) optionally having substituents, and among them, for example, a methyl group, an ethyl group, an n-propyl group, an isopropyl group, an n-butyl group, an isobutyl group, etc. are preferably used. Particularly, for example, a methyl group, an ethyl group, an n-propyl group and the like, etc. are preferred, and an ethyl group is particularly preferred.

As R^{1'}, for example, a lower alkyl group having 1 to 6 carbon atoms (e.g., a methyl group, an ethyl group, an n-propyl group, an isopropyl group, an n-butyl group, an isobutyl group, a tert-butoxycarbonylmethyl group, a hydroxyethyl group and the like) optionally having substituents, and of them, for example, a methyl group, an ethyl group, an n-propyl group, an isopropyl group, an n-butyl group, an isobutyl group, etc. are preferably used. Particularly, a methyl group, an ethyl group, an n-propyl group and the like, etc. are preferred, and an ethyl group is preferred particularly.

Examples of the substituent of the methylene group optionally having substituents represented by Y, include a C₁₋₆ alkyl group such as a methyl group, an ethyl group, an n-propyl group, an isopropyl group, an n-butyl group, an isobutyl group, etc., a hydroxy substituted-C₁₋₆ alkyl group such as a hydroxymethyl group, a hydroxyethyl group, etc., and a C₁₋₄ alkoxy-carbonyl-C₁₋₄ alkyl group such as a methoxycarbonylmethyl group, an ethoxycarbonylmethyl group, a tert-butoxycarbonylmethyl group, a methoxycarbonylethyl group, an ethoxycarbonylethyl group, a tert-butoxycarbonylethyl group, etc., or the like. Among them, a hydrogen atom and a methyl group is preferred and a hydrogen atom is particularly preferred.

Examples of the substituent of the nitrogen atom optionally having substituents represented by Y, include a C₁₋₆ alkyl group such as a methyl group, an ethyl group, an n-propyl group, an isopropyl group, an n-butyl group, an isobutyl group, etc., a hydroxy substituted-C₁₋₆ alkyl group such as a hydroxymethyl group, a hydroxyethyl group, etc., and a C₁₋₄ alkoxy-carbonyl-C₁₋₄ alkyl group such as a methoxycarbonylmethyl group, an ethoxycarbonylmethyl group, a tert-butoxycarbonylmethyl group, a methoxycarbonylethyl group, an ethoxycarbonylethyl group, a tert-butoxycarbonylethyl group, etc., or the like. Among them, a hydrogen atom and a methyl group is preferred and a hydrogen atom is particularly preferred.

As the "aromatic hydrocarbon group" of the "aromatic hydrocarbon group optionally having substituents" represented by Ar', an aromatic hydrocarbon group having 6 to 14 carbon atoms (e.g., a phenyl group, a naphthyl group, a biphenyl group, an anthryl group, an indenyl group and the like) and the like, and particularly an aryl group having 6 to 10 carbon atoms (e.g., phenyl and naphthyl groups) and the like are preferred and a phenyl group and the like are particularly preferred.

As the "substituent" of the "aromatic hydrocarbon group optionally having substituents" represented by Ar', for example, a halogen atom (e.g., fluorine, chlorine, bromine, iodine and the like), a lower (C₁₋₄) alkyl group (e.g., a methyl group, an ethyl group, a propyl group, a butyl group and the like), a lower (C₁₋₄) alkoxy group (e.g., a methoxy group, an ethoxy group, a propoxy group, a butoxy group and the like), a lower (C₁₋₄) alkoxycarbonyl group (e.g., a methoxycarbonyl group, an ethoxycarbonyl group, a propoxycarbonyl group, a butoxycarbonyl group and the like), a carboxyl group, a nitro group, a cyano group, a hydroxyl group, an acylamino group (e.g., an alkanoylamino group having 1 to 4 carbon atoms such as an acetylamino group, a propionylamino group, a butyrylamino group and the like), a cycloalkyl group having 3 to 6 carbon atoms (e.g., a cyclopropyl group, a cyclopentyl group and the like), an aryl group having 6 to 10 carbon atoms (e.g., a phenyl group, a naphthyl group, an indenyl group and the like), a halogeno-lower (C₁₋₄) alkyl group (e.g., a trifluoromethyl group, a trifluoroethyl group and the like), a halogeno-lower (C₁₋₄) alkoxy group (e.g., a trifluoromethoxy group, a 1,1,2,2-tetrafluoroethoxy group, a 2,2,3,3,3-pentafluoropropoxy group and the like), a lower (C₁₋₄) alkylthio group (e.g., a methylthio group, an ethylthio group, a propionylthio group and the like), a lower (C₁₋₄) alkylsulfonyl group (e.g., a methanesulfonyl group, an ethanesulfonyl group, a propanesulfonyl group and the like), a lower (C₁₋₄) alkanoyl group (e.g., a formyl group, an acetyl group, a propionyl group and the like), a 5-membered aromatic heterocyclic group (e.g., a 1,2,3-triazolyl group, a 1,2,4-triazolyl group, a tetrazolyl group, a thiazolyl group, an isothiazolyl group, an oxazolyl group, an isooxazolyl group, a thiadiazolyl group, a thienyl group, a furyl group and the like), a carbamoyl group, a lower (C₁₋₄) alkyl-carbamoyl group (e.g., a methylcarbamoyl group, a dimethylcarbamoyl group, a propionylcarbamoyl group and the like), a lower (C₁₋₄) alkoxy-carbonyl-lower (C₁₋₄) alkyl-carbamoyl group (e.g., a butoxycarbonylmethylcarbamoyl group, an ethoxycarbonylmethylcarbamoyl group and the like), a 1,3-diacylguanidino-lower (C₁₋₄) alkyl group (e.g., 1,3-diacetylguanidinomethyl, 1,3-bis-tert-butoxycarbonylguanidinomethyl and the like) and the like are used, and a halogen atom (e.g., fluorine, chlorine, bromine, iodine atoms and the like), a lower (C₁₋₄) alkyl group (e.g., a methyl group, an ethyl group, a propyl group, a butyl group and the like) and the like are preferably used, and a fluorine atom, a chlorine atom and a methyl group are more preferably used.

These substituents are substituted at substitutable positions in the aromatic hydrocarbon group, and the number of the substituents is preferably 1 to 5, more preferably 1 to 3, most preferably 1 to 2. When two or more of such substituents are present, they may be the same or different.

Specifically, as Ar', for example, a phenyl group, a halogenophenyl group, a lower (C₁₋₄) alkylphenyl group, a lower (C₁₋₄) alkoxy-phenyl group, a lower (C₁₋₄) alkoxy-carbonylphenyl group, a carboxylphenyl group, a nitrophenyl group, a cyanophenyl group, a halogeno-lower (C₁₋₄) alkyl-phenyl group, a halogeno-lower (C₁₋₄) alkoxy-phenyl group, a lower (C₁₋₄) alkanoyl-phenyl group, a 5-membered aromatic heterocycle-substituted phenyl group, a lower (C₁₋₄) alkoxy-carbonyl-lower (C₁₋₄) alkyl-carbamoylphenyl group, 1,3-diacylguanidino-lower (C₁₋₄) alkyl-phenyl group, a halogen- and lower (C₁₋₄) alkyl-substituted phenyl group, a halogen- and lower (C₁₋₄) alkoxy-carbonyl-substituted phenyl group, a halogen- and cyano-substituted phenyl group, a halogen- and 5-membered aromatic heterocycle-substituted phenyl group, a halogen- and lower (C₁-₄) alkoxycarbonyl-lower (C₁₋₄) alkyl-carbamoyl-substituted phenyl group and the like are used.

As the halogenophenyl group, for example, a 2,3-difluorophenyl group, a 2,3-dichlorophenyl group, a 2,4-difluorophenyl group, a 2,4-dichlorophenyl group, a 2,5-difluorophenyl group, a 2,5-dichlorophenyl group, a 2,6-difluorophenyl group, a 2,6-dichlorophenyl group, a 3,4-difluorophenyl group, a 3,4-dichlorophenyl group, a 3,5-difluorophenyl group, a 3,5-dichlorphenyl group, a 2-fluorophenyl group, a 2-chlorophenyl group, a 3-fluorophenyl group, a 3-chlorophenyl group, a 4-fluorophenyl group, a 4-chlorophenyl group, a 2-fluoro-4-chlorophenyl group, a 2-chloro-4-fluorophenyl group, a 4-bromo-2-fluorophenyl group, a 2,3,4-trifluorophenyl group, a 2,4,5-trifluorophenyl group, a 2,4,6-trifluorohenyl group and the like are used.

As the lower (C₁₋₄) alkyl-phenyl group, for example, a 2-ethylphenyl group, a 2,6-diisopropylphenyl group and the like are preferably used, and as the lower (C₁₋₄) alkoxy-phenyl group, for example, a 4-methoxyphenyl group and the like are preferably used.

As the lower (C₁₋₄) alkoxy-carbonylphenyl group, a 2-ethoxycarbonylphenyl group, a 2-methoxycarbonylphenyl group, a 4-methoxycarbonylphenyl group and the like are preferably used, and as the halogeno-lower (C₁₋₄) alkyl-phenyl group, for example, a 2-trifluoromethylphenyl group and the like are preferably used, and as the halogeno-lower (C₁₋₄) alkoxy-phenyl group, a 2-trifluoromethoxyphenyl group, a 4-(2,2,3,3,3-pentafluoropropoxy)phenyl group and the like are preferably used.

As the lower (C₁₋₄) alkanoyl-phenyl group, for example, a 2-acetylphenyl group and the like are preferably used, and as the 5-membered aromatic heterocycle-substituted phenyl, for example, a 4-(2H-1,2,3-triazol-2-yl)phenyl group, a 4-(2H-tetrazol-2-yl)phenyl group, a 4-(1H-tetrazol-1-yl)phenyl group, a 4-(1H-1,2,3-triazol-1-yl)phenyl group and the like are preferably used, and as the lower (C₁₋₄) alkoxy-carbonyl-lower (C₁₋₄) alkyl-carbamoylphenyl group, for example, a 4-(N-ethoxycarbonylmethylcarbamoyl) phenyl group and the like are preferably used, and as the 1,3-diacylguanidino-lower (C₁₋₄) alkyl-phenyl group, for example, a 4-(1,3-bis-tert-butoxycarbonylguanidinomethyl)phenyl group and the like are preferably used.

As the phenyl group substituted by halogen and lower (C₁-₄) alkyl, for example, a 2-fluoro-4-methylphenyl group, a 2-chloro-4-methylphenyl group, a 4-fluoro-2-methylphenyl group and the like are preferably used, and as the phenyl group substituted by halogen and lower (C₁₋₄) alkoxycarbonyl, for example, a 2-chloro-4-methoxycarbonylphenyl group and the like are preferably used, and the phenyl group substituted by halogen and cyano, for example, a 2-chloro-4-cyanophenyl group and the like are preferably used, and as the phenyl group substituted by halogen and 5-membered aromatic heterocycle, for example, a 2-fluoro-4-(1H-1,2,4-triazol-1-yl)phenyl group and the like are preferably used, and as the phenyl group substituted by halogen and lower (C₁₋₄) alkoxycarbonyl-lower (C₁₋₄) alkyl-carbamoyl, for example, a 2-chloro-4-(N-tert-butoxycarbonylmethylcarbamoyl)phenyl group, a 2-chloro-4-(N-ethoxycarbonylmethylcarbamoyl) phenyl group and the like are preferably used.

As Ar', a halogenophenyl group, a lower (C₁₋₉) alkyl-phenyl group, a halogen- and lower (C₁₋₉) alkoxy-carbonyl-substituted phenyl group and the like are preferably used.

More specifically, as Ar', a phenyl group, a phenyl group substituted with 1 to 3 (particularly 1 to 2) halogen atoms (e.g., a 2,3-difluorophenyl group, a 2,3-dichlorophenyl group, a 2,4-difluorophenyl group, a 2,4-dichlorophenyl group, a 2,5-difluorophenyl group, a 2,5-dichlorophenyl group, a 2,6-difluorophenyl group, a 2,6-dichlorophenyl group, a 3,4-difluorophenyl group, a 3,4-dichlorophenyl group, a 3,5-difluorophenyl group, a 3,5-dichlorophenyl group, a 4-bromo-2-fluorophenyl group, a 2-fluorophenyl group, a 2-chlorophenyl group, a 3-fluorophenyl group, a 3-chlorophenyl group, a 4-fluorophenyl group, a 4-chlorophenyl group, a 2-fluoro-4-chlorophenyl group, a 2-chloro-4-fluorophenyl group, a 2,3,4-trifluorophenyl group, a 2,4,5-trifluorophenyl group and the like), a phenyl group substituted by halogen and lower (C₁₋₄) alkyl (e.g., a 2-chloro-4-methylphenyl group, a 4-fluoro-2-methylphenyl group and the like), etc. are preferred. Among them, a phenyl group substituted with 1 to 3 (particularly 1 to 2) halogen atoms (e.g., a 2,3-dichlorophenyl group, a 2,4-difluorophenyl group, a 2,4-dichlorophenyl group, a 2,6-diclorophenyl group, a 2-fluorophenyl group, a 2-chlorophenyl group, a 3-chlorophenyl group, a 2-chloro-4-fluorophenyl group, a 2,4,5-trifluorophenyl group and the like), a phenyl group substituted by halogen and lower (C₁₋₄) alkyl (e.g., a 2-chloro-4-methylphenyl group, a 4-fluoro-2-methylphenyl group and the like), etc. are preferred. Particularly, As Ar', a group represented by the formula: 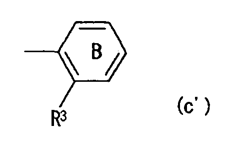 is preferred, and a group represented by the formula: 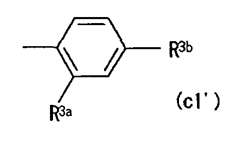 is more preferred.

As a halogen atom which is a substituent of a ring B and represented by R³ in the formula (c'), and a halogen atom represented by R^{3a} and R^{3b} in the formula (c1'), a fluorine atom or a chlorine atom is preferred. Example of the lower alkyl group represented by R³ in the formula (c') includes a C₁₋₄ alkyl group such as methyl, ethyl, propyl or the like. Among the groups represented by the formula (c'), 2,4-difluorophenyl group, 2-chloro-4-fluorophenyl group, a 2-methyl-4-chlorophenyl group and the like are preferred, and among the groups represented by the formula (c1') 2,4-difluorophenyl group, 2-chloro-4-fluorophenyl group and the like are preferred.

X represents a methylene group, a nitrogen atom, a sulfur atom, or an oxygen atom, and among them, a nitrogen atom, a sulfur atom or an oxygen atom is particularly preferred.

The ring A is substituted by the groups represented by the formula: -CO-R^{1'} (wherein, R^{1'} is as defined above) and the formula: -SO₂-Y-Ar' (wherein, Y and Ar' are as defined above), and particularly represents a 5 to 8 membered ring optionally further substituted by 1 to 4 substituent(s) selected from the group consisting of (1) an aliphatic hydrocarbon group optionally having substituents, (2) an aromatic hydrocarbon group optionally having substituents, (3) a group represented by the formula: -OR^{2'} (wherein R^{2'} represents a hydrogen atom or an aliphatic hydrocarbon group optionally having substituents) and (4) a halogen atom, and particularly a 5 to 8 membered ring optionally substituted by 1 to 4 substituent(s) selected from the group consisting of (1) an aliphatic hydrocarbon group optionally having substituents, (2) an aromatic hydrocarbon group optionally having substituents and (4) a halogen atom is preferred.

As the "aliphatic hydrocarbon group optionally having substituents" represented by R^{2'}, for example, same ones as the "aliphatic hydrocarbon group optionally having substituents" represented by R¹' described above can be used.

These substituents are substituted at substitutable portions in ring A. When X forming a ring is a nitrogen atom or a methylene group, the nitrogen atom or the methylene group also can be substituted. When ring A is substituted by plural number of substituents, the substituents may be the same or different. Also, the same carbon atom may be substituted by two substituents.

Examples of the "aliphatic hydrocarbon group optionally having substituents", and "aromatic hydrocarbon group optionally having substituents" as a substituent on the ring A, include the same ones as the "aliphatic hydrocarbon group optionally having substituents" and "aromatic hydrocarbon group optionally having substituents" represented by R¹' described above.

As the substituents of ring A, 1 or 2 of C₁₋₆ alkyl group (e.g. a C₁₋₄ alkyl group such as a methyl group, a tert-butyl group, etc.), a phenyl group, a halogen atom (e.g., a fluorine atom, a chlorine atom, a boron atom, an iodine atom, etc.), are preferably used.
m represents an integer of 0 to 2, n' represents an integer of 1 to 3, sum of m and n' is 4 or less, and m is preferred to be 1 and n' is also preferred to be 1.

As a compound represented by the formula (II), for example, following compounds and the like are preferred.
· A compound (II) wherein R¹' is a group represented by the formula OR^{1a"} (R^{1a"} represents a C₁₋₆ alkyl group), a group represented by the formula: 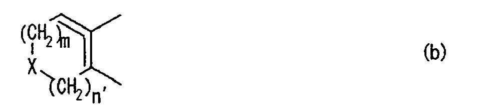 is a group represented by the formula: 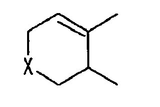 X is methylene or an oxygen atom, Y is methylene or -NH-, and Ar' is a phenyl group optionally having 1 or 2 substituents selected from the group consisting of a halogen atom and C₁₋₆ alkoxy.
· A compound (II) wherein R¹' is a group represented by the formula OR^{1a"} (R^{1a}", represents a C₁₋₆ alkyl group), a group represented by the formula: 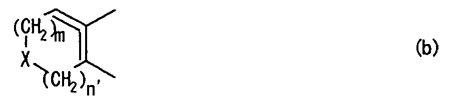 is a group represented by the formula: 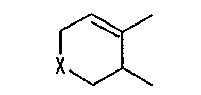 X and Y are methylene, or X is an oxygen atom and Y is -NH-, and Ar' is a phenyl group (e.g. a 2-chloro-4-fluorophenyl group, etc.) optionally having 2 halogen atoms.

(3) ethyl 6-(benzylsulfonyl)-1-cyclohexene-1-carboxylate (compound 86), ethyl 6-[(4-methoxybenzyl)sulfonyl]-1-cyclohexene-1-carboxylate (compound 87), ethyl 6-[(2,4-difluorobenzyl)sulfonyl]-1-cyclohexene-1-carboxylate (compound 88), ethyl 6-[(2-chloro-4-fluorobenzyl)sulfonyl]-1-cyclohexene-1-carboxylate (compound 89), ethyl (-)-6-[(2-chloro-4-fluorobenzyl)sulfonyl]-1-cyclohexene-1-carboxylate (compound 90), ethyl (+)-6-[(2-chloro-4-fluorobenzyl)sulfonyl]-cyclohexene-1-carboxylate (compound 91), ethyl 3-[(2,4-difluorophenyl)sulfamoyl]-3,6-dihydro-2H-pyran-4-carboxylate (compound 92) or ethyl 3-[(2-chloro-4-fluorophenyl)sulfamoyl]-3,6-dihydro-2H-pyran-4-carboxylate (compound 93).
(4) ethyl 6-[(2-chloro-4-fluorobenzyl)sulfonyl]-1-cyclohexene-1-carboxylate (compound 89), ethyl (+)-6-[(2-chloro-4-fluorobenzyl)sulfonyl]-1-cyclohexene-1-carboxylate (compound 91) or ethyl 3-[(2-chloro-4-fluorophenyl)sulfamoyl]-3,6-dihydro-2H-pyran-4-carboxylate (compound 93).

A salt of the compound represented by the formula (II), includes a salt with an inorganic base, organic base, inorganic acid, organic acid, basic or acidic amino acid. A salt with an inorganic base may, for example, be an alkaline metal salt such as sodium and potassium salts, an alkaline earth metal salt such as calcium and magnesium salts, aluminum and ammonium salts, and a salt with an organic base may, for example, be a salt with trimethylamine, triethylamine, pyridine, picoline, ethanolamine, diethanolamine, triethanolamine, dicyclohexylamine or N,N'-dibenzylethylenediamine. A salt with an inorganic acid may, for example, be a salt with hydrochloric acid, hydrobromic acid, nitric acid, sulfuric acid or phosphoric acid, and a salt with an organic acid may, for example, be a salt with formic acid, acetic acid, trifluoroacetic acid, fumaric acid, oxalic acid, tartaric acid, maleic acid, citric acid, succinic acid, malic acid, methanesulfonic acid, benzenesulfonic acid or p-toluenesulfonic acid. A salt with a basic amino acid may, for example, be a salt with arginine, lysine or ornithine, and a salt with acidic amino acid may, for example, be a salt with aspartic acid or glutamic acid.

When a compound and a salt thereof represented by formula (II) have stereoisomers, any of such stereoisomers and mixtures thereof are included in the invention.

In addition, when a compound and a salt thereof represented by formula (II) have optical isomers, any of such optical isomers and mixtures thereof are included in the invention.

The aforementioned compound can be produced according to a method known per se, for example, a production method described in WO01/10826.

A prodrug for an inventive Compound or a salt thereof is a compound which is converted into an inventive compound under a physiological condition within the living body as a result of a reaction with an enzyme or gastric acid, thus a compound undergoing an enzymatic oxidation, reduction or hydrolyzation to form an inventive Compound and a compound hydrolyzed by gastric acid to form an inventive Compound. A prodrug for an inventive Compound may, for example, be a compound obtained by subjecting an amino group in an inventive Compound to an acylation, alkylation or phosphorylation (e.g., a compound obtained by subjecting an amino group in an inventive Compound to an eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation and tert-butylation); a compound obtained by subjecting a hydroxy group in an inventive Compound to an acylation, alkylation, phosphorylation and boration (e.g., a compound obtained by subjecting a hydroxy group in an inventive Compound to an acetylation, palmitoylation, propanoylation, pivaloylation, succinylation, fumarylation, alanylation and dimethylaminomethylcarbonylation); a compound obtained by subjecting a carboxyl group in an inventive Compound to an esterification or amidation (e.g., a compound obtained by subjecting a carboxyl group in an inventive Compound to an ethylesterification, phenylesterification, carboxymethylesterification, dimethylaminomethylesterification, pivaloyloxymethylesterification, ethoxycarbonyloxyethylesterification, phthalidylesterification, (5-methyl-2-oxo-1,3-dioxolen-4-yl)methylesterification, cyclohexyloxycarbonylethylesterification and methylamidation) and the like. These compounds can be produced from the inventive compounds according to a method known per se.

Also, a prodrug for an inventive compound may also be one which is converted into an inventive compound under a physiological condition, such as those described in "IYAKUHIN no KAIHATSU (Development of Pharmaceuticals)", Vol.7, Design of Molecules, p.163-198, Published by HIROKAWA SHOTEN (1990).

The inventive compound, a salt thereof and a prodrug thereof can be produced according to a method known per se, for example, a production method described in WO99/46242 or a method analogous thereto.

The inventive compound, a salt thereof and a prodrug thereof may be a hydrate or non-hydrate.

The inventive compound, a salt thereof and a prodrug thereof may be labeled with an isotope (e.g., ³H, ¹⁴C, ³⁵S, ¹²⁵I etc.) and the like.

According to the composition of the present invention, a compound stable in an acidic range such as the inventive Compound, or a salt thereof and a prodrug thereof having poor water solubility can be used effectively as a component of the composition constituted by an emulsifier.

A compound stable in an acidic range, specifically the inventive compound or a salt thereof and a prodrug thereof may exist in a state of a liquid or solid in an oil phase, and the composition of the present invention is an oil-in-water (O/W type) or S/O/W type emulsion composition.

The composition of the present invention, for example, can be produced using an emulsifier.

The composition of the present invention specifically consists of disperse phase particles containing oil component, emulsifier, and a compound stable in an acidic range (specifically the inventive compound or a salt thereof and a prodrug thereof), and water in which the disperse phase particles are dispersed and emulsifier is contained. The disperse phase particle is a disperse phase that one of two liquids not mixing to each other, exists on the other liquid side as a fine particle.

As the oil component, any pharmaceutically acceptable fats and oils generally used for the preparation of fat emulsion in the field of pharmaceutical technology can be used. Examples of the fats and oils include vegetable oil, fats and oils obtainable by partial hydrogenation of vegetable oils, oils obtainable by transesterification (simple glycerides and mixed glycerides), and glycerol esters of medium chain fatty acids.

The aforementioned fats and oils include, for example, a glycerol ester of a fatty acid having about 6 to 30 carbon atoms, preferably about 6 to 22 carbon atoms. Examples of the aforementioned fatty acid include saturated fatty acids such as caproic acid, caprylic acid, capric acid, lauric acid, myristic acid, palmitic acid, stearic acid, behenic acid and the like; unsaturated fatty acids such as palmitooleic acid, oleic acid, linoleic acid, arachidonic acid, eicosapentaenoic acid, docosahexaenoic acid and the like.

Preferred examples of the oil component include vegetable oils such as soybean oil, cottonseed oil, rapeseed oil, peanut oil, safflower oil, sesame oil, rice bran oil, corn germ oil, sunflower oil, poppy oil, olive oil and the like. Of these vegetable oils, soybean oil and the like are preferably used.

As the fats and oils, triglyceride of a medium chain fatty acid having about 6 to 14 carbon atoms, preferably about 8 to 12 carbon atoms, can be also used. Preferable glycerine ester of a medium chain fatty acid is, for example, caprylic/capric triglycerides such as "Migriol 810" and "Migriol 812" (both trade names, manufactured by Huls Co., Ltd., available from Mitsuba Trading Co., Ltd.), a glyceryl tricaprylate (tricaprylin) such as "Panasate 800" (trade name, manufactured by NOF Corporation, Japan), and the like.

The composition of the present invention contains an oil component in a proportion of, for example, about 1 to about 30 wt% by weight, preferably about 2 to about 25 wt%, and more preferably about 2.5 to about 22.5 wt%, of the entire composition.

As the emulsifier, any medically permitted emulsifier can be used. Specifically, medically permitted phospholipids and non-ionic surfactant are preferred. The emulsifier can be used alone or as a mixture of two or more kinds thereof.

The phospholipid contains, for example, naturally-occurring phospholipids (e.g., egg yolk lecithin, soybean lecithin, and the like), a hydrogenation product of these, or a synthetically-obtained phospholipid (e.g., phosphatidylcholine, phosphatidylethanolamines, phosphatidic acid, phosphatidylserine, phosphatidylinositol, or phosphatidylglycerol, and the like). Among them, egg yolk lecithin, soybean lecithin and phosphatidylcholine originated from egg yolk and soyabean are preferred. The most preferable phospholipid is lecithin. In addition, anionic phospholipid is preferred among the synthetic phospholipid, and specific examples of the anionic synthetic phospholipid include dimyristoylphosphatidylglycerol, dipalmitoylphosphatidylglycerol, distearoylphosphatidylglycerol, dioleoylphosphatidylglycerol, oleoylpalmitoylphosphatidylglycerol, dioctanoylphosphatidic acid, didecanoylphosphatidic acid, dilauroylphosphatidic acid, dimyristoylphosphatidic acid, dipalmitoylphosphatidic acid, diheptadecanoylphosphatidic acid, distearoylphosphatidic acid, dioleoylphosphatidic acid, arachidonylstearoylphosphatidic acid, dipalmitoylphosphatidylserine, dioleoylphosphatidylserine, dimyristoylphosphatidylinositol, dipalmitoylphosphatidylinositol, distearoylphosphatidylinositol, dioleoylphosphatidylinositol, dimyristoylphosphatidylserine, distearoylphosphatidylserine and the like, and preferable example includes dimyristoylphosphatidylglycerol.

These anionic synthetic phospholipids can be chemically synthesized according to a method known per se or may be obtained by purification.

The non-ionic surfactant is exemplified by polymer surfactants having a molecular weight of about 800 - 20000, such as polyoxyethylene-polyoxypropylene copolymer, polyoxyethylene alkyl ether, polyoxyethylene alkyl aryl ether, hydrogenated castor oil polyoxyethylene derivative, polyoxyethylene sorbitan derivative, polyoxyethylene sorbitol derivative, polyoxyethylene alkyl ether sulfate and the like.

The phospholipid and the non-ionic surfactant as an emulsifier can be used singly or as a mixture of two or more kinds. It is also possible to use a commercially available phospholipid.

The total amount of the emulsifier in a composition of the present invention relative to the entire composition of the present invention is generally about 0.1 to about 10% (W/V), preferably about 0.2 to about 7% (W/V), and more preferably about 0.5 to about 5% (W/V). Wherein the anionic synthetic phospholipid relative to the entire composition is in a proportion of about 0.0001 to about 5%(W/V).

In the composition of the present invention, the proportion of the emulsifier relative to the oil component is, for example, about 0.1 to about 150 wt%, preferably about 0.5 to about 125 wt%, more preferably about 1 to about 100 wt%. The emulsifier is often used in a proportion of generally about 1 to about 15 wt%, particularly about 1 to about 10 wt%, relative to the an oil component.

Water to be used in the present invention is not particularly limited as long as it is permitted as a medical material, and an example includes purified water, water for an injection purpose (distilled water for injection). When preparing a product other than medications, it is not particularly limited.

Water amount to be used in the present invention relative to the entire composition is generally about 40 to 99% (W/V), and preferably about 55 to 98.8% (W/V).

The composition of the present invention can be prepared by mixing a disperse phase component consisting of the inventive compound, a salt thereof and a prodrug thereof (main drug), the oil component and emulsifier to water so as to be emulsified, and a buffering agent may be added to an aqueous phase prior to an emulsification or may be added to an emulsified composition after an emulsification. Where necessary, a stabilizer for improving the stability of the aforementioned main drug, an isotonic agent for adjusting the osmotic pressure, an emulsifying-auxiliaries to enhance emulsifying capability, an emulsification stabilizer for enhancing the stability of the emulsifier and the like may be added.

Examples of the stabilizer include, for example, antioxidants (for example, ascorbic acid, tocopherol, sorbic acid, retinol and the like), chelating agents (for example, edetic acid, citric acid, tartaric acid and the like, and the salt thereof) and the like. The stabilizer is used in an amount of generally about 0.00001 - about 10% (W/V), preferably about 0.0001 - about 5% (W/V), relative to the entire composition of the present invention.

Examples of the isotonic agent include glycerin, sugar alcohols, monosaccharides, disaccharides, amino acid, dextran, albumin and the like. These isotonic agents may be used singly or in combination of two or more kinds.

Examples of the emulsifying-auxiliaries include fatty acids having about 6 to 30 carbon atoms, salts of these fatty acids, monoglycerides of the fatty acids. Examples of the aforementioned fatty acid include caproic acid, caprylic acid, capric acid, lauric acid, myristic acid, palmitic acid, stearic acid, behenic acid, palmitooleic acid, oleic acid, linolic acid, arachidonic acid, eicosapentaenoic acid, docosahexaenoic acid and the like. Examples of the fatty acids salts include alkali metal salts such as sodium salt and potassium salt, calcium salt and the like.

Examples of the emulsifying stabilizer include cholesterol, cholesterol esters, tocopherol, albumin, fatty acid amide derivatives, polysaccharides, derivatives of fatty acid esters of polysaccharides and the like.

The concentration of the compound stable in an acidic range (hereinafter, simply referred to as an inventive Compound, a salt thereof or a prodrug thereof) in the composition of the present invention varies depending on the pharmacological activities or kinetics in blood of the compound, and is usually about 0.001 to about 5% (W/V), preferably about 0.01 to about 2% (W/V), more preferably about 0.1 to about 1.5% (W/V). It is also possible to set the content of the inventive Compound, a salt thereof or a prodrug thereof in the composition of the present invention to about 1 to about 5000 mg, preferably about 10 to about 2000 mg, more preferably about 100 to about 1500 mg, per 100 ml of the composition. Furthermore, the content of the inventive Compound, a salt thereof or a prodrug thereof in the composition of the present invention can be adjusted to about 0.001 to about 95 wt%, preferably about 0.01 to about 30 wt%, more preferably about 0.1 to about 3 wt%, based on the total volume of the composition.

The proportion (wt%) of the inventive Compound, a salt thereof or a prodrug thereof relative to the disperse phase comprising an oil component and an emulsifier is generally about 0.0047 to about 24%, and preferably about 0.047 to about 9.4%.

The composition of the present invention is adjusted to have a pH of about 3.7 to about 5.5, more preferably about 3.7 to about 5.0, and more preferably about 4.0 to about 5.0.

The pH adjusting agent is exemplified by phosphoric acid, carbonic acid, citric acid, hydrochloric acid, sodium hydroxide and the like, with particular preference given to hydrochloric acid, sodium hydroxide and the like.

As the aforementioned buffering agent, any medically permitted buffering agents can be used. Buffering agent consisting of following components is preferred, consisted component examples include acetic acid, glacial acetic acid, lactic acid, citric acid, phosphoric acid, carbonic acid, histidine, glycine, barbital, phthalic acid, adipic acid, ascorbic acid, maleic acid, succinic acid, tartaric acid, glutamic acid, benzoic acid, aspartic acid, and salts thereof (e.g., potassium, sodium, etc.), and specific examples include sodium acetate, sodium lactate, sodium citrate, disodium hydrogenphosphate, monosodium dihydrogen phosphate, sodium carbonate, sodium hydrogencarbonate, hydrochloric acid, sodium hydroxide or the like. Also respective buffering agents may be used in combination. Particularly, one, two or more buffering agents selected from acetate buffer, glacial acetate buffer, lactate buffer, citrate buffer, and phosphate buffer, are preferably be used.

As the buffering agent combinations of (i) acetic acid or glacial acetic acid, and sodium acetate (acetate buffer or glacial acetate buffer), (ii) lactic acid and sodium lactate (lactate buffer) and the like are favorably used.

Concentration of the buffering agent is generally 100 mM or less, specifically about 0.1 to about 100 mM, preferably about 0.2 to about 50 mM, and more preferably about 5 mM to about 40 mM.

pH adjusting agent is an acid or an alkali compound to be added for adjusting a pH to an aimed pH of the solution.

In general, blending quantity of pH adjusting agent to be blended in an injection is very small, and in a lot of fat emulsifier commercially available in Japan, blended quantity of sodium hydroxide compounded as a pH adjusting agent in fat emulsifier is 5 mM or less. At the time of adjusting a solution, it is possible to adjust to an aimed pH, but it is difficult to keep the pH as pH easily varies by an addition of acid or alkali.

The buffering agent is acting to loose pH variation due to an addition of acid or alkali, meaning that it is a compound having a buffering action. Mostly it is a mixture solution of weak acid and a salt thereof, or weak base and a salt thereof.

The emulsified composition of the present invention is not affected by a generation of free fatty acids according to a blending of buffering agent, and it is possible to constantly keep the pH of the emulsified composition at the time of high-pressure autoclave-sterilization by steam and long term storage.

Blending amount of the buffering agent to be used in a general injection is large as it is aiming for a buffering action, and for example, the amount of the acetate buffer in an injection solution commercially available in Japan is about 0.2 mM to about 100 mM.

The composition of the present invention is preferred to be used for example as a composition for an injection.

The composition of the present invention can be produced principally according to a known method or a method analogous thereto. The emulsification can be conducted in a conventional emulsifying technique. It is preferable to disperse or dissolve the inventive Compound, a salt thereof or a prodrug thereof in an oil component beforehand. That is, a mixture of (1) a disperse phase containing the oil component and the emulsifier, and (2) the inventive Compound, a salt thereof or a prodrug thereof is dispersed in water to give a composition consisting of an 0/W type or S/O/W type emulsion. The buffering agent can be added to an aqueous phase prior to an emulsification or can be added to an emulsified composition after an emulsification. The mixture to be emulsified can be produced by mixing an aqueous phase with an oil phase or mixing an oil phase with an aqueous phase. Moreover, additives such as stabilizer, isotonic agent etc. can be added to any of an aqueous phase and an oil phase.

Preferred examples of the method include a method comprising homogenizing a heterogeneous mixture containing a liquid mixture of the main drug, an oil component, an emulsifier, an additive such as an isotonic agent where necessary and the like, and water containing a buffering agent in an emulsifying apparatus to give a roughly emulsified emulsion, followed by, if necessary, adding water, further homogenizing the resultant rough emulsion using an emulsifying apparatus and removing large particles by a filtering means such as a filter and the like to give an oil-in-water composition. The aforementioned mixture is generally heated to, for example, about 30 to about 90°C, preferably about 40 to about 80°C to dissolve or disperse the main drug. Examples of the emulsifying apparatus for the emulsification of the heterogeneous mixture containing the aforementioned mixture and water include a conventional apparatus such as a homogenizer including a pressure jetting homogenizer, an ultrasonic homogenizer and the like, and a homomixer such as a high-rate mixer and the like. For removing large particles in the emulsifier, the homogenized emulsion can be subjected to a filtering means such as a filter. The pore diameter of the filter to be used is, for example, about 0.8 - about 20 µm, preferably about 1 - about 10 µm.

In the composition of the present invention, the mean particle size distribution of the disperse phase wherein the inventive Compound, a salt thereof or a prodrug thereof is dissolved, is, for example, mostly about 0.01 to about 7 µm, and preferably about 0.02 to about 5 µm. In view of the stability of the emulsion and biodistribution after administration, the average particle size of the disperse phase particle wherein the inventive Compound, a salt thereof or a prodrug thereof is dissolved, is, for example, about 0.025 to about 0.7 µm, and preferably about 0.05 to about 0.4 µm.

The average particle size used in the specification indicates an average particle size based on the volume distribution, and is the disperse phase particle size measured by a Laser Diffraction Particle Size Analyzer based on a laser diffraction confusion method as a measurement principle.

A pyrogen can be removed from the composition of the present invention according to a method known per se.

The composition of the present invention is sterilized and sealed after nitrogen gas displacement as necessary.

The emulsion composition of the present invention is adjusted to about pH 3.7 - about pH 5.5 by adding a buffering agent. Therefore, the pH of the emulsion composition and the average particle size of the disperse phase particles therein scarcely vary even after sterilization in an autoclave etc. or after a long-term preservation, and the composition is stable. Consequently, the emulsion composition of the present invention and a compound, a salt thereof or a prodrug thereof, which is the active ingredient of the emulsion composition, exhibit superior stability. Moreover, sterilization of the emulsion composition of the present invention in an autoclave etc. and a long-term preservation thereof do not result in the production of visually observable free oil drops. In other words, the disperse phase particle and water, in which the disperse phase particle has been dispersed, do not show phase separation and are stable.

In addition, since the emulsion composition of the present invention can be adjusted to pH about 3.7 - about 5.5 by adding a buffering agent to give a stable emulsion composition, an optimal pH can be determined depending on various other conditions such as the characteristics of the emulsifier and stability of the compound and the like. Therefore, even when other conditions that can be overcome by adjusting pH to about 3.7 - about 5.5 are present, such as the case where the stability of the emulsion composition is influenced, and the like, an emulsion composition satisfying various conditions can be afforded by adopting the adjustment of pH.

The emulsion composition of the present invention moreover shows long-term preservation stability for 24 months. This exceeds a general period of use of 18 months of emulsion preparations.

In addition, in the emulsion composition of the present invention, the concentration of the compound stable in an acidic range (particularly the inventive Compound, a salt thereof or a prodrug thereof) can be increased. By combing, as an optimal preparation design, optimal selection of oil component and emulsifier, addition of nonionic surfactant (polyoxyethylene compound (such as polyethylene glycol, polysorbate and the like) and the like) and the like, surface modification of the disperse phase particle, control of the particle size of the disperse phase particle and the like, retentivity in blood, blood vessel permeability and migration performance into inflammatory site can be enhanced. Therefore, the pharmacokinetics and biodistribution of the inventive Compound, a salt thereof or a prodrug thereof can be improved, thereby enabling targeting, which in turn leads to more effective administration of a drug with a less side effect. Thus, the emulsion composition of the present invention is useful for the treatment of target disease particularly by an intravenous administration.

When the compound stable in an acidic range is an inventive Compound, or a salt thereof or a prodrug thereof, since an inventive Compound, a salt thereof and a prodrug thereof have low toxicity, an nitric oxide (NO) production-inhibiting effect and an inhibitory effect on the production of an inflammatory cytokine such as TNF-α, IL-1 and IL-6, the composition of the present invention is useful for prophylaxis/treatment of sepsis including a severe sepsis as well as in a mammal (e.g., cat, cattle, dog, horse, goat, monkey, human and the like) against heart disease, autoimmune disease, inflammatory disease, central nervous system disease, infectious disease, septic shock, compromised immune function and the like, examples including ichorrhemia, endotoxin shock, exotoxin shock, systemic inflammatory response syndrome (SIRS), compensatory anti-inflammatory response syndrome (CARS), burn injury, trauma, postoperative complication, cardiac deficiency, shock, hypotension, rheumatoid arthritis, osteoarthritis, gastritis, ulcerative colitis, peptic ulcer, stress-induced gastric ulcer, Crohn's disease, autoimmune disease, post-transplant tissue failure and rejection, postischemic reperfusion failure, acute coronary microvascular embolism, shock-induced vascular embolism (disseminated intravascular coagulation (DIC) and the like), ischemic cerebral disorder, arterial sclerosis, malignant anemia, Fanconi's anemia, drepanocythemia, pancreatitis, nephrose syndrome, nephritis, renal failure, insulin-dependent diabetes, insulin-independent diabetes, hepatic porphyria, alcoholism, Parkinson's disease, chronic leukemia, acute leukemia, tumor, myeloma, infantile and adult respiratory distress syndrome, pulmonary emphysema, dementia, Alzheimer's disease, multiple sclerosis, vitamin E deficiency, aging, sunburn, muscular dystrophy, myocarditis, cardiomyopathy, myocardial infarction, sequela of myocardial infarction, osteoporosis, pneumonia, hepatitis, psoriasis, pain, cataract, influenza infection, malaria, human immunodeficiency virus (HIV) infection, radiation-induced failure, burn, in vitro fertilization efficiency, hypercalcemia, tonic spondylitis, osteopenia, bone Behcet's disease, osteomalacia, fracture, acute bacterial meningitis, Helicobactor pylori infection, invasive staphylococcal infection, tuberculosis, systemic mycosis, herpes simplex virus infection, varicella-herpes zoster virus infection, human papilloma virus infection, acute viral encephalitis, encephalitis, cerebral meningitis, infection-induced compromised immune function, asthma, atopic dermatitis, allergic rhinitis, reflux esophagitis, fever, hyper cholesteremia, hyperglycemia, hyperlipidemia, diabetic complication, diabetic renal disease, diabetic neuropathy, diabetic retinopathy, gout, gastric atony, hemorrhoid, systemic lupus erythematosus, spinal damage, insomnia, schizophrenia, epilepsy, cirrhosis, hepatic failure, instable angina, valvular disease, dialysis-induced thrombocytopenia, acute ischemic cerebral apoplexy, acute cerebral thrombosis, cancer metastasis, urinary bladder cancer, mammary cancer, uterine cervical cancer, colon cancer, gastric cancer, ovarian cancer, prostatic cancer, parvicellular pulmonary cancer, non-parvicellular pulmonary cancer, malignant melanoma, Hodgkin's disease, non-Hodgkin lymphoma, side effects of anticancer agents or immunity control agents administration and the like.

In addition, the composition of the present invention is useful as an inhibitory agent of TLR signal. 'TLR signal' means an optional Toll-like receptor transmitting a signal to induce a biological defense response by recognizing a fungous component or the like of microorganism, and examples include generally known signal transmission through TLR 1 to TLR 10.

The inventive Compound, a salt thereof and a prodrug thereof have low toxicity and a TLR signal inhibitory effect, and since it controls an inflammatory mediator such as NO and/or cytokine production, the composition of the present invention is useful for prophylaxis/treatment of diseases causing the signal variation, for example, organopathy and the like. In here, the organ refers to each systems of central nervous system, circulatory system, respiratory system, osteo-artho- system, alimentary system, or kidney · urinary system.

The composition of the present invention is useful for prophylaxis/treatment of following diseases caused by a TLR signal variation as an inhibitory agent of TLR signal.
(1) central nervous system disease [(i) neurodegenerative disease (e.g., geriatric dementia, Alzheimer's disease, Down's syndrome, Parkinson's disease, Creutzfeldt-Jakob disease, amyotrophic spinal lateral sclerosis, diabetic(al) neuropathy, etc.,), (ii) nervous disorder at the time of cerebral vascular disorder (e.g., cerebrovascular insufficiency such as cerebral infraction, cerebral hemorrhage, brain arteriosclerosis and the like), head trauma·spinal cord injury, sequelae of encephalitis, or cerebral paralysis, (iii) memory disorder (e.g., geriatric dementia, amnesia), or the like], particularly, Alzheimer's disease,
(2) circulatory system disease [(i) ischemic syndromes of acute myocardial infarction, unstable angina, or the like, (ii) peripheral arterial obliterative disease, (iii) post-coronary intervention (percutaneous transluminal coronary angioplasty (PTCA), atherectomy (DCA), stent placement or the like) restenosis, (iv) post-coronary-artery bypass surgery restenosis (v) post- intervention of other peripheral artery (angioplasty, atherectomy, stenting or the like) and bypass surgery restenosis, (vi) ischemic heart diseases such as cardiac infraction, angina, and the like, (vii) claudicatio intermittens (viii) apoplexy (e.g., cerebral infraction, cerebral embolus, cerebral hemorrhage or the like), (ix) lacuner infarct, (x) cerebrovascular dementia, (xi) arteriosclerosis (e.g., atherosclerosis or the like) and diseases caused by this (e.g., ischemic heart disease such as cardiac infarction or the like, and cerebrovascular diseases such as brain infarction·cerebral apoplexy or the like), (xii) cardiac deficiency, (xiii) arrhythmia, (xiv) arteriosclerosis nidal development, (xv) thrombus formation, (xvi) hypotension, (xvii) shock, (xviii) vessel embolus caused by shock (e.g., disseminated intravascular coagulation (DIC) or the like)], particularly arteriosclerosis,
(3) respiratory disease [respiratory distress syndrome, respiratory failure, pulmonary emphysema, pneumonia, bronchial infection, bronchiolitis or the like],
(4) osteo- · artho- system disease [arthritis rheumatoides, osteoporosis, osteomalacia, osteopenia, osteo-Behcet's disease or the like], particularly arthritis rheumatoides,
(5) alimentary · liver, gall bladder, and pancreas system disease [ulcerative colitis, gastritis, peptic ulcer, cirrhosis, hepatic failure, hepatitis, cholecystitis, pancreatitis, or the like] particularly, ulcerative colitis,
(6) kidney · urinary system disease [nephritis, renal failure, cystitis or the like], or combination of these diseases (multi organ failure, etc.). Also the composition of the present invention is useful for preventing and/or treating infections, and particularly a sepsis (severe sepsis) involving organopathy caused by a TLR signal variation as an inhibitory agent of TLR signal.

While the dose of the composition of the present invention may vary depending on the kind of the compound stable in an acidic range (particularly, an inventive Compound, a salt thereof and a prodrug thereof), age, body weight and condition, the dosage form, the mode and the period of the administration, it may, for example, be generally about 0.01 to about 1000 mg/kg, preferably about 0.01 to about 100 mg/kg, more preferably about 0.1 to about 100 mg/kg, most preferably about 0.1 to about 50 mg/kg, and particularly about 1.0 to about 30 mg/kg, as the inventive Compound (Iaa) or (Ie), per day in a patient having a sepsis (adult weighing about 60 kg), said daily dose being given intravenously all at once or in several portions during a day. Alternatively, it may be continuously administered by an intravenous drip. It is a matter of course that a lower daily dose may be sufficient or an excessive dose may be required since the dose may vary depending on various factors as discussed above.

The composition of the present invention, for example, can be used concurrently with a drug other than the inventive Compound, or a salt thereof or a prodrug thereof.

The drugs that can be used concurrently with the inventive Compound, a salt thereof or a prodrug thereof (hereinafter sometimes to be briefly referred to as a combination drug) are, for example, antibacterial agent, antifungal agent, non-steroidal antiinflammatory drug, steroid, anticoagulant, platelet aggregation inhibitor, thrombolytic drug, immunomodulator, antiprotozoal, antibiotic, antitussive and expectorant drug, sedative, anesthetic, antiulcer drug, antiarrhythmic, hypotensive diuretic, tranquilizer, antipsychotic, antitumor drug, hypolipidemic drug, muscle relaxant, anticonvulsant, antidepressant, antiallergic drug, cardiac, antiarrhythmic, vasodilator, vasoconstrictor, hypotensive diuretic, antidiabetic drug, antinarcotic, vitamin, vitamin derivative, therapeutic agent for arthritis, antirheumatic, antiasthmatic, therapeutic agent for pollakiuria/anischuria, therapeutic agent for atopic dermatitis, therapeutic agent for allergic rhinitis, hypertensive drug, endotoxin-antagonist or -antibody, signal transduction inhibitor, inhibitor of inflammatory mediator activity, antibody to inhibit inflammatory mediator activity, inhibitor of anti-inflammatory mediator activity, antibody to inhibit anti-inflammatory mediator activity and the like. Of these, antibacterial agent, antifungal agent, non-steroidal antiinflammatory drug, steroid, anticoagulant and the like are preferable. Specific examples thereof include the following.
(1) antibacterial agent
   <1> sulfa drug sulfamethizole, sulfisoxazole, sulfamonomethoxine, sulfamethizole, salazosulfapyridine, silver sulfadiazine and the like.
   <2> quinoline antibacterial agent nalidixic acid, pipemidic acid trihydrate, enoxacin, norfloxacin, ofloxacin, tosufloxacin tosilate, ciprofloxacin hydrochloride, lomefloxacin hydrochloride, sparfloxacin, fleroxacin and the like.
   <3> antiphthisic isoniazid, ethambutol (ethambutol hydrochloride), p-aminosalicylic acid (calcium p-aminosalicylate), pyrazinamide, ethionamide, protionamide, rifampicin, streptomycin sulfate, kanamycin sulfate, cycloserine and the like.
   <4> antiacidfast bacterium drug diaphenylsulfone, rifampicin and the like.
   <5> antiviral drug idoxuridine, aciclovir, vidarabine, ganciclovir and the like.
   <6> anti-HIV agent zidovudine, didanosine, zalcitabine, indinavir sulfate ethanolate, ritonavir and the like.
   <7> antispirochetele
   <8> antibiotic tetracycline hydrochloride, ampicillin, piperacillin, gentamicin, dibekacin, kanendomycin, lividomycin, tobramycin, amikacin, fradiomycin, sisomicin, tetracycline, oxytetracycline, rolitetracycline, doxycycline, ampicillin, piperacillin, ticarcillin, cephalothin, cephapirin, cephaloridine, cefaclor, cephalexin, cefroxadine, cefadroxil, cefamandole, cefotoam, cefuroxime, cefotiam, cefotiam hexetil, cefuroxime axetil, cefdinir, cefditoren piboxil, ceftazidime, cefpiramide, cefsulodin, cefmenoxime, cefpodoxime proxetil, cefpirome, cefozopran, cefepime, cefsulodin, cefmenoxime, cefmetazole, cefminox, cefoxitin, cefbuperazone, latamoxef, flomoxef, cefazolin, cefotaxime, cefoperazone, ceftizoxime, moxalactam, thienamycin, sulfazecin, aztreonam or a salt thereof, griseofulvin, lankacidin-group antibiotics (J. Antibiotics,38,877-885(1985)) and the like.
(2) antifungal agent
   <1> polyethylene antibiotic (e.g., amphotericin B, nystatin, trichomycin)
   <2> griseofulvin, pyrrolnitrin and the like
   <3> cytosine metabolism antagonist (e.g., flucytosine)
   <4> imidazole derivative (e.g., econazole, clotrimazole, miconazole nitrate, bifonazole, croconazole)
   <5> triazole derivative (e.g. fluconazole, itraconazole, azole compound [2-[(1R,2R)-2-(2,4-difluorophenyl)-2-hydroxy-1-methyl-3-(1H-1,2,4-triazol-1-yl)propyl]-4-[4-(2,2,3,3-tetrafluoropropoxy)phenyl]-3(2H,4H)-1,2,4-triazolone]
   <6> thiocarbamic acid derivative (e.g. trinaphthol)
   <7> echinocandin derivative (e.g., caspofungin, micafungin, anidulafungin) and the like.
(3) non-steroidal antiinflammatory drug acetaminophen, phenacetin, ethenzamide, sulpyrine, antipyrin, migrenin, aspirin, mefenamic acid, flufenamic acid, diclofenac sodium, loxoprofen sodium, phenylbutazone, indomethacin, ibuprofen, ketoprofen, naproxen, oxaprozin, flurbiprofen, fenbufen, pranoprofen, floctafenine, epirizole, tiaramide hydrochloride, zaltoprofen, gabexate mesilate, camostat mesilate, urinastatin, colchicine, probenecid, sulfinpyrazone, benzbromarone, allopurinol, gold sodium thiomalate, sodium hyaluronate, sodium salicylate, morphine hydrochloride, salicylic acid, atropine, scopolamine, morphine, pethidine, levorphanol, ketoprofen, naproxen, oxymorphone or a salt thereof, and the like.
(4) steroid dexamethasone, hexestrol, methimazole, betamethasone, triamcinolone, triamcinolone acetonide, fluocinonide, fluocinolone acetonide, prednisolone, methylprednisolone, cortisone acetate, hydrocortisone, fluorometholone, beclometasone dipropionate, estriol and the like.
(5) anticoagulant heparin sodium, sodium citrate, activated protein C, tissue factor pathway inhibitor, antithrombin III, dalteparin sodium, warfarin potassium, argatroban, gabexate, sodium citrate and the like.
(6) platelet aggregation inhibitor ozagrel sodium, ethyl icosapentate, beraprost sodium, alprostadil, ticlopidine hydrochloride, pentoxifylline, dipyridamole and the like.
(7) thrombolytic drug tisokinase, urokinase, streptokinase and the like.
(8) immunomodulator cyclosporin, tacrolimus, gusperimus, azathioprine, antilymphocyte serum, dried sulfonated immunoglobulin, erythropoietin, colony-stimulating factor, interleukin, interferon and the like.
(9) antiprotozoal metronidazole, tinidazole, diethylcarbamazine citrate, quinine hydrochloride, quinine sulfate and the like.
(10) antitussive and expectorant drug ephedrine hydrochloride, noscapine hydrochloride, codeine phosphate, dihydrocodeine phosphate, isoproterenol hydrochloride, ephedrine hydrochloride, methylephedrine hydrochloride, noscapine hydrochloride, alloclamide, chlophedianol, picoperidamine, chloperastine, protokylol, isoproterenol, salbutamol, terbutaline, oxymetabanol, morphine hydrochloride, dextromethorphan hydrobromide, oxycodone hydrochloride, dimemorphan phosphate, tipepidine hibenzate, pentoxyverine citrate, clofedanol hydrochloride, benzonatate, guaifenesin, bromhexine hydrochloride, ambroxol hydrochloride, acetylcysteine, ethyl cysteine hydrochloride, carbocysteine and the like.
(11) sedative chlorpromazine hydrochloride, atropine sulfate, phenobarbital, barbital, amobarbital, pentobarbital, thiopental sodium, thiamylal sodium, nitrazepam, estazolam, flurazepam, haloxazolam, triazolam, flunitrazepam, bromovalerylurea, chloral hydrate, triclofos sodium and the like.
(12) anesthetic
   (12-1) local anesthetic cocaine hydrochloride, procaine hydrochloride, lidocaine, dibucaine hydrochloride, tetracaine hydrochloride, mepivacaine hydrochloride, bupivacaine hydrochloride, oxybuprocaine hydrochloride, ethyl aminobenzoate, oxethazaine) and the like.
   (12-2) general anesthetic
      <1> inhalation anesthetic (e.g., ether, halothane, nitrous oxide, isoflurane, enflurane),
      <2> intravenous anesthetic (e.g., ketamine hydrochloride, droperidol, thiopental sodium, thiamylal sodium, pentobarbital) and the like.
(13) antiulcer drug metoclopromide, histidine hydrochloride, lansoprazole, metoclopramide, pirenzepine, cimetidine, ranitidine, famotidine, urogastrone, oxethazaine, proglumide, omeprazole, sucralfate, sulpiride, cetraxate, gefarnate, aldioxa, teprenone, prostaglandin and the like.
(14) antiarrhythmic
   <1> Na channel blocker (e.g., quinidine, procainamide, disopyramide, ajmaline, lidocaine, mexiletine, phenitoin),
   <2> β-blocker (e.g., propranolol, alprenolol, bufetolol, oxprenolol, atenolol, acebutolol, metoprolol, bisoprolol, pindolol, carteolol, arotinolol,
   <3> K channel blocker (e.g., amiodarone),
   <4> Ca channel blocker (e.g., verapamil, diltiazem) and the like.
(15) hypotensive diuretic hexamethonium bromide, clonidine hydrochloride, hydrochlorothiazide, trichlormethiazide, furosemide, ethacrynic acid, bumetanide, mefruside, azosemide, spironolactone, potassium canrenoate, triamterene, amiloride, acetazolamide, D-mannitol, isosorbide, aminophyllin and the like.
(16) tranquilizer diazepam, lorazepam, oxazepam, chlordiazepoxide, medazepam, oxazolam, cloxazolam, clotiazepam, bromazepam, etizolam, fludiazepam, hydroxyzine and the like.
(17) antipsychotic chlorpromazine hydrochloride, prochlorperazine, trifluoperazine, thioridazine hydrochloride, perphenazine maleate, fluphenazine enanthate, prochlorperazine maleate, levomepromazine maleate, promethazine hydrochloride, haloperidol, bromperidol, spiperone, reserpine, clocapramine dihydrochloride, sulpiride, zotepine and the like.
(18) antitumor drug 6-O-(N-chloroacetylcarbamoyl)fumagillol, bleomycin, methotrexate, actinomycin D, mitomycin C, daunorubicin, adriamycin, neocarzinostatin, cytosine arabinoside, fluorouracil, tetrahydrofuryl-5-fluorouracil, picibanil, lentinan, levamisole, bestatin, azimexon, glycyrrhizin, doxorubicin hydrochloride, aclarubicin hydrochloride, bleomycin hydrochloride, peplomycin sulfate, vincristine sulfate, vinblastine sulfate, irinotecan hydrochloride, cyclophosphamide, melphalan, busulphan, thiotepa, procarbazine hydrochloride, cisplatin, azathioprine, mercaptopurine, tegafur, carmofur, cytarabine, methyltestosterone, testosterone propionate, testosterone enanthate, mepitiostane, fosfestol, chlormadinone acetate, leuprorelin acetate, buserelin acetate and the like.
(19) hypolipidemic drug clofibrate, ethyl 2-chloro-3-[4-(2-methyl-2-phenylpropoxy)phenyl]propionate (Chem. Pharm. Bull, 38, 2792-2796 (1990)), pravastatin, simvastatin, probucol, bezafibrate, clinofibrate, nicomol, cholestyramine, dextran sulfate sodium and the like.
(20) muscle relaxant pridinol, tubocurarine, pancuronium, tolperisone hydrochloride, chlorphenesin carbamate, baclofen, chlormezanone, mephenesin, chlorzoxazone, eperisone, tizanidine and the like.
(21) anticonvulsant phenytoin, ethosuximide, acetazolamide, chlordiazepoxide, trimethadione, carbamazepine, phenobarbital, primidone, sulthiame, sodium valproate, clonazepam, diazepam, nitrazepam and the like.
(22) antidepressant imipramine, clomipramine, noxiptiline, phenelzine, amitriptyline hydrochloride, nortriptyline hydrochloride, amoxapine, mianserin hydrochloride, maprotiline hydrochloride, sulpiride, fluvoxamine maleate, trazodone hydrochloride and the like.
(23) antiallergic drug diphenhydramine, chlorpheniramine, tripelennamine, metodilamine, clemizole, diphenylpyraline, methoxyphenamine, disodium cromoglycate, tranilast, repirinast, amlexanox, ibudilast, ketotifen, terfenadine, mequitazine, azelastine, epinastine, ozagrel hydrochloride, pranlkast hydrate, seratrodast and the like.
(24) cardiac trans-bioxocamphor, terephyllol, aminophyllin, etilefrine, dopamine, dobutamine, denopamine, aminophyllin, vesnarin, amrinone, pimobendan, ubidecarenone, digitoxin, digoxin, methyldigoxin, lanatoside C, G-strophanthin and the like.
(25) vasodilator oxyfedrine, diltiazem, tolazoline, hexobendine, bamethan, clonidine, methyldopa, guanabenz and the like.
(26) vasoconstrictor dopamine, dobutamine, denopamine and the like.
(27) hypotensive diuretic hexamethonium bromide, pentolinium, mecamylamine, ecarazine, clonidine, diltiazem, nifedipine and the like.
(28) antidiabetic drug tolbutamide, chlorpropamide, acetohexamide, glibenclamide, tolazamide, acarbose, epalrestat, troglitazone, glucagon, glymidine, glipzide, phenformin, buformin, metformin and the like.
(29) antinarcotic levallorphan, nalorphine, naloxone or a salt thereof and the like.
(30) fat-soluble vitamin
   <1> vitamin A:vitamin A₁, vitamin A₂ and retinol palmitate
   <2> vitamin D:vitamin D₁, D₂, D₃, D₄ and D₅
   <3> vitamin E:α-tocopherol, β-tocopherol, γ-tocopherol, δ-tocopherol, dl-α-tocopherol nicotinate
   <4> vitamin K:vitamin K₁, K₂, K₃ and K₄
   <5> folic acid (vitamin M) and the like.
(31) vitamin derivative various derivatives of vitamins, for example, vitamin D₃ derivatives such as 5,6-trans-cholecalciferol, 2,5-hydroxycholecalciferol, 1-α-hydroxycholecalciferol and the like, vitamin D₂ derivatives such as 5,6-trans-ergocalciferol and the like.
(32) antiasthmatic isoprenaline hydrochloride, salbutamol sulfate, procaterol hydrochloride, terbutaline sulfate, trimetoquinol hydrochloride, tulobuterol hydrochloride, orciprenaline sulfate, fenoterol hydrobromide, ephedrine hydrochloride, ipratropium bromide, oxitropium bromide, flutropium bromide, theophyline, aminophyllin, disodium cromoglycate, tranilast, repirinast, anrexanone, ibudilast, ketotifen, terfenadine, mequitazine, azelastine, epinastine, ozagrel hydrochloride, pranlkast hydrate, seratrodast, dexamethasone, prednisolone, hydrocortisone, beclometasone dipropionate and the like.
(33) therapeutic agent for pollakiuria/anischuria flavoxate hydrochloride and the like.
(34) atopic dermatitis disodium cromoglycate and the like.
(35) therapeutic agent for allergic rhinitis disodium cromoglycate, chlorpheniramine maleate, alimemazine tartrate, clemastine fumarate, homochlorcyclizine hydrochloride, terfenadine, mequitazine and the like.
(36) hypertensive drug dopamine, dobutamine, denopamine, digitoxin, digoxin, methyldigoxin, lanatoside C, G-strophanthin and the like.
(37) Others hydroxicam, diaserine, megestrol acetate, nicerogolin, prostaglandins and the like.

A combined use of the inventive Compound, or a salt thereof or a prodrug thereof and a combination drug provides the following effects.
(1) The dose of the inventive Compound, a salt thereof and a prodrug thereof can be reduced than a sole administration of the inventive Compound, or a salt thereof or a prodrug thereof.
(2) A synergistic therapeutic effect can be achieved against the above-mentioned sepsis, septic shock, inflammatory diseases, infectious diseases and the like.
(3) A broad range of therapeutic effect can be achieved against various diseases developed in association with viral infection and the like.

With regard to the use of the inventive Compound, or a salt thereof or a prodrug thereof and a combination drug, the inventive Compound, or a salt thereof or a prodrug thereof and the combination drug are free of any limitation on the timing of the administration, or the inventive Compound, a salt thereof or a prodrug thereof, and the combination drug may be simultaneously administered to the administration object, or may be administered with time difference. The dose of the combination drug follows a clinical dose and can be appropriately determined depending on the administration object, administration route, disease, combination and the like.

The mode of administration of the inventive Compound, a salt thereof or a prodrug thereof and the combination drug is not particularly limited, as long as the inventive Compound, a salt thereof or a prodrug thereof and the combination drug are combined for administration. While the mode of such drug and the like, for example, (1) administration of a preparation obtained by simultaneous addition of the inventive Compound, a salt thereof or a prodrug thereof and the combination drug, (2) simultaneous administration of two kinds of preparations obtained by separate preparation of the composition of the present invention and a pharmaceutical composition of the combination drug, by a single administration route, (3) time stagger administration of two kinds of preparations obtained by separate preparation of the composition of the present invention and a pharmaceutical composition of the combination drug, by a single administration route, (4) simultaneous administration of two kinds of preparations obtained by separate preparation of the composition of the present invention and a pharmaceutical composition of the combination drug, by different administration routes, (5) time stagger administration of two kinds of preparations obtained by separate preparation of the composition of the present invention and a pharmaceutical composition of the combination drug, by different administration routes (e.g., administration in the order of the composition of the present invention and then a pharmaceutical composition of the combination drug, or in a reversed order, and the like).

A pharmaceutical composition of the combination drug has low toxicity and can be administered safely by admixing the combination drug with, for example, a pharmacologically acceptable carrier according to a method known per se to give a pharmaceutical composition, such as tablets (inclusive of sugar-coated tablets and film-coated tablets), powders, granules, capsules (inclusive of soft capsules), liquids, injections, suppositories, sustained release agents and the like, for oral or parenteral (e.g., topical, rectal or intravenous administration) administration. An injection can be administered intravenously, intramuscularly, subcutaneously, into the organs or directly into the lesion.

As the pharmacologically acceptable carrier usable for the production of the pharmaceutical composition of a combination drug, there are mentioned various conventional organic or inorganic carriers as a material for the preparation. Examples thereof include excipients, lubricants, binders and disintegrators for solid preparations, and solvents, solubilizing aids, suspending agents, isotonic agents and soothing agents for liquid preparations. Where necessary, conventional additives such as antiseptics, antioxidants, coloring agents, sweeteners, absorbents, moistening agents and the like can be used appropriately in suitable amounts.

As the excipient, there are mentioned, for example, lactose, sucrose, D-mannitol, starch, corn starch, crystalline cellulose, light anhydrous silicic acid and the like.

As the lubricant, there are mentioned, for example, magnesium stearate, calcium stearate, talc, colloidal silica and the like.

As the binder, there are mentioned, for example, crystalline cellulose, saccharose, D-mannitol, dextrin, hydroxypropylcellulose, hydroxypropylmethylcellulose, polyvinylpyrrolidone, starch, sucrose, gelatin, methylcellulose, carboxymethylcellulose sodium and the like.

As the disintegrator, there are mentioned, for example, starch, carboxymethylcellulose, carboxymethylcellulose calcium, sodium carboxymethyl starch, L-hydroxypropylcellulose and the like.

As the solvent, there are mentioned, for example, injectable water, alcohol, propylene glycol, Macrogol, sesame oil, corn oil, olive oil and the like.

As the solubilizing aid, there are mentioned, for example, polyethylene glycol, propylene glycol, D-mannitol, benzyl benzoate, ethanol, tris-aminomethane, cholesterol, triethanolamine, sodium carbonate, sodium citrate and the like.

As the suspending agent, there are mentioned, for example, surfactants such as stearyl triethanolamine, sodium lauryl sulfate, lauryl aminopropionic acid, lecithin, benzalkonium chloride, benzethonium chloride, glyceryl monostearate and the like; hydrophilic polymers such as polyvinyl alcohol, polyvinylpyrrolidone, carboxymethylcellulose sodium, methylcellulose, hydroxymethylcellulose, hydroxyethylcellulose, hydroxypropylcellulose and the like.

As the isotonic agent, there are mentioned, for example, glucose, D-sorbitol, sodium chloride, glycerine, D-mannitol and the like.

As the soothing agent, there are mentioned, for example, benzyl alcohol and the like.

As the antiseptic, there are mentioned, for example, p-oxybenzoates, chlorobutanol, benzyl alcohol, phenethyl alcohol, dehydroacetic acid, sorbic acid and the like.

As the antioxidant, there are mentioned, for example, sulfite, ascorbic acid, α-tocopherol and the like.

When a combination drug is added to the composition of the present invention, the amount of the combination drug can be appropriately determined depending on the administration object, administration route, disease and the like, and can be adjusted as are the inventive Compound, a salt thereof and a prodrug thereof.

When the composition of the present invention and a pharmaceutical composition of the combination drug are used in combination, the content of the combination drug in the pharmaceutical composition of the combination drug can be appropriately determined depending on the administration object, administration route, disease and the like. It is generally about 0.01 - 100 wt%, preferably about 0.1 - 50 wt%, more preferably about 0.5 - 20 wt%, based on the preparation in total.

The content of the additive, such as a carrier, in the pharmaceutical composition of the combination drug varies depending on the form of the preparation. It is generally about 1 - 99.99 wt%, preferably about 10 - 90 wt%, based on the preparation in total.

Pharmaceutical composition of the combination drug can be prepared by a method known per se of which a generally known preparation process is used.

For example, a combination drug can be prepared into an aqueous injection together with a dispersant (e.g., Tween 80 (ATLASPOWDER, USA), HCO60 (NIKKO CHEMICALS), polyethylene glycol, carboxymethylcellulose, sodium arginate, hydroxypropylmethylcellulose, dextrin and the like, a stabilizing agent (e.g., ascorbic acid, sodium pyrosulfite and the like), a surfactant (e.g., polysorbate 80, Macrogol and the like), a solubilizing agent (e.g., glycerine, ethanol and the like), an isotonic agent (e.g., sodium chloride, potassium chloride, mannitol, sorbitol, glucose and the like), a pH adjusting agent (hydrochloric acid, sodium hydroxide and the like), a preservative (ethyl p-hydroxybenzoate, benzoic acid, methylparaben, propylparaben, benzyl alcohol and the like), a solubilizer (e.g., conc. glycerine, meglumine and the like), a solubilizing aid (e.g., propylene glycol, sucrose and the like), a soothing agent (e.g., glucose, benzyl alcohol and the like) and the like, or by dissolving, suspending or emulsifying in a vegetable oil (e.g., olive oil, sesame oil, cottonseed oil, corn oil and the like) or a solubilizing aid such as propylene glycol and the like to form an oil-based injection formulation.

In addition, a combination drug may be used instead of the inventive Compound, a salt thereof or a prodrug thereof to give an emulsion composition for injection of the present invention.

An oral formulation can be produced by a generally known method of compressing a combination drug with an addition of an excipient (e.g., lactose, sucrose, starch and the like), a disintegrant (e.g., starch, calcium carbonate and the like), a binder (e.g., starch, gum arabic, carboxymethyl cellulose, polyvinyl pyrrolidone, hydroxypropyl cellulose and the like) or a glidant (e.g., talc, magnesium stearate, polyethylene glycol 6000 and the like) as appropriate, followed by a coating process known per se for the purpose of masking a taste, forming an enteric coat, or achieving a sustained release. Such coating may, for example, be hydroxypropylmethyl cellulose, ethyl cellulose, hydroxymethyl cellulose, hydroxypropyl cellulose, polyoxyethylene glycol, Tween 80, Pluronic F68, cellulose acetate phthalate, hydroxypropylmethyl cellulose phthalate, hydroxymethyl cellulose acetate succinate, Eudragid (ROHME, Germany, a copolymer of methacrylic acid and acrylic acid), a dye (e.g., colcothar, titanium oxide and the like) as appropriate. The preparation for oral administration may be either a rapid release preparation or a sustained release preparation.

For example, when a suppository is produced, a combination drug may be formulated also as an oil-based or aqueous solid or semi-solid or liquid suppository by a method known per se. An oil-based suppository base may, for example, be a higher fatty glyceride (e.g., cocoa butter, WITEPSOL (DYNAMIT NOBEL, Germany) and the like), a middle fatty acid (e.g., MYGLYOL (DYNAMIT NOBEL, Germany) and the like), or a vegetable oil (e.g., sesame oil, soybean oil, cottonseed oil and the like) and the like as appropriate. An aqueous base may, for example, be a polyethylene glycols or a propylene glycol, and an aqueous gel base may, for example, be a natural gum, a cellulose derivative, a vinyl polymer, an acrylic polymer and the like.

Examples of the above-mentioned sustained release preparation include sustained release microcapsule and the like.

A sustained release microcapsule can be prepared by a method known per se, and for example, a sustained release preparation shown in the following [2] is preferably formed and administered.

A combination drug can be formed into a preparation for oral administration such as a solid preparation (e.g., powder, granule, tablet, and capsule) and the like, or a preparation for rectal administration such as a suppository and the like, depending on the kind of the drug.

In the following, [1] an injection of the combination drug and preparation thereof, [2] a sustained release preparation or a rapid release preparation of a combination drug and preparation thereof, and [3] a sublingual tablet, buccal or oral cavity rapid disintegrator of a combination drug and preparation thereof are concretely explained.

### [1] Injection and preparation thereof

An injection containing a combination drug dissolved in water is preferable. The injection may contain benzoate and/or salicylate.

The injection is obtained by dissolving both a combination drug and, where desired, benzoate and/or salicylate in water.

The salt of the above-mentioned benzoic acid and salicylic acid includes, for example, alkali metal salts such as sodium, potassium and the like, alkaline earth metal salts such as calcium, magnesium and the like, ammonium salt, meglumine salt, and organic acid salt such as tromethamol and the like, and the like.

The concentration of the combination drug in the injection is about 0.5 to 50 w/v%, preferably about 3 to 20 w/v%. The concentration of the benzoate and/or salicylate is preferably 0.5 to 50 w/v%, more preferably 3 to 20 w/v%.

The injection may contain additives generally used for injections, such as a stabilizing agent (e.g., ascorbic acid, sodium pyrosulfite and the like), a surfactant (e.g., polysorbate 80, Macrogol and the like), a solubilizing agent (e.g., glycerine, ethanol and the like), an isotonic agent (e.g., sodium chloride, potassium chloride and the like), a dispersing agent (e.g., hydroxypropylmethylcellulose, dextrin), a pH adjusting agent (hydrochloric acid, sodium hydroxide and the like), a preservative (ethyl p-oxybenzoate, benzoic acid and the like), a solubilizer (e.g., conc. glycerine, meglumine and the like), a solubilizing aid (e.g., propylene glycol, sucrose and the like), a soothing agent (e.g., glucose, benzyl alcohol and the like) and the like as appropriate. These additives are added in a proportion generally employed for injections.

The injection can be obtained by dissolving both the combination drug and, where desired, benzoate and/or salicylate, and where necessary, the above-mentioned additives in water. These may be dissolved in any order in a suitable manner as in conventional production of injections.

The injectable aqueous solution is preferably heated and, in the same manner as with conventional injections, subjected to, for example, sterilization by filtration, high pressure sterilization by heating and the like to provide an injection.

The injectable aqueous solution is preferably subjected to high pressure sterilization by heating at, for example, 100°C to 121°C for 5 min to 30 min.

It may be prepared into an antibacterial solution, so that it can be used as a preparation for plural subdivided administrations.

### [2] Sustained release preparation or rapid release preparation and preparation thereof

A sustained release preparation wherein a core containing a combination drug is covered on demand with a film forming agent, such as a water-insoluble material, a swellable polymer and the like, is preferable. For example, a sustained release preparation for oral administration once a day is preferable.

The water-insoluble material to be used for the film forming agent is, for example, cellulose ethers such as ethylcellulose, butylcellulose and the like; cellulose esters such as cellulose acetate, cellulose propionate and the like; polyvinyl esters such as poly(vinyl acetate), poly(vinyl butyrate) and the like; acrylic polymers such as acrylic acid/methacrylic acid copolymer, methyl methacrylate copolymer, ethoxyethyl methacrylate/cinnamoethyl methacrylate/aminoalkyl methacrylate copolymer, polyacrylic acid, polymethacrylic acid, methacrylic acid alkylamide copolymer, poly(methyl methacrylate), polymethacrylate, polymethacrylic amide, aminoalkyl methacrylate copolymer, poly(methacrylic anhydride) and glycidyl methacrylate copolymer, particularly Eudragits (Rohm Pharma) such as Eudragit RS-100, RL-100, RS-30D, RL-30D, RL-PO, RS-PO (ethyl acrylate-methyl methacrylate·ethyl methacrylate-trimethylammonium chloride copolymer), Eudragit NE-30D (methyl methacrylate-ethyl acrylate copolymer) and the like, and the like; hydrogenated oils such as hydrogenated castor oil (e.g., Lovely Wax (Freund Inc.)) and the like) and the like; waxes such as carnauba wax, fatty acid glycerine ester, paraffin and the like; polyglycerine fatty acid ester and the like.

As the swellable polymer, a polymer having an acidic dissociable group, which shows pH-dependent swelling, is preferable, and a polymer having an acidic dissociable group, which shows less swelling in an acidic range, such as in the stomach, but otherwise in a neutral range, such as in the small intestine and large intestine, is preferable.

Examples of the polymer having an acidic dissociable group, which shows pH-dependent swelling, include crosslinking type polyacrylic acid polymers such as Carbomer 934P, 940, 941, 974P, 980, 1342 and the like, polycarbophil, calcium polycarbophil (all mentioned above are the product of BF Goodrich), HI-BIS-WAKO 103, 104, 105, 304 (all being products of Waco Pure Chemicals Industries, Ltd.) and the like.

The film forming agent to be used for the sustained release preparation may further contain a hydrophilic material.

Examples of the hydrophilic material include polysaccharides optionally having a sulfuric acid group such as pullulan, dextrin, alkali metal salt of alginic acid and the like; polysaccharides having a hydroxy alkyl group or a carboxy alkyl group such as hydroxypropylcellulose, hydroxypropylmethylcellulose, carboxymethylcellulose sodium and the like; methylcellulose, polyvinylpyrrolidone, polyvinyl alcohol, polyethylene glycol and the like.

The content of the water-insoluble material of the film forming agent for a sustained release preparation is about 30 to about 90% (w/w), preferably about 35 to about 80% (w/w), more preferably about 40 to 75% (w/w), and the content of the swellable polymer is about 3 to about 30% (w/w), preferably about 3 to about 15% (w/w). The film forming agent may further contain a hydrophilic material, in which case the content of the hydrophilic material for film forming agent is not more than about 50% (w/w), preferably about 5 to about 40% (w/w), more preferably about 5 to about 35% (w/w). As used herein, the above-mentioned % (w/w) is a weight % relative to the film forming agent composition wherein the solvent (e.g., water, lower alcohol such as methanol, ethanol and the like) has been removed from the film forming liquid agent.

A sustained release preparation is produced by preparing a core containing a drug and coating the resulting core with a film forming liquid agent prepared by dissolving by heating or dissolving or dispersing in a solvent a water-insoluble material, a swellable polymer and the like, as exemplarily mentioned below.

### I. Preparation of core containing a drug

The form of the core containing a drug (hereinafter sometimes simply referred to as a core) to be coated with a film forming agent is not particularly limited, but it is preferably formed into particles such as granules, subtilized granules and the like.

When the core is made of granules or subtilized granules, the average particle size thereof is preferably about 150 to 2,000 µm, more preferably about 500 to about 1,400 µm.

The core can be prepared by a typical production method. For example, a drug is mixed with suitable excipients, binders, disintegrators, lubricants, stabilizers and the like, and subjected to wet extrusion granulation, fluidized bed granulation and the like.

The drug content of the core is about 0.5 to about 95% (w/w), preferably about 5.0 to about 80% (w/w), more preferably about 30 to about 70% (w/w).

Examples of the excipient to be contained in the core include saccharides such as sucrose, lactose, mannitol, glucose and the like, starch, crystalline cellulose, calcium phosphate, corn starch and the like. Of these, crystalline cellulose and corn starch are preferable.

Examples of the binder include polyvinyl alcohol, hydroxypropylcellulose, polyethylene glycol, polyvinylpyrrolidone, Pluronic F68, gum arabic, gelatin, starch and the like. Examples of the disintegrator include carboxymethylcellulose calcium (ECG505), crosscarmellose sodium (Ac-Di-Sol), crosslinked polyvinylpyrrolidone (Crospovidone), low substituted hydroxypropylcellulose (L-HPC) and the like. Of these, hydroxypropylcellulose, polyvinylpyrrolidone and low substituted hydroxypropylcellulose are preferable. Examples of the lubricant and coagulation preventive include talc, magnesium stearate and inorganic salts thereof, and examples of the lubricant include polyethylene glycol and the like. Examples of the stabilizer include acids such as tartaric acid, citric acid, succinic acid, fumaric acid, maleic acid and the like.

The core can be also prepared, besides the above-mentioned production methods, by, for example, rolling granulation wherein a drug or a mixture of a drug and an excipient, a lubricant and the like is added by small portions while spraying a binder dissolved in a suitable solvent such as water, lower alcohol (e.g., methanol, ethanol and the like) and the like on an inert carrier particles to be the center of the core, a pan coating method, a fluidized bed coating method or a melt granulating method. Examples of the inert carrier particle include those prepared from sucrose, lactose, starch, crystalline cellulose and waxes, which preferably has an average particle size of about 100 µm to about 1,500 µm.

To separate the drug contained in the core from the film forming agent, the surface of the core may be coated with a protective agent. Examples of the protective agent include the aforementioned hydrophilic material, water-insoluble material and the like. As the protective agent, preferably polyethylene glycol, polysaccharides having a hydroxy alkyl group or a carboxy alkyl group, more preferably hydroxypropylmethylcellulose and hydroxypropylcellulose are used. The protective agent may contain, as a stabilizer, an acid such as tartaric acid, citric acid, succinic acid, fumaric acid, maleic acid and the like, and a lubricant such as talc and the like. When the protective agent is used, the amount to be coated is about 1 to about 15% (w/w), preferably about 1 to about 10% (w/w), more preferably about 2 to about 8% (w/w), relative to the core.

The protective agent can be coated by a typical coating method. Specifically, the protective agent is, for example, spray-coated to the core by a fluidized bed coating method, a pan coating method, and the like.

### II. Coating of core with a film forming agent

The core obtained in the aforementioned I is coated with a film forming liquid agent prepared by dissolving by heating or dissolving or dispersing in a solvent the aforementioned water-insoluble material, a pH-dependent swellable polymer, and a hydrophilic material to provide a sustained release preparation.

For coating a film forming liquid agent to a core, for example, a spray coating method and the like can be employed.

The composition ratio of the water-insoluble material, swellable polymer or hydrophilic material in the film forming liquid agent is suitably determined such that the content of each component of the coating film becomes the aforementioned content.

The coating amount of the film forming agent is about 1 to about 90% (w/w), preferably about 5 to about 50% (w/w), more preferably about 5 to 35% (w/w), relative to the core (exclusive of the coating amount of protective agent).

As the solvent for the film forming liquid agent, water and organic solvents can be used alone or in a mixture of the both. The mixing ratio (water/organic solvent: weight ratio) of water and the organic a solvent in the mixture can vary within the range of 1 to 100%, which is preferably 1 to about 30%. The organic solvent is not subject to any particular limitation as long as it dissolves the water-insoluble material. For example, lower alcohols such as methyl alcohol, ethyl alcohol, isopropyl alcohol, n-butyl alcohol and the like, lower alkanone such as acetone and the like, acetonitrile, chloroform, methylene chloride and the like are used. Of these, lower alcohol is preferable, and ethyl alcohol and isopropyl alcohol are particularly preferable. Water and a mixture of water and an organic solvent is preferably used as a solvent of the film forming agent. Where necessary, the film forming liquid agent may contain an acid such as tartaric acid, citric acid, succinic acid, fumaric acid, maleic acid and the like for the stabilization of the film forming liquid agent.

When spray coating is employed, the method follows a conventional coating method, which is specifically spray coating of a film forming liquid agent to the core by, for example, a fluidized bed coating method, a pan coating method and the like. Where necessary, talc, titanium oxide, magnesium stearate, calcium stearate, light anhydrous silicic acid and the like may be added as a lubricant, and glycerine fatty acid ester, hydrogenated castor oil, triethyl citrate, cetyl alcohol, stearyl alcohol and the like may be added as a plasticizer.

After coating of a film forming agent, an antistatic agent such as talc and the like may be added as necessary.

A rapid release preparation may be a liquid (solution, suspension, emulsion and the like) or a solid (particles, pill, tablet and the like). While an oral administration agent, and a parenteral administration agent, such as injection and the like, are used, with preference given to an agent for oral administration.

A rapid release preparation may generally contain, in addition to the drug which is an active ingredient, carriers, additives and excipients (hereinafter sometimes simply referred to as an excipient) conventionally used in the field of preparation. The excipient for a preparation is not subject to any particular limitation as long as it is conventionally employed as an excipient for a preparation. For example, the excipient for the oral solid preparation includes lactose, starch, corn starch, crystalline cellulose (Asahi Kasei Corporation, Avicel PH101 and the like), powder sugar, granulated sugar, mannitol, light anhydrous silicic acid, magnesium carbonate, calcium carbonate, L-cysteine and the like, preferably corn starch and mannitol and the like. These excipients may be used alone or in combination. The content of the excipient is, for example, about 4.5 to about 99.4 w/w%, preferably about 20 to about 98.5 w/w%, more preferably about 30 to about 97 w/w%, relative to the total amount of the rapid release preparation.

The drug content of the rapid release preparation is appropriately determined from the range of about 0.5 to about 95%, preferably about 1 to about 60%, relative to the total amount of the rapid release preparation.

When the rapid release preparation is an oral solid preparation, it generally contains a disintegrator in addition to the above-mentioned components. Examples of the disintegrator include calcium carboxymethylcellulose (manufactured by Gotoku Yakuhin, ECG-505), crosscarmellose sodium (e.g., Asahi Kasei Corporation, Actisol), Crospovidone (e.g., Colicone CL, BASF), low substituted hydroxypropylcellulose (Shin-Etsu Chemical Co., Ltd.), carboxymethyl starch (Matsutani Chemical Industry Co., Ltd.), sodium carboxymethyl starch (manufactured by Kimura Sangyo, Exprotab), partially α starch (PCS, Asahi Kasei Corporation) and the like. For example, one capable of disintegrating granules by water absorption, swelling, forming a channel between the active ingredient constituting the core and an excipient upon contact with water and the like can be used. These disintegrators can be used alone or in combination. The amount of the disintegrator is appropriately determined depending on the kind of the combination drug to be used and amount thereof, design of the release preparation and the like. It is generally about 0.05 to about 30 w/w%, preferably about 0.5 to about 15 w/w%, relative to the total amount of the rapid release preparation.

When the rapid release preparation is an oral solid preparation, the oral solid preparation may further contain, in addition to the above-mentioned composition, routine additives used for solid preparation on demand. Examples of the additive include a binder (e.g., sucrose, gelatin, gum arabic powder, methylcellulose, hydroxypropylcellulose, hydroxypropylmethylcellulose, carboxymethylcellulose, polyvinylpyrrolidone, Pullulan, dextrin and the like), a lubricant (e.g., polyethylene glycol, magnesium stearate, talc, light anhydrous silicic acid (e.g., Aerosil (Nippon Aerosil)), a surfactant (e.g., anionic surfactant such as sodium alkylsulfate and the like, non-ionic surfactant such as polyoxyethylene fatty acid ester and polyoxyethylenesorbitan fatty acid ester, polyoxyethylene castor oil derivative and the like, and the like), a coloring agent (e.g., synthetic color, caramel, iron oxide red, titanium oxide, riboflavins), where necessary, a corrigent (e.g., a sweetener, flavor and the like), an absorbent, an antiseptic, a moistening agent, an antistatic agent and the like. As the stabilizer, an organic acid such as tartaric acid, citric acid, succinic acid, fumaric acid and the like may be added.

Examples of the above-mentioned binder preferably include hydroxypropylcellulose, polyethylene glycol, polyvinylpyrrolidone and the like.

The rapid release preparation can be prepared based on the conventional preparation method, by mixing each of the aforementioned components, and where necessary, further kneading and forming. The above-mentioned mixing can be performed by a conventional method, such as mixing, kneading and the like. Specifically, for example, when a rapid release preparation is formed into particles, a vertical granulator, a universal kneader (manufactured by Hata Tekkosho), a fluidized bed granulator FD-5S (Powrex Corporation) and the like are used for mixing, which is followed by granulating by wet extrusion granulation, fluidized bed granulation and the like, to give the preparation, as in the preparation of the core of the aforementioned sustained release preparation.

The rapid release preparation and the sustained release preparation thus obtained may be used as they are. Alternatively, after suitable separate preparation along with an excipient for a preparation and the like according to a conventional method, they may be administered simultaneously or at optional administration intervals. Alternatively, they may be prepared into a single preparation for oral administration (e.g., granule, subtilized granule, tablet, capsule and the like) as they are or along with excipient for preparation and the like as appropriate. The both preparations are converted to granules or subtilized granules and filled in a single capsule and the like to give a preparation for oral administration.

### [3] A sublingual tablet, buccal or oral cavity rapid disintegrator and preparation thereof

The sublingual tablet, buccal preparation and oral cavity rapid disintegrator may be a solid preparation such as tablet and the like or an oral cavity mucous membrane adhesion tablet (film).

As the sublingual tablet, buccal or oral cavity rapid disintegrator, a preparation containing a combination drug and an excipient is preferable. It may contain auxiliaries such as a lubricant, an isotonic agent, a hydrophilic carrier, a water dispersible polymer, a stabilizer and the like. For easy absorption and enhanced bioavailability, β-cyclodextrin or β-cyclodextrin derivative (e.g., hydroxypropyl-β-cyclodextrin and the like) and the like may be contained.

Examples of the above-mentioned excipient include lactose, sucrose, D-mannitol, starch, crystalline cellulose, light anhydrous silicic acid and the like. Examples of the lubricant include magnesium stearate, calcium stearate, talc, colloidal silica and the like, particularly magnesium stearate and colloidal silica are preferable. Examples of the isotonic agent include sodium chloride, glucose, fructose, mannitol, sorbitol, lactose, saccharose, glycerine, urea and the like, particularly mannitol is preferable. Examples of the hydrophilic carrier include swellable hydrophilic carriers such as crystalline cellulose, ethylcellulose, crosslinked polyvinylpyrrolidone, light anhydrous silicic acid, silicic acid, dicalcium phosphate, calcium carbonate and the like, particularly crystalline cellulose (e.g., microcrystalline cellulose and the like) is preferable. Examples of the water dispersible polymer include gum (e.g., gum tragacanth, acacia gum, guar gum), alginate (e.g., sodium alginate), cellulose derivative (e.g., methylcellulose, carboxymethylcellulose, hydroxymethylcellulose, hydroxypropylcellulose, hydroxypropylmethylcellulose), gelatin, water-soluble starch, polyacrylic acid (e.g., carbomer), polymethacrylic acid, polyvinyl alcohol, polyethylene glycol, polyvinylpyrrolidone, polycarbofil, ascorbate, palmitate and the like, with preference given to hydroxypropylmethylcellulose, polyacrylic acid, alginate, gelatin, carboxymethylcellulose, polyvinylpyrrolidone, polyethylene glycol and the like. Particularly, hydroxypropylmethylcellulose is preferable. Examples of the stabilizer include cysteine, thiosorbitol, tartaric acid, citric acid, sodium carbonate, ascorbic acid, glycine, sodium sulfite and the like, particularly, citric acid and ascorbic acid are preferable.

The sublingual tablet, buccal and oral cavity rapid disintegrator can be produced by mixing a combination drug and an excipient by a method known per se. Where desired, the above-mentioned auxiliaries such as a lubricant, an isotonic agent, a hydrophilic carrier, a water dispersible polymer, a stabilizer, a coloring agent, a sweetener, an antiseptic and the like may be contained. After mixing the above-mentioned components simultaneously or with time staggering, the mixture is compassion formed under pressure to give sublingual tablet, buccal tablet or oral cavity rapid disintegrating tablet. To achieve a suitable hardness, a solvent such as water, alcohol and the like is used to moisten or wet as necessary before and after the compassion forming. After the forming, the tablets are dried.

When a mucous membrane adhesion tablet (film) is produced, a combination drug and the above-mentioned water dispersible polymer (preferably, hydroxypropylcellulose, hydroxypropylmethylcellulose), an excipient and the like are dissolved in a solvent such as water and the like, and the obtained solution is cast to give a film. In addition, an additive such as a plasticizer, a stabilizer, an antioxidant, a preservative, a coloring agent, a sweetener and the like may be added. To impart suitable elasticity to the film, glycols such as polyethylene glycol, propylene glycol and the like may be added, and to increase adhesion of the film to the oral cavity mucous membrane lining, bioadhesive polymer (e.g., polycarbofil, carbopol) may be added. The casting includes pouring the solution on a non-adhesive surface, spreading the solution in a uniform thickness (preferably about 10 to 1000 µ) with a coating tool such as doctor blade and the like and drying the solution to give a film. The film thus formed may be dried at room temperature or under heating and cut into a desired surface area.

Example of preferable oral cavity rapid disintegrator is a solid rapid diffusing administration agent having a net structure of a combination drug and water soluble or water diffusable carrier which are inert to the combination drug. The net structure can be obtained by evaporation of a solvent from the solid composition consisting of a solution obtained by dissolving a combination drug in a suitable solvent and the carrier.

The composition of the oral cavity rapid disintegrator preferably contains, in addition to the combination drug, a matrix forming agent and a secondary component.

Examples of the matrix forming agent include animal proteins or vegetable proteins such as gelatins, dextrins, soybeans, wheat, psyllium seed protein and the like ; rubber substances such as gum arabic, guar gum, agar, xanthan and the like; polysaccharides; alginic acids; carboxymethylcelluloses; carragheenans; dextrans; pectins; synthetic polymers such as polyvinylpyrrolidone and the like; a material derived from a gelatin-gum arabic complex and the like. In addition, saccharides such as mannitol, dextrose, lactose, galactose, trehalose and the like; cyclic saccharides such as cyclodextrin and the like; inorganic salts such as sodium phosphate, sodium chloride, aluminum silicate and the like; amino acid having 2 to 12 carbon atoms such as glycine, L-alanine, L-aspartic acid, L-glutamic acid, L-hydroxyproline, L-isoleucine, L-leucine, L-phenylalanine and the like are exemplified.

It is possible to introduce one or more matrix forming agents into a solution or suspension before preparation into a solid. Such matrix forming agent may exist with a surfactant or without a surfactant. The matrix forming agent can form a matrix, and also can help maintain the diffusion of the inventive Compound or a combination drug in the solution or suspension.

The composition may contain a secondary component such as a preservative, an antioxidant, a surfactant, a thickener, a coloring agent, a pH adjusting agent, a flavor, a sweetener, a taste masking reagent and the like. Examples of a suitable coloring agent include red, black and yellow ferric oxides and FD&C dyes of Elis and Eberald, such as FD&C blue NO. 2, FD&C red No. 40 and the like. A suitable flavor contains mint, raspberry, licorice, orange, lemon, grapefruit, caramel, vanilla, cherry, grape flavor and a combination of these. Suitable pH adjusting agent includes citric acid, tartaric acid, phosphoric acid, hydrochloric acid and maleic acid. Suitable sweetener includes aspartame, acesulfame K, thaumatin and the like. Suitable taste masking agent includes sodium bicarbonate, ion exchange resin, cyclodextrin inclusion compound, adsorbent substance and microcapsuled apomorphine.

The preparation contains a combination drug generally in a proportion of about 0.1 to about 50 wt%, preferably about 0.1 to about 30 wt%, and a preparation (such as the above-mentioned sublingual tablet, buccal and the like) which is capable of dissolving 90% or more of a combination drug in water for about 1 min - about 60 min, preferably about 1 min to about 15 min, more preferably about 2 min - about 5 min, and an oral cavity rapid disintegrator that disintegrates within 1 to 60 sec, preferably 1 to 30 sec, more preferably 1 to 10 sec, after being placed in an oral cavity is preferable.

The content of the above-mentioned excipient in the whole preparation is about 10 to about 99 wt%, preferably about 30 to about 90 wt%. The content of the β-cyclodextrin or β-cyclodextrin derivative relative to the whole preparation is 0 to about 30 wt%. The content of the lubricant relative to the whole preparation is about 0.01 to about 10 wt%, preferably about 1 to about 5 wt%. The content of the isotonic agent relative to the whole preparation is about 0.1 to about 90 wt%, preferably about 10 to about 70 wt%. The content of the hydrophilic carrier relative to the whole preparation is about 0.1 to about 50 wt%, preferably about 10 to about 30 wt%. The content of the water dispersible polymer relative to the whole preparation is about 0.1 to about 30 wt%, preferably about 10 to about 25 wt%. The content of the stabilizer relative to the whole preparation is about 0.1 to about 10 wt%, preferably about 1 to about 5 wt%. The above-mentioned preparation may contain additives such as a coloring agent, a sweetener, an antiseptic and the like as necessary.

While the dose of the pharmaceutical composition of the combination drug varies depending on the kind of the combination drug, the patient's age, body weight and condition, the dosage form, the mode and the period of the administration, the amount of the combination drug may, for example, be generally about 0.01 to about 1000 mg/kg, preferably about 0.01 to about 100 mg/kg, more preferably about 0.1 to about 100 mg/kg, most preferably about 0.1 to about 50 mg/kg, and particularly about 1.5 to about 30 mg/kg per day in a patient (adult weighing about 60 kg), said daily dose being given intravenously all at once or in several portions during a day. It is a matter of course that a lower daily dose may be sufficient or an excessive dose may be required since the dose may vary depending on various factors as discussed above.

The combination drug may be contained in any amount as long as a side effect does not pose a problem. While the daily dose of the combination drug may vary depending on the disease state, the age, sex, body weight and difference in sensitivity of the administration object, timing and interval of administration, characteristics, dispensing and kind of the pharmaceutical preparation, the kind of active ingredient and the like and is not particularly limited, the amount of the drug is generally about 0.001 to 2000 mg, preferably about 0.01 to 500 mg, more preferably about 0.1 to 100 mg, per 1 kg body weight of mammal by oral administration, which is generally administered all at once or in 2 to 4 portions during a day.

When the composition of the present invention and the pharmaceutical composition of a combination drug are concurrently administered, they may be administered at the same time, or the pharmaceutical composition of a combination drug may be administered first, and then the composition of the present invention may be administered. Alternatively, the composition of the present invention may be administered first, and then the pharmaceutical composition of a combination drug may be administered. For time stagger administration, the time difference varies depending on the active ingredient to be administered, dosage form and administration route. For example, when the pharmaceutical composition of a combination drug is to be administered first, the composition of the present invention is administered within 1 min to 3 days, preferably 10 min to 1 day, more preferably 15 min to 1 hour, after the administration of the pharmaceutical composition of a combination drug. When the composition of the present invention is to be administered first, the pharmaceutical composition of a combination drug is administered within 1 min to 1 day, preferably 10 min to 6 hours, more preferably 15 min to 1 hour, after the administration of the composition of the present invention.

### [Examples]

Next, the present invention is further described with referring to Reference Examples, Examples and Comparative examples. The following examples or the like are to describe the present invention and the invention is not limited to this.

A ¹H NMR spectrum was determined by a VARIAN GEMINI 200 (200 MHz) spectrometer using tetramethyl silane as an internal standard and represented as the entire δ values in ppm. The number in a bracket [()] when a solvent mixture was employed is the volume ratio of each solvent. A% is a% by weight unless otherwise specified. The ratio of the solvents in a chromatography on silica gel is the volume ratio of the solvents to be admixed.

A more polar diastereomer means a diastereomer having a smaller Rf value when determined by a normal phase thin layer chromatography under a same condition (for example using ethyl acetate/hexane as an eluent), whereas a less polar diastereomer means a diastereomer having a larger Rf value in such determination.

The meanings of the abbreviations as used in the Examples are as follows:
s: singlet d: doublet: t: triplet q: quartet
dd: double doublet tt: triple triplet m: multiplet
br: broad J: coupling constant

The Reference Example A mentioned below can be produced according to Reference Example of WO99/46424 and Reference Example B can be produced according to Example of WO99/46424.

### [Reference Example A]

Reference Example A1 ethyl 2-sulfo-1-cyclohexene-1-carboxylate
Reference Example A2 ethyl 2-chlorosulfonyl-1-cyclohexene-1-carboxylate
Reference Example A3 ethyl 2-chlorosulfonyl-1-cyclopentene-1-carboxylate
Reference Example A4 ethyl 2-chlorosulfonyl-1-cycloheptene-1-carboxylate
Reference Example A5 6-[N-(4-chloro-2-fluorophenyl)sulfamoyl]-1-cyclohexene-1-carboxylic acid sodium salt
Reference Example A6 1-(3-fluoro-4-nitrophenyl)-1H-1,2,4-triazole
Reference Example A7 1-(4-amino-3-fluorophenyl)-1H-1,2,4-triazole
Reference Example A8 methyl 4-benzyloxycarbonylamino-3-chlorobenzoate
Reference Example A9 4-benzyloxycarbonylamino-3-chlorobenzoic acid
Reference Example A10 tert-butyl N-(4-benzyloxycarbonylamino-3-chlorobenzoyl)glycinate
Reference Example A11 tert-butyl N-(4-amino-3-chlorobenzoyl)glycinate
Reference Example A12 6-[N-(2,4-difluorophenyl)sulfamoyl]-1-cyclohexene-1-carboxylic acid
Reference Example A13 ethyl 2-mercapto-5-phenyl-1-cyclohexene-1-carboxylate
Reference Example A14 ethyl 2-chlorosulfonyl-5-phenyl-1-cyclohexene-1-carboxylate
Reference Example A15 ethyl 5-tert-butyl-2-mercapto-1-cyclohexene-1-carboxylate
Reference Example A16 ethyl 5-tert-butyl-2-chlorosulfonyl-1-cyclohexene-1-carboxylate
Reference Example A17 ethyl 5,5-dimethyl-2-mercapto-1-cyclohexene-1-carboxylate
Reference Example A18 ethyl 2-chlorosulfonyl-5,5-dimethyl-1-cyclohexene-1-carboxylate

### [Reference Example B]

Reference Example B1 ethyl 6-[N-(4-chloro-2-fluorophenyl)sulfamoyl]-1-cyclohexene-1-carboxylate (compound 1)
Reference Example B2 ethyl 6-[N-(4-chloro-2-fluorophenyl)-N-methylsulfamoyl]-1-cyclohexene-1-carboxylate (compound 2) Reference Example B3 ethyl 6-[N-(2,4-difluorophenyl)sulfamoyl]-1-cyclohexene-1-carboxylate (compound 3)
Reference Example B4 ethyl 6-[N-(2,6-diisopropylphenyl)sulfamoyl]-1-cyclohexene-1-carboxylate (compound 4)
Reference Example B5 ethyl 6-[N-(4-nitrophenyl)sulfamoyl]-1-cyclohexene-1-carboxylate (compound 5)
Reference Example B6 ethyl 6-(N-phenylsulfamoyl)-1-cyclohexene-1-carboxylate (compound 6)
ethyl 2-(N-phenylsulfamoyl)-1-cyclohexene-1-carboxylate (compound 7)
Reference Example B7 ethyl 2-[N-(4-chloro-2-fluorophenyl)sulfamoyl]-1-cyclohexene-1-carboxylate (compound 9)
Reference Example B8 2-(4-methoxyphenyl)-4,5,6,7tetrahydro-1,2-benzisothiazol-3(2H)-one 1,1-dioxide (compound 67) ethyl 2-[N-(4-methoxyphenyl)sulfamoyl]-1-cyclohexene-1-carboxylate (compound 8)
Reference Example B9 ethyl 6-[N-(2-fluorophenyl)sulfamoyl]-1-cyclohexene-1-carboxylate (compound 10)
Reference Example B10 ethyl 6-[N-(3-fluorophenyl)sulfamoyl]-1-cyclohexene-1-carboxylate (compound 11)
Reference Example B11 2-(4-fluorophenyl)-4,5,6,7-tetrahydro-1,2-benzisothiazol-3(2H)-one 1,1-dioxide (compound 68)
ethyl 6-[N-(4-fluorophenyl)sulfamoyl]-1-cyclohexene-1-carboxylate (compound 12)
ethyl 2-[N-(4-fluorophenyl)sulfamoyl]-1-cyclohexene-1-carboxylate (compound 18)
Reference Example B12 ethyl 6-[N-(2,6-difluorophenyl)sulfamoyl]-1-cyclohexene-1-carboxylate (compound 13)
Reference Example B13 ethyl 6-[N-(2,3-difluorophenyl)sulfamoyl]-1-cyclohexene-1-carboxylate (compound 14)
Reference Example B14 ethyl 6-[N-(2,5-difluorophenyl)sulfamoyl]-1-cyclohexene-1-carboxylate (compound 15)
Reference Example B15 ethyl 6-[N-(3,4-difluorophenyl)sulfamoyl]-1-cyclohexene-1-carboxylate (compound 16)
Reference Example B16 ethyl 6-[N-(3,5-difluorophenyl)sulfamoyl]-1-cyclohexene-1-carboxylate (compound 17)
Reference Example B17 1-ethyl 6-[N-(2,4-difluorophenyl)sulfamoyl]-1-cyclohexene-1-carboxylate (compound 19)
d-ethyl 6-[N-(2,4-difluorophenyl)sulfamoyl]-1-cyclohexene-1-carboxylate (d-ethyl 6-[N-(2,4-difluorophenyl)sulfamoyl]cyxlohex-1-en-1-carboxylate)(compound 20)
Reference Example B18 ethyl 6-[N-(2-ethoxycarbonylphenyl)sulfamoyl]-1-cyclohexene-1-carboxylate (compound 21)
Reference Example B19 methyl 6-[N-(2,4-difluorophenyl)sulfamoyl]-1-cyclohexene-1-carboxylate (compound 22)
Reference Example B20 propyl 6-[N-(2,4-difluorophenyl)sulfamoyl]-1-cyclohexene-1-carboxylate (compound 23)
Reference Example B21 methyl 6-[N-(4-chloro-2-fluorophenyl)sulfamoyl]-1-cyclohexene-1-carboxylate (compound 24)
Reference Example B22 isopropyl 6-[N-(2,4-difluorophenyl)sulfamoyl]-1-cyclohexene-1-carboxylate (compound 25)
Reference Example B23 ethyl 6-[N-(2-methoxycarbonylphenyl)sulfamoyl]-1-cyclohexene-1-carboxylate (compound 26)
Reference Example B24 ethyl 6-[N-(2-fluoro-4-methylphenyl)sulfamoyl]-1-cyclohexene-1-carboxylate (compound 27)
Reference Example B25 ethyl 6-[N-(2-chlorophenyl)sulfamoyl]-1-cyclohexene-1-carboxylate (ethyl 6-[N-(2-chlorophenyl)sulfamoyl]cyclohex-1-en-1-carboxylate) (compound 28)
Reference Example B26 ethyl 6-[N-(2-chloro-4-fluorophenyl)sulfamoyl]-1-cyclohexene-1-carboxylate (compound 29)
Reference Example B27 ethyl 6-[N-(4-chlorophenyl)sulfamoyl]-1-cyclohexene-1-carboxylate (compound 30)
Reference Example B28 ethyl 6-[N-(2,3,4-trifluorophenyl)sulfamoyl]-1-cyclohexene-1-carboxylate (compound 31)
Reference Example B29 isobutyl 6-[N-(2,4-difluorophenyl)sulfamoyl]-1-cyclohexene-1-carboxylate (compound 32)
Reference Example B30 butyl 6-[N-(2,4-difluorophenyl)sulfamoyl]-1-cyclohexene-1-carboxylate (compound 33)
Reference Example B31 ethyl 6-[N-(4-bromo-2-fluorophenyl)sulfamoyl]-1-cyclohexene-1-carboxylate (compound 34)
Reference Example B32 ethyl 6-[N-(2,4-dichlorophenyl)sulfamoyl]-1-cyclohexene-1-carboxylate (compound 35)
Reference Example B33 ethyl 6-[N-(2-acetoxyphenyl)sulfamoyl]-1-cyclohexene-1-carboxylate (compound 36)
Reference Example B34 ethyl 6-[N-(3-chlorophenyl)sulfamoyl]-1-cyclohexene-1-carboxylate (compound 37)
Reference Example B35 ethyl 6-[N-(2,3-dichlorophenyl)sulfamoyl]-1-cyclohexene-1-carboxylate (compound 38)
Reference Example B36 ethyl 6-[N-(2-ethylphenyl)sulfamoyl]-1-cyclohexene-1-carboxylate (compound 39)
Reference Example B37 ethyl 6-[N-[4-(2H-1,2,3-triazol-2-yl)phenyl]sulfamoyl]-1-cyclohexene-1-carboxylate (compound 40) Reference Example B38 ethyl 6-[N-(2,5-dichlorophenyl)sulfamoyl]-1-cyclohexene-1-carboxylate (compound 41)
Reference Example B39 ethyl 6-[N-(2-trifluoromethoxyphenyl)sulfamoyl]-1-cyclohexene-1-carboxylate (compound 42)
Reference Example B40 ethyl 6-[N-(2,4,5-trifluorophenyl)sulfamoyl]-1-cyclohexene-1-carboxylate (compound 43)
Reference Example B41 ethyl 6-[N-[4-(2H-tetrazol-2-yl)phenyl]sulfamoyl]-1-cyclohexene-1-carboxylate (compound 44)
Reference Example B42 ethyl 6-[N-(2-chloro-4-methylphenyl)sulfamoyl]-1-cyclohexene-1-carboxylate (ethyl 6-[N-(2-chloro-4-methylphenyl)sulfamoyl]cyclohex-1-en-1-carboxylate)(compound 45)
Reference Example B43 ethyl 6-[N-(4-fluoro-2-methylphenyl)sulfamoyl]-1-cyclohexene-1-carboxylate (compound 46)
Reference Example B44 ethyl 6-[N-(2,6-dichlorophenyl)sulfamoyl]-1-cyclohexene-1-carboxylate (compound 47)
Reference Example B45 ethyl 6-[N-[4-(1H-tetrazol-1-yl)phenyl]sulfamoyl]-1-cyclohexene-1-carboxylate (compound 48) Reference Example B46 ethyl 6-[N-(4-(1H-1,2,3-triazol-1-yl)phenyl]sulfamoyl]-1-cyclohexene-1-carboxylate (compound 49)
Reference Example B47 ethyl 6-[N-(2-trifluoromethylphenyl)sulfamoyl]-1-cyclohexene-1-carboxylate (compound 50)
Reference Example B48 ethyl 6-[N-(4-methoxycarbonylphenyl)sulfamoyl]-1-cyclohexene-1-carboxylate (compound 51)
Reference Example B49 benzyl 6-[N-(2,4-difluorophenyl)sulfamoyl]-1-cyclohexene-1-carboxylate(compound 52)
Reference Example B50 ethyl 6-[N-[4-[2,3-bis(tert-butoxycarbonyl)guanidinomethyl]phenyl]sulfamoyl]-1-cyclohexene-1-carboxylate (compound 53)
Reference Example B51 ethyl 6-[N-(2-chloro-4-methoxycarbonylphenyl)sulfamoyl]-1-cyclohexene-1-carboxylate (compound 54)
Reference Example B52 ethyl 6-[N-(2-chloro-4-cyanophenyl)sulfamoyl]-1-cyclohexene-1-carboxylate (compound 55)
Reference Example B53 2-hydroxyethyl 6-[N-(2,4-difluorophenyl)sulfamoyl]-1-cyclohexene-1-carboxylate (compound 56)
Reference Example B54 ethyl 6-[N-[2-fluoro-4-(1H-1,2,4-triazol-1-yl)phenyl]sulfamoyl]-1-cyclohexene-1-carboxylate (compound 57)
Reference Example B55 ethyl 2-[N-(2,4-difluorophenyl)sulfamoyl]-1-cyclopentene-1-carboxylate (compound 66)
ethyl 5-[N-(2,4-difluorophenyl)sulfamoyl]-1-cyclopentene-1-carboxylate (compound 58)
Reference Example B56 tert-butyl [6-[N-(2,4-difluorophenyl)sulfamoyl]-1-cyclohexen-1-yl]carbonyloxyacetate (compound 59)
Reference Example B57 [6-[N-(2,4-difluorophenyl)sulfamoyl]-1-cyclohexen-1-yl]carbonyloxyacetic acid (compound 60)
Reference Example B58 ethyl 7-[N-(2,4-difluorophenyl)sulfamoyl]-1-cycloheptene-1-carboxylate (compound 61)
Reference Example B59 ethyl 6-[N-[2-chloro-4-(N-tert-butoxycarbonylmethylcarbamoyl)phenyl]sulfamoyl]-1-cyclohexene-1-carboxylate (compound 62)
Reference Example B60 ethyl 6-[N-[2-chloro-4-(N-ethoxycarbonylmethylcarbamoyl)phenyl]sulfamoyl]-1-cyclohexene-1-carboxylate (compound 63)
Reference Example B61 ethyl 5-[N-(2-chloro-4-fluorophenyl)sulfamoyl]-1-cyclopentene-1-carboxylate (compound 64)
Reference Example B62 2-[4-(2,2,3,3,3-pentafluoropropoxy)phenyl]-4,5,6,7-tetrahydro-1,2-benzisothiazol-3(2H)-one 1,1-dioxide (compound 69)
Reference Example B63 ethyl 7-[N-(2-chloro-4-fluorophenyl)sulfamoyl]-1-cycloheptene-1-carboxylate (compound 65)
Reference Example B64 2-(2,4-difluorophenyl)-5,6,7,7a-tetrahydro-1,2-benzisothiazol-3(2H)-one 1,1-dioxide (compound 70)
Reference Example B65 ethyl 6-[N-(2-chloro-4-fluorophenyl)sulfamoyl]-1-cyclohexene-1-carboxylate (compound 29)
Reference Example B66 1-ethyl 6-[N-(2-chloro-4-fluorophenyl)sulfamoyl]-1-cyclohexene-1-carboxylate (compound 71)
d-ethyl 6-[N-(2-chloro-4-fluorophenyl)sulfamoyl]-1-cyclohexene-1-carboxylate (ethyl (6R)-6-[N-(2-chloro-4-fluorophenyl)sulfamoyl]cyclohex-1-en-1-carboxylate)(compound 72)
Reference Example B67 ethyl 6-[N-(2-bromo-4-fluorophenyl)sulfamoyl]-1-cyclohexene-1-carboxylate (compound 73)
Reference Example B68 ethyl 6-[N-(4-bromo-2-chlorophenyl)sulfamoyl]-1-cyclohexene-1-carboxylate (compound 74)
Reference Example B69 high polarity diastereomer (compound 75) and low polarity diastereomer (compound 76) of ethyl 6-[N-(2,4-difluorophenyl)sulfamoyl]-3-phenyl-1-cyclohexene-1-carboxylate
Reference Example B70 high polarity diastereomer (compound 77) and low polarity diastereomer (compound 78) of ethyl 6-[N-(2-chloro-4-fluorophenyl)sulfamoyl]-3-phenyl-1-cyclohexene-1-carboxylate
Reference Example B71 high polarity diastereomer (compound 79) and low polarity diastereomer (compound 80) of ethyl 6-[N-(2,4-difluorophenyl)sulfamoyl]-3-tert-butyl-1-cyclohexene-1-carboxylate
Reference Example B72 high polarity diastereomer (compound 81) and low polarity diastereomer (compound 82) of ethyl 6-[N-(2-chloro-4-fluorophenyl)sulfamoyl]-3-tert-butyl-1-cyclohexene-1-carboxylate
Reference Example B73 ethyl 6-[N-(2,4-difluorophenyl)sulfamoyl]-3,3-dimethyl-1-cyclohexene-1-carboxylate (compound 83)
Reference Example B74 ethyl 6-[N-(2-chloro-4-fluorophenyl)sulfamoyl]-3,3-dimethyl-1-cyclohexene-1-carboxylate (compound 84)
Reference Example B75 ethyl 3-bromo-6-[N-(2,4-difluorophenyl)sulfamoyl]-1-cyclohexene-1-carboxylate (compound 85)

Specific examples are shown in Tables 1 to 5.

**[Table 1-1]**

| | | | | |
|---|---|---|---|---|
| 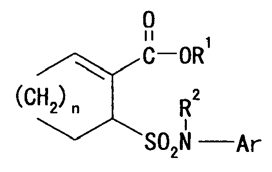 | | | | |

| Comp . No. | R¹ | R² | Ar | n |
|---|---|---|---|---|
| 1 | C₂H₅ | H | 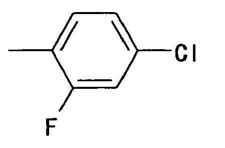 | 2 |
| 2 | C₂H₅ | CH₃ | 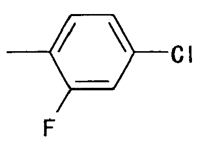 | 2 |
| 3 | C₂H₅ | H | 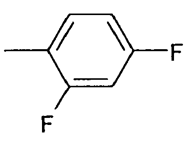 | 2 |
| 4 | C₂H₅ | H | 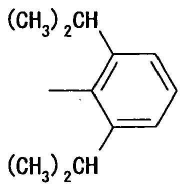 | 2 |
| 5 | C₂H₅ | H | 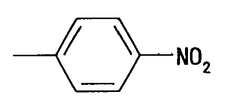 | 2 |
| 6 | C₂H₅ | H | 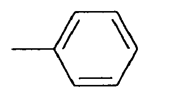 | 2 |
| 10 | C₂H₅ | H | 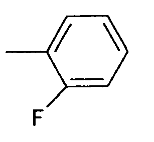 | 2 |

**[Table 1-2]**

| Comp. No. | R¹ | R² | Ar | n |
|---|---|---|---|---|
| 11 | C₂H₅ | H | 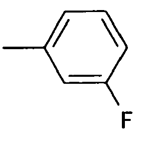 | 2 |
| 12 | C₂H₅ | H | 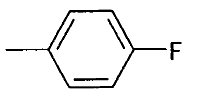 | 2 |
| 13 | C₂H₅ | H | 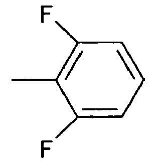 | 2 |
| 14 | C₂H₅ | H | 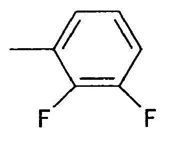 | 2 |
| 15 | C₂H₅ | H | 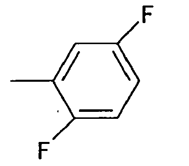 | 2 |
| 16 | C₂H₅ | H | 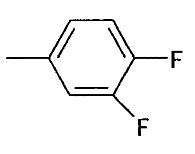 | 2 |
| 17 | C₂H₅ | H | 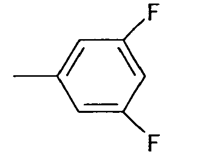 | 2 |
| 19 (1-form) | C₂H₅ | H | 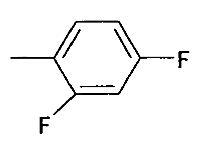 | 2 |

**[Table 1-3]**

| Comp.No. | R¹ | R² | Ar | n |
|---|---|---|---|---|
| 20 (d-form) | C₂H₅ | H | 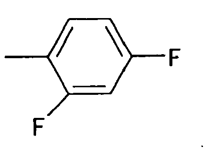 | 2 |
| 21 | C₂H₅ | H | 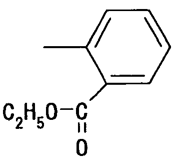 | 2 |
| 22 | CH₃ | H | 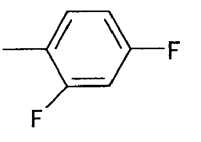 | 2 |
| 23 | (CH₂)₂CH₃ | H | 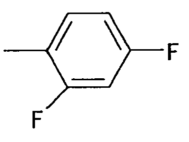 | 2 |
| 24 | CH₃ | H | 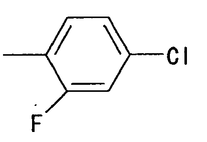 | 2 |
| 25 | CH (CH₃)₂ | H | 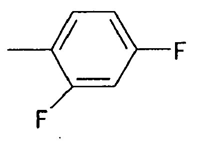 | 2 |
| 2 6 | C₂H₅ | H | 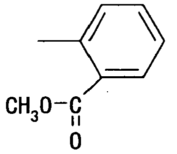 | 2 |
| 27 | C₂H₅ | H | 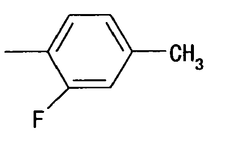 | 2 |

**[Table 1-4]**

| Comp.No. | R¹ | R² | Ar | n |
|---|---|---|---|---|
| 28 | C₂H₅ | H | 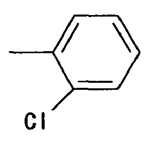 | 2 |
| 29 | C₂H₅ | H | 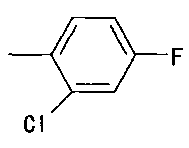 | 2 |
| 30 | C₂H₅ | H | 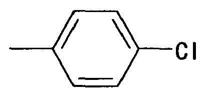 | 2 |
| 31 | C₂H₅ | H | 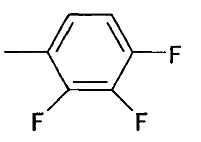 | 2 |
| 32 | CH₂CH(CH₃)₂ | H | 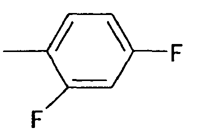 | 2 |
| 33 | (CH₂)₃CH₃ | H | 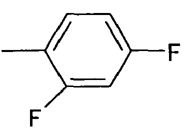 | 2 |
| 34 | C₂H₅ | H | 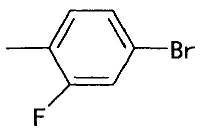 | 2 |
| 35 | C₂H₅ | H | 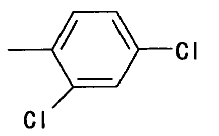 | 2 |
| 36 | C₂H₅ | H | 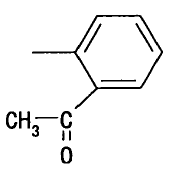 | 2 |

**[Table 1-5]**

| Comp.No. | R¹ | R² | Ar | n |
|---|---|---|---|---|
| 37 | C₂H₅ | H | 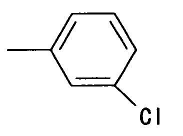 | 2 |
| 38 | C₂H₅ | H | 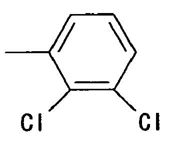 | 2 |
| 39 | C₂H₅ | H | 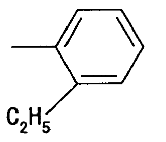 | 2 |
| 40 | C₂H₅ | H | 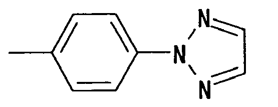 | 2 |
| 41 | C₂H₅ | H | 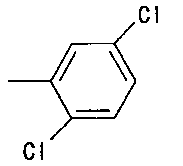 | 2 |
| 42 | C₂H₅ | H | 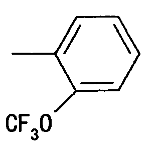 | 2 |
| 43 | C₂H₅ | H | 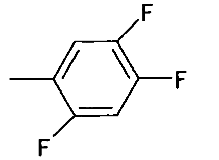 | 2 |
| 44 | C₂H₅ | H | 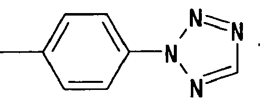 | 2 |
| 45 | C₂H₅ | H | 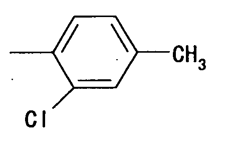 | 2 |

**[Table 1-6]**

| Comp.No. | R¹ | R² | Ar | n |
|---|---|---|---|---|
| 46 | C₂H₅ | H | 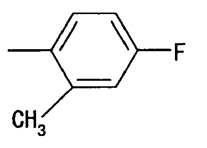 | 2 |
| 47 | C₂H₅ | H | 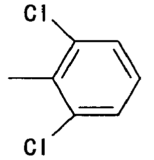 | 2 |
| 48 | C₂H₅ | H | 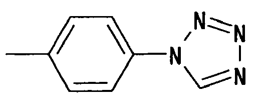 | 2 |
| 49 | C₂H₅ | H | 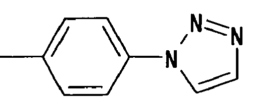 | 2 |
| 50 | C₂H₅ | H | 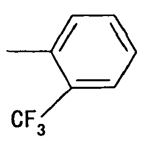 | 2 |
| 51 | C₂H₅ | H | 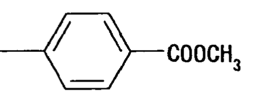 | 2 |
| 52 | 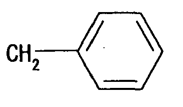 | H | 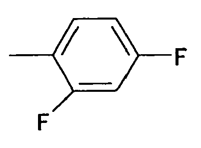 | 2 |
| 53 | C₂H₅ | H | 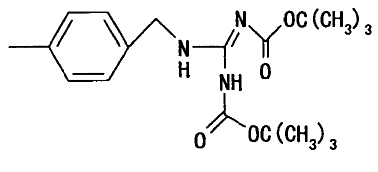 | 2 |
| 54 | C₂H₅ | H | 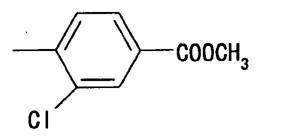 | 2 |

**[Table 1-7]**

| Comp . No . | R¹ | R² | Ar | n |
|---|---|---|---|---|
| 55 | C₂H₅ | H | 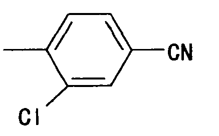 | 2 |
| 56 | (CH₂)₂O | H | 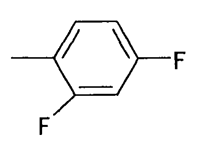 | 2 |
| 57 | C₂H₅ | H | 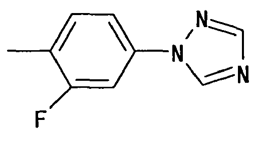 | 2 |
| 58 | C₂H₅ | H | 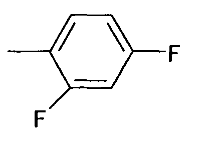 | 1 |
| 59 | CH₂COOC(CH₃)₃ | H | 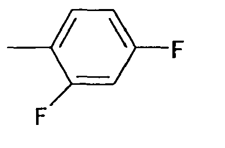 | 2 |
| 60 | CH₂COOH | H | 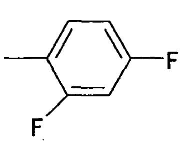 | 2 |
| 61 | C₂H₅ | H | 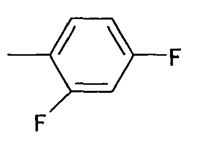 | 3 |
| 62 | C₂H₅ | H | 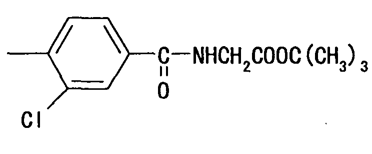 | 2 |

**[Table 1-8]**

| Comp . No . | R¹ | R² | Ar | n |
|---|---|---|---|---|
| 63 | C₂H₅ | H | 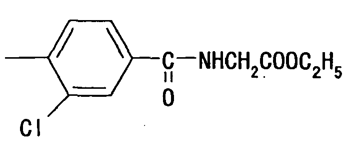 | 2 |
| 64 | C₂H₅ | H | 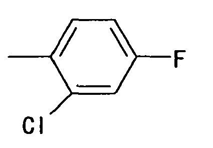 | 1 |
| 65 | C₂H₅ | H | 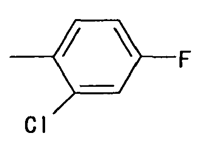 | 3 |
| 71 (1-form) | C₂H₅ | H | 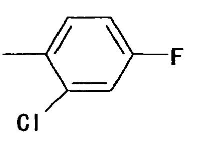 | 2 |
| 72 (d-form) | C₂H₅ | H | 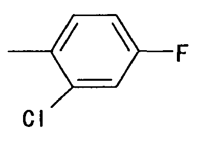 | 2 |
| 73 | C₂H₅ | H | 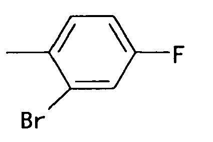 | 2 |
| 74 | C₂H₅ | H | 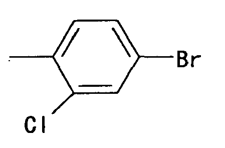 | 2 |

**[Table 2]**

| | | | |
|---|---|---|---|
| 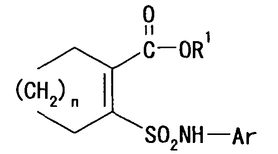 | | | |

| Comp. No. | R¹ | Ar | n |
|---|---|---|---|
| 7 | C₂H₅ | 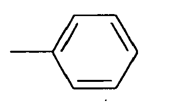 | 2 |
| 8 | C₂H₅ | 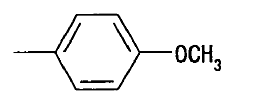 | 2 |
| 9 | C₂H₅ | 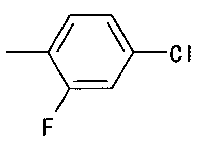 | 2 |
| 18 | C₂H₅ | 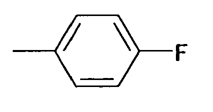 | 2 |
| 66 | C₂H₅ | 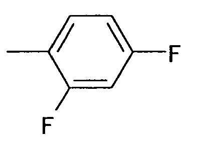 | 1 |

**[Table 3]**

| | | |
|---|---|---|
| 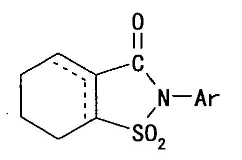 | | |

| Comp.No. | 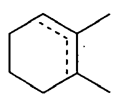 | Ar |
|---|---|---|
| 67 | 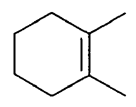 | 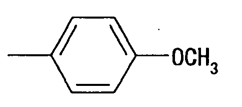 |
| 68 | 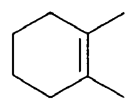 | 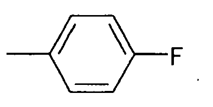 |
| 69 | 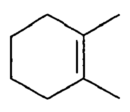 | 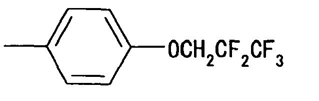 |
| 70 | 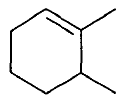 | 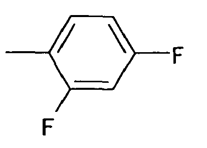 |

**[Table 4-1]**

| | | | | |
|---|---|---|---|---|
| 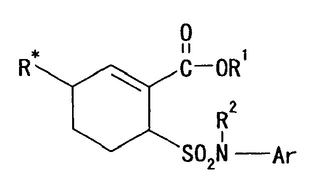 | | | | |

| Comp.No. | R¹ | R² | R* | Ar |
|---|---|---|---|---|
| 75 (high polarity diastereomer) | C₂H₅ | H | 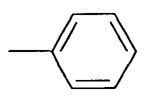 | 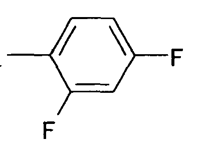 |
| 76 (low polarity diastereomer) | C₂H₅ | H | 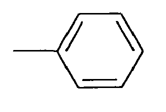 | 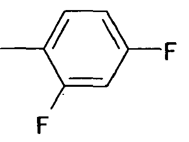 |
| 77 (high polarity diastereomer) | C₂H₅ | H | 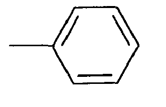 | 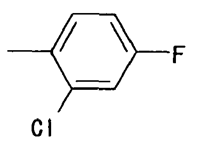 |
| 78 (low polarity diastereomer) | C₂H₅ | H | 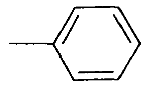 | 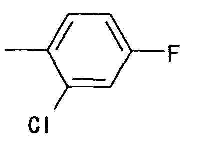 |
| 79 (high polarity diastereomer) | C₂H₅ | H | C (CH₃)₃ | 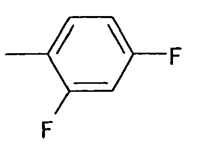 |
| 80 (low polarity diastereomer) | C₂H₅ | H | C (CH₃) | 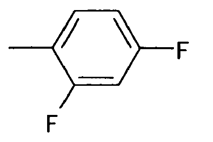 |

**[Table 4-2]**

| Comp.No. | R¹ | R² | R* | Ar |
|---|---|---|---|---|
| 81 (high polarity diastereomer) | C₂H₅ | H | C(CH₃)₃ | 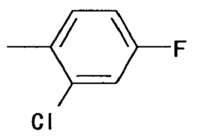 |
| 82 (low polarity diastereomer) | C₂H₅ | H | C(CH₃)₃ | 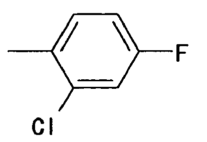 |
| 85 | C₂H₅ | H | Br | 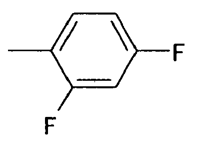 |

**[Table 5]**

| | |
|---|---|
| 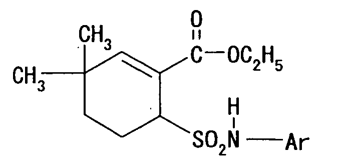 | |
| Comp. No. | Ar |
| 83 | 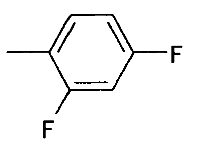 |
| 84 | 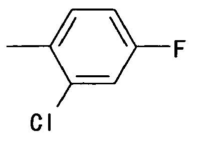 |

The Reference Example c mentioned below can be produced according to Reference Example of WO01/10826 and Reference Example D can be produced according to Example of WO01/10826.

### [Reference Example C]

### Reference Example C1

To a solution of ethyl 6-[N-(2,4-difluorophenyl)sulfamoyl]-1-cyclohexene-1-carboxylate (1 g, synthesized according to the method disclosed in JP-A-10-056492) and phenylmethanethiol (719 mg) in N,N-dimethylformamide (20 ml) was added dropwise under ice-cooling 1,8-diazabicyclo[5,4,0]-7-undecene (441 mg) and the mixture was stirred at room temperature for 18 h. The reaction mixture was diluted with ethyl acetate (100 ml), washed with water (70 ml x 2) and saturated brine (70 ml), and dried over anhydrous sodium sulfate. The solvent was evaporated and the obtained residue was purified by flash silica gel column chromatography (eluent: toluene) to give ethyl 6-(benzylsulfanyl)-1-cyclohexene-1-carboxylate (673 mg) as a colorless oil.
¹H-NMR (CDCl₃) δ : 1.27 (3H, t, J = 7.0Hz), 1.55-2.36 (6H, m), 3.76 (1H, m), 3.86 (2H, s), 4.19 (2H, q, J = 7.0Hz), 6.95 (1H, t, J = 4.0Hz), 7.22-7.39 (5H, m).

### Reference Example C2

In the same manner as in Reference Example Cl, ethyl 6-[N-(2,4-difluorophenyl)sulfamoyl]-1-cyclohexene-1-carboxylate (1 g) and (4-methoxyphenyl)methanethiol (893 mg) were reacted to give ethyl 6-[(4-methoxybenzyl)-sulfanyl]-1-cyclohexene-1-carboxylate (848 mg) as a colorless oil.
¹H-NMR (CDCl₃) δ : 1.28 (3H, t, J = 7.0Hz), 1.57-2.32 (6H, m), 3.74 (1H, m), 3.80 (3H, s), 3.82 (2H, s), t 4.20 (2H, q, J = 7.0Hz), 6.84 (2H, d, J = 8.4Hz), 6.94 (1H, t, J = 4.0Hz), 7.29 (2H, d, J = 8.4Hz).

### Reference Example C3

In the same manner as in Reference Example C1, ethyl 6-[N-(2,4-difluorophenyl)sulfamoyl]-1-cyclohexene-1-carboxylate (455 mg) and (2,4-difluorophenyl)-methanethiol (421 mg) were reacted to give ethyl 6-[(2,4-difluorobenzyl)sulfanyl]-1-cyclohexene-1-carboxylate (185 mg) as a colorless oil.
¹H-NMR (CDCl₃) δ: 1.26 (3H, t, J = 7.0Hz), 1.60-2.40 (6H, m), 3.78 (1H, m), 3.84 (2H, s), 4.18 (2H, q, J = 7.0Hz), 6.76-6.88 (2H, m), 6.97 (1H, t, J = 4.4Hz), 7.34-7.46 (1H, m).

### Reference Example C4

In the same manner as in Reference Example Cl, ethyl 6-[N-(2,4-difluorophenyl)sulfamoyl]-1-cyclohexene-1-carboxylate (835 mg) and (2-chloro-4-fluorophenyl)-methanethiol (853 mg) were reacted to give ethyl 6-[(2-chloro-4-fluorobenzyl)sulfanyl]-1-cyclohexene-1-carboxylate (625 mg) as a colorless oil.
¹H-NMR (CDCl₃) δ: 1.26 (3H, t, J = 7.0Hz), 1.56-2.36 (6H, m), 3.82 (1H, m), 3.94 (2H, s), 4.19 (2H, q, J = 7.0Hz), 6.96 (1H, td, J = 8.6Hz, 2.6Hz), 6.98 (1H, m), 7.12 (1H, dd, J= 8.6Hz, 2.6Hz), 7.46 (1H, dd, J= 8.6Hz, 6.0Hz).

### Reference Example C5

3-Pyranone (20.0 g) was reacted in the same manner as described in Tetrahedron., vol. 19, p. 1625 (1963) to give ethyl 5-hydroxy-3,6-dihydro-2H-pyran-4-carboxylate (7.52 g) as a colorless oil.
¹H-NMR (CDCl₃) δ: 1.32 (3H, t, J = 7.2 Hz), 2.31-2.38 (2H, m), 3.79 (2H, t, J = 5.6 Hz), 4.14 (2H, t, J = 1.8 Hz), 4.24 (2H, q, J = 7.2 Hz), 11.85 (1H, s).
SIMS: 172 (M⁺)

### Reference Example C6

Ethyl 5-hydroxy-3,6-dihydro-2H-pyran-4-carboxylate (12.9 g) was reacted in the same manner as described in Tetrahedron., vol. 30, p. 3753 (1974) to give ethyl 5-sulfanyl-3,6-dihydro-2H-pyran-4-carboxylate (12.0 g) as a pale-blue oil.

¹H-NMR (CDCl₃) δ: 1.32 (3H, t, J = 7.2 Hz), 2.42-2.50 (2H, m), 3.70 (1H, s), 3.84 (2H, t, J = 5.6 Hz), 4.22 (2H, t, J = 2.2 Hz), 4.25 (2H, q, J = 7.2 Hz).
elemental analysis value: as C₈H₁₂O₃S
Calculated (%):C,51.04; H,6.43; S, 17.03.
Found (%):C,50.99; H,6.54; S, 16.91.

### Reference Example C7

Sodium peroxoborate tetrahydrate (24.5 g) was added to acetic acid (130 ml) and the mixture was heated to 50-55°C. Thereto was added dropwise a solution of ethyl 5-sulfanyl-3,6-dihydro-2H-pyran-4-carboxylate (10.0 g) in acetic acid (30 ml) over 2 hours(hrs). The mixture was stirred at 50-55°C for 3 hrs, and the reaction mixture was concentrated under reduced pressure. To the residue was added acetonitrile (230 ml) and the mixture was stirred at room temperature for 2 days, and the resultant insoluble material was removed off by filtration and washed with acetonitrile (70 ml). The filtrate and washing were combined and the mixture was concentrated under reduced pressure. The residue was dissolved in acetonitrile (160 ml) and the mixture was stirred at room temperature for 6 hrs. The resultant insoluble material was removed off by filtration, and the filtrate was concentrated under reduced pressure. To the residue, diisopropyl ether (100 ml) was added, and the precipitated insoluble material was filtered off to give 4-(ethoxycarbonyl)-5,6-dihydro-2H-pyran-3-sulfonic acid as a pale-yellow oil (27.6 g) containing an inorganic product.
¹H-NMR(DMSO-d₆) δ: 1.19 (3H, t, J = 7.2 Hz), 2.17-2.21 (2H, m), 3.65 (2H, t, J = 5.5 Hz), 4.04 (2H, q, J = 7.2 Hz), 4.16 (2H, t, J = 2.4 Hz).

### Reference Example C8

4-(Ethoxycarbonyl)-5,6-dihydro-2H-pyran-3-sulfonic acid (27.5 g) was dissolved in thionyl chloride (82.6 ml) and the mixture was stirred at room temperature -> 85°C for 3 hrs. The reaction mixture was concentrated to dryness under reduced pressure and the residue was dissolved in ethyl acetate (100 ml). The obtained solution was partitioned by adding dilute brine (120 ml). The ethyl acetate layer was washed twice with saturated brine (50 ml) and dried over anhydrous sodium sulfate. The solvent was evaporated and the obtained residue was purified by silica gel column chromatography (eluent:ethyl acetate/hexane=1/7->1/5). The objective product was concentrated under reduced pressure and the crystals produced by freezing were washed with hexane to give ethyl 5-(chlorosulfonyl)-3,6-dihydro-2H-pyran-4-carboxylate (7.81 g) as pale-yellow crystals.

¹H-NMR (CDCl₃) δ: 1.37 (3H, t, J = 7.2 Hz), 2.62-2.70 (2H, m), 3.87 (2H, t, J = 5.5 Hz), 4.34 (2H, q, J = 7.2 Hz), 4.53 (2H, t, J = 2.6 Hz).
elemental analysis value: as C₈H₁₁ClO₅S
Calculated (%): C,37.73; H,4.35.
Found (%): C,37.64; H,4.27.

### [Reference Example D]

### Reference Example D1

To a solution of ethyl 6-(benzylsulfanyl)-1-cyclohexene-1-carboxylate (100 mg) obtained in Reference Example C1 in methylene chloride (3 ml) was added m-chlorobenzoic acid (196 mg) under ice-cooling, and the mixture was stirred at room temperature for 1 hr. To the reaction solution, saturated aqueous sodium hydrogencarbonate solution (20 ml) was added and the mixture was extracted with ethyl acetate (20 ml x 2). The ethyl acetate layer was washed with saturated aqueous sodium hydrogencarbonate solution (20 ml), water (20 ml) and saturated brine (20 ml), and dried over anhydrous sodium sulfate. The solvent was evaporated and the obtained residue was purified by flash silica gel chromatography (eluent: hexane->ethyl acetate/hexane = 1/30) and crystallized from hexane to give ethyl 6-(benzylsulfonyl)-1-cyclohexene-1-carboxylate (compound 86, 106 mg) as white crystals.
¹H-NMR (CDCl₃) δ: 1.34 (3H, t, J = 7.2Hz), 1.41-2.50 (6H, m), 4.28 (2H, q, J = 7.2Hz), 4.29 (1H, d, J= 13.8Hz), 4.35 (1H, m), 4.55 (1H, d, J= 13.8Hz), 7.37-7.45 (4H, m) 7.50-7.55 (2H, m).
elemental analysis value: as C₁₆H₂₀O₄S.0.5H₂O
Calculated (%): C, 60.55; H, 6.67
Found (%): C, 60.98; H, 6.32.

### Reference Example D2

In the same manner as in Reference Example D1, ethyl 6-[(4-methoxybenzyl)sulfanyl]-1-cyclohexene-1-carboxylate (98 mg) obtained in Reference Example C2 was reacted to give ethyl 6-[(4-methoxybenzyl)sulfonyl]-1-cyclohexene-1-carboxylate (compound 87, 88 mg) as white crystals.
¹H-NMR (CDCl₃) δ: 1.34 (3H, t, J = 7.0Hz), 1.42-2.50 (6H, m), 3.82 (3H, s), 4.21 (1H, d, J = 13.6Hz), 4.28 (2H, q, J = 7.0Hz), 4.31 (1H, m), 4.50 (1H, d, J = 13.6Hz), 6.92 (2H, d, J = 8.8Hz), 7.41 (1H, t, J = 3.6Hz), 7.47 (2H, d, J = 8.8Hz). Elemental analysis value: as C₁₇H₂₂O₅S
Calculated (%): C, 60.33; H, 6.55
Found (%): C, 60.42; H, 6.58.
Reference Example D3

In the same manner as in Reference Example D1, ethyl 6-[(2,4-difluorobenzyl)sulfanyl]-1-cyclohexene-1-carboxylate (161 mg) obtained in Reference Example C3 was reacted to give ethyl 6-[(2,4-difluorobenzyl)sulfonyl]-1-cyclohexene-1-carboxylate (compound 88, 134 mg) as white crystals.
¹H-NMR (CDCl₃) δ: 1.32 (3H, t, J = 7.0Hz), 1.59-2.50 (6H, m), 4.27 (2H, q, J = 7.0Hz), 4.35 (1H, d, J = 14.OHz), 4.39 (1H, m), 4.51 (1H, d, J = 14.0Hz), 6.83-6.96 (2H, m), 7.42 (1H, t, J = 4.0Hz), 7.49-7.61 (1H, m).
Elemental analysis value: as C₁₆H₁₈F₂O₉S
Calculated (%) : C, 55.80; H, 5.27
Found (%) : C, 55.95; H, 5.40.

### Reference Example D4

In the same manner as in Reference Example D1, ethyl 6-[(2-chloro-4-fluorobenzyl)sulfanyl]-1-cyclohexene-1-carboxylate (509 mg) obtained in Reference Example C4 was reacted to give ethyl 6-[(2-chloro-4-fluorobenzyl)-sulfonyl]-1-cyclohexene-1-carboxylate (compound 89, 422 mg) as white crystals.
¹H-NMR (CDCl₃)δ: 1.32 (3H, t, J = 7.0Hz), 1.55-2.52 (6H, m), 4.25 (2H, q, J = 7.0Hz), 4.41 (1H, d, J = 5.6Hz), 4.59 (2H, s), 7.03 (1H, td, J = 8.4Hz, 2.6Hz), 7.21 (1H, dd, J=8.4Hz, 2.6Hz), 7.42 (1H, t, J = 4.0Hz), 7.62 (1H, dd, J = 8.4Hz, 6.2Hz).
Elemental analysis value: as C₁₆H₁₈ClFO₄S
Calculated (%): C, 53.26; H, 5.03
Found (%): C, 53.08; H, 4.95.
Reference Example D5

Ethyl 6-[(2-chloro-4-fluorobenzyl)sulfonyl]-1-cyclohexene-1-carboxylate (compound 89, 100 mg) obtained in Reference Example D4 was resolved in two enantiomer by high performance liquid chromatography (CHIRALPAK AD; eluent: hexane/ethanol 8/2). The eluants were filtered through a 0.45 µm filter, concentrated and crystallized from hexane to respectively give ethyl (-)-6-[(2-chloro-4-fluorobenzyl)sulfonyl]-1-cyclohexene-1-carboxylate (compound 90, 50 mg) and ethyl (+)-6-[(2-chloro-4-fluorobenzyl) sulfonyl]-1-cyclohexene-1-carboxylate (compound 91, 49 mg) each as white crystals.

### Compound 90

¹H-NMR (CDCl₃) δ: 1.32 (3H, t, J = 7.0Hz), 1.56-2.55 (6H, m), 4.26 (2H, q, J = 7.0Hz), 4.42 (1H, d, J = 5.6Hz), 4.59 (2H, s), 7.03 (1H, td, J = 8.6Hz, 2.4Hz), 7.21 (1H, dd, J= 8.6Hz, 2.4Hz), 7.42 (1H, t, J = 4.2Hz), 7.61 (1H, dd, J = 8.6Hz, 6.0Hz).
Elemental analysis value: as C₁₆H₁₈ClFO₄S
Calculated (%): C, 53.26; H, 5.03
Found (%): C, 53.24; H, 4.85.
[α]_{D}²⁰ -97.0° (c = 0.5, in methanol).

### Compound 91

¹H-NMR (CDCl₃) δ: 1.32 (3H, t, J = 7.0Hz), 1.56-2.55 (6H, m), 4.26 (2H, q, J = 7.0Hz), 4 . 42 (1H, d, J = 6.2Hz), 4.59 (2H, s), 7.03 (1H, td, J = 8.6Hz, 2.4Hz), 7.21 (1H, dd, J= 8.6Hz, 2.4Hz), 7.42 (1H, t, J = 4.4Hz), 7.60 (1H, dd, J = 8.6Hz, 6.OHz) .
Elemental analysis value: as C₁₆H₁₈ClFO₄S
Calculated (%): C, 53.26; H, 5.03
Found (%): C, 53.29; H, 4.82.
[α]_{D}²⁰ +95.0° (c =0.5, in methanol).

### Reference Example D6

2,4-Difluoroaniline (0.45g) was dissolved in ethyl acetate (10 ml) and triethylamine (0.55 mg) was added to the obtained solution under ice-cooling. Then, a solution of ethyl 5-(chlorosulfonyl)-3,6-dihydro-2H-pyran-4-carboxylate (0.69 g) obtained in Reference Example C8 in ethyl acetate (4 ml) was added dropwise. The reaction mixture was stirred under a nitrogen stream at 0°C for 30 min and at room temperature for 5.8 h. The reaction mixture was diluted with ethyl acetate and washed successively with water (50 ml), 0.5N hydrochloric acid (50 ml), water (50 ml x 2) and saturated brine (50 ml). The ethyl acetate layer was dried over magnesium sulfate and the solvent was evaporated under reduced pressure. The residue was purified by silica gel column chromatography (eluent: ethyl acetate/hexane=1/2). The objective fraction was concentrated under reduced pressure and the residue was crystallized from a mixture of ethyl acetate and diisopropyl ether to give ethyl 3-[(2,4-difluorophenyl)-sulfamoyl]-3,6-dihydro-2H-pyran-4-carboxylate (compound 92; 0.57 g) as white crystals.
¹H-NMR(DMSO-d₆) δ: 1.14 (3H, t, J = 7.0 Hz), 3.69 (1H, dd, J = 12.8 Hz, 3.0 Hz), 4.08 (2H, q, J = 7.0 Hz), 4.25 (2H, s), 4.33 (1H, d, J = 1.8 Hz), 4.41-4.48 (1H, m), 7.00-7.05 (1H, m), 7.12 (1H, br), 7.22-7.33 (1H, m), 7.43-7.55 (1H, m), 9.82(1H, s) .
Elemental analysis value: as C₁₄H₁₅F₂NO₅S
Calculated (%): C,48.41; H,4.35; N,4.03.
Found (%) : C,48.47; H, 4.35; N,3.96

### Reference Example D7

In the same manner as in Reference Example D6, ethyl 5-(chlorosulfonyl)-3,6-dihydro-2H-pyran-4-carboxylate (0.70 g) obtained in Reference Example C8 was reacted with 2-chloro-4-fluoroaniline (0.52 g) to give ethyl 3-[(2-chloro-4-fluorophenyl)sulfamoyl]-3,6-dihydro-2H-pyran-4-carboxylate (compound 93; 0.54 g) as white crystals.
¹H-NMR(DMSO-d₆) δ: 1.11 (3H, t, J = 7.0 Hz), 3.72 (1H, dd, J = 12.8 Hz, 3.0 Hz), 4.07 (2H, q, J = 7.0 Hz), 4.15-4.25 (2H, m), 4.37 (1H, d, J = 2.2 Hz), 4.46-4.55 (1H, m), 7.15 (1H, br), 7.22-7.26 (1H, m), 7.46-7.59 (2H, m), 9.68 (1H, s).
Elemental analysis value: as C₁₉H₁₅ClFNO₅S
Calculated (%): C,46.22; H,4.16; N,3.85.
Found (%) : C,46.35; H,4.11; N,3.73.

Specific examples of the compound of the present invention that can be synthesized in the same manner as in the aforementioned Reference Examples are shown in Table 6 and Table 7. However, the present invention is not limited to the compounds exemplarily shown in Table 6 and Table 7.

**[Table 6]**

| | |
|---|---|
| 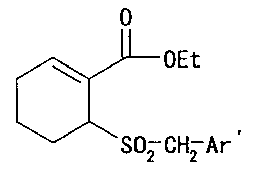 | |

| Comp.No. | Ar' |
|---|---|
| 86 | 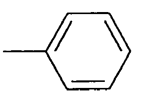 |
| 87 | 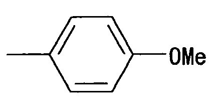 |
| 88 | 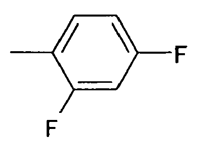 |
| 89 | 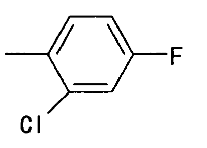 |
| 90 (-)-form | 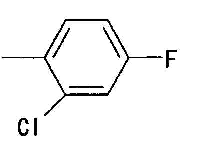 |
| 91 (+)-form | 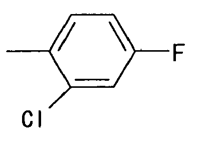 |

**[Table 7]**

| | |
|---|---|
| 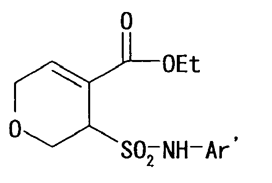 | |

| Comp.No. | Ar' |
|---|---|
| 92 | 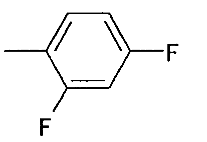 |
| 93 | 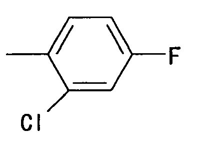 |

### Example 1

| | Formulation 1 | Comparative Formulation 2 |
|---|---|---|
| Compound 72 | 11g | 17g |
| Soybean Oil | 220g | 340g |
| egg yolk lecithin | 13.2g | 20.4g |
| glycerine | 24.7g | 38.25g |
| dimyristoylphosphatidylglycerol | 2.2g | 3.4g |
| acetic acid | 11.2mM | - |
| Anhydrous sodium acetate | 8.8mM | - |
| distilled water total amount | 1.1L | 1.7L |

11g of the compound 72 was dissolved in 220g of soybean oil, and 13.2g of egg yolk lecithin and 2.2g of dimyristoylphosphatidylglycerol were further dissolved. 24.7g of glycerine was dissolved in distilled water, and acetate buffering agent is mixed and dissolved so that the final concentrations are 11.2 mM of acetic acid and 8.8 mM of sodium acetate. These were mixed and roughly emulsified, and then finely emulsified at 8000 psi pressure by using a high pressure homogenizer. The obtained emulsion composition was filled in 20 mL vial by 20 mL. After processing a nitrogen displacement, the vial was tightly sealed with a rubber stopper and a plastic cap. This composition was sterilized in an autoclave at 121°C or above for not less than 15 min to give an emulsion composition having the aforementioned composition of the formulation 1.

Next, as a comparison, 17g of compound 72 was dissolved in 340g of soybean oil, and 20.4g of egg yolk lecithin and 3.4g of dimyristoylphosphatidylglycerol were further dissolved. 38.25g of glycerine was dissolved in distilled water. These were mixed and roughly emulsified, and then finely emulsified at 8000 psi pressure by using a high pressure homogenizer. The obtained emulsion composition was filled in 20 mL vial by 20 mL. After processing a nitrogen displacement, the composition was tightly sealed with a rubber stopper and a plastic cap. This composition was sterilized in an autoclave at 121°C or above for not less than 15 min to give an emulsion composition having the aforementioned composition of the comparative formulation 2.

### Example 2

The emulsion compositions of the formulation 1 and the comparative formulation 2 obtained in Example 1 were preserved at 25°C, and appearances, pH values and an average particle size thereof were measured in time intervals. The particle size was measured by Malvern Mastersizer S.

The results are shown in table 8.

In the formulation 1 containing an acetate buffering agent, pH value hardly changed before and after the sterilization and at the time of long term storage in a condition of 25°C, and the formulation 1 had stable appearances, pH value and average particle size. However, in the comparative formulation 2, pH value decreased from 4.6 to 4.0 before and after the sterilization, and further decreased to 3.6 when stored for 3 months at 25°C. According to the decrease in pH values, instability of the emulsion composition was occurred. When it was stored for 6 months at 25°C, the appearance became inappropriate since the disperse phase particle and water wherein the disperse phase particle is dispersed were phase separated, and thus the average particle size was increased to 0.8 µm. It became possible to improve a decrease in pH values at the time of sterilization or long term storage by adding an acetate buffering agent to the emulsion composition containing compound 72.

**[Table 8]**

| | | Appearance | pH | Average Particle size(µm) |
|---|---|---|---|---|
| | Before Sterilization | app¹⁾ | 4.4 | 0.3 |
| | Initial | app | 4.4 | 0.3 |
| Formulation 1 | 3 months at 25°C | app | 4.4 | 0.3 |
| | 6 months at 25°C | app | 4.5 | 0.3 |
| | Before Sterilization | app | 4.6 | 0.3 |
| Comparative Formulation 2 | Initial | app | 4.0 | 0.3 |
| | 3 months at 25°C | app | 3.6 | 0.3 |
| | 6 months at 25°C | inapp²⁾ | 3.6 | 0.8 |

| | | | | |
|---|---|---|---|---|
| app: appropriate inapp: inappropriate | | | | |

1) Free oil droplet, creaming and phase separation were not observed.
2) The disperse phase particle and the water in which the disperse phase particle is dispersed were phase separated.

**Example 3**

| | Formulation 3 | Comparative Formulation 4 |
|---|---|---|
| Compound 72 | 600 g | 600 g |
| Soybean Oil | 12000 g | 12000 g |
| egg yolk lecithin | 720 g | 720 g |
| glycerine | 1350 g | 1350 g |
| dimyristoylphosphatidylglycerol | 120 g | 120 g |
| glacial acetic acid | 40.35 g | - |
| sodium acetate trihydrate | 71.85 g | - |
| distilled water total amount | 60 L | 60 L |

600g of compound 72 was dissolved in 12kg of soybean oil, and 720g of purified egg yolk lecithin and 120g of dimyristoylphosphatidylglycerol were further dissolved at 50 to 60°C. 1350g of glycerine was dissolved in 20 kg of distilled water, and 40.35g of glacial acetic acid and 71.85g of sodium acetate trihydrate were mixed and dissolved at 50 to 60°C. These were then mixed, distilled water was added to total amount of 60 L and roughly emulsified by using a homogenizer Polytron for 5 min, and finely emulsified in conditions of 8000 psi pressure and 8-pass by using a high pressure homogenizer. The dissolution and emulsification of compound 72 were performed under a nitrogen gas stream. The obtained emulsion composition was passed through a membrane filter having a pore size of 4.5 µm, and filled in 20 mL vial by 20 mL. After processing a nitrogen displacement, the composition was tightly sealed with a rubber stopper and a plastic cap. This composition was sterilized in an autoclave at 121°C or above for not less than 15 min to give an emulsion composition formulation 3 having the aforementioned composition.

Next, as a comparison, 600g of compound 72 was dissolved in 12kg of soybean oil, and 720g of purified egg yolk lecithin and 120g of dimyristoylphosphatidylglycerol were further dissolved at 50 to 60°C. 1350g of glycerine was mixed and dissolved in 35 kg of distilled water at 50 to 60°C. These were then mixed, distilled water was added to total amount of 60 L and roughly emulsified by using a homogenizer Polytron for 5 min.

Next, they were finely emulsified in conditions of 8000 psi pressure and 8-pass by using the high pressure homogenizer. The dissolution and emulsification of compound 72 were performed under the nitrogen gas stream. The obtained emulsion composition was passed through a membrane filter having a pore size of 5 µm, and filled in 20 mL vial by 20 mL. After processing a nitrogen displacement, the composition was tightly sealed with a rubber stopper and a plastic cap. This composition was sterilized in an autoclave at 121°C or above for not less than 15 min to give a comparative emulsion composition formulation 4 having the aforementioned composition.

### Example 4

The emulsion compositions of the formulation 3 and the comparative formulation 4 which were obtained in Example 3 were stored at 25°C, and appearances, pH values and average particle size thereof were measured in time intervals. The average particle size of the formulation 3 was measured by Malvern Mastersizer 2000, and the average particle size of the comparative formulation 4 was measured by Malvern Mastersizer S.

The results are shown in table 9. In the formulation 3 containing an acetate buffering agent, pH value was stable before and after the emulsification-sterilization, and the pH value did not decrease even after a long term storage of 18 months at 25°C and also the appearance and the average particle size did not change, thus the formulation 3 was highly stable.

However, in the comparative formulation 4, the pH value decreased after a 3 months storage at 25°C, and the appearance was inappropriate since free oil-droplets became present on the surface of emulsion composition, and the average particle size increased to 3.6 µm after a 6 months storage at 25°C. Accordingly, an emulsion composition having a pH value not decreasing at the time of preparation, sterilization, and long term storage, was possibly obtained by a process of adding 20 mM of acetate buffering agent to the emulsion composition containing the compound 72. In this manner, it became possible to prepare emulsion compositions having a high stability.

**[Table 9]**

| | | Appearance | pH | Average Particle size (µm) |
|---|---|---|---|---|
| | before emulsification - sterilization | app¹⁾ | 4.5 | - |
| | Initial | app | 4.4 | 0.2 |
| | 3 months at 25°C | app | 4.5 | 0.2 |
| Formulation 3 | 6 months at 25°C | app | 4.5 | 0.2 |
| | 15 months at 25°C | app | 4.5 | 0.2 |
| | 18 months at 25°C | app | 4.5 | 0.2 |
| | 24 months at 25°C | app | 4.5 | 0.2 |
| Comparative Formulation 4 | before emulsification - sterilization | app | 5.2 | - |
| | Initial | app | 4.0 | 0.3 |
| | 3 months at 25°C | app | 3.5 | 0.3 |
| | 6 months at 25°C | inapp²⁾ | 3.5 | 3.6 |

| | | | | |
|---|---|---|---|---|
| app: appropriate inapp: inappropriate | | | | |

1) Free oil droplet, creaming and phase separation were not observed.
2) Free oil-droplet was visibly observed. Example 5

In order to prepare total volume of 25 ml formulations containing various types of buffering agents with a same ratio as the comparative formulation 2, buffering agents of various concentrations were added and dissolved in an aqueous phase composed of glycerine and distilled water. Compositions of each buffering agent are acetic acid and sodium acetate in the acetate buffering agent, lactic acid and sodium lactate in the lactate buffering agent, citric acid and sodium citrate in the citrate buffering agent, disodium hydrogenphosphate and citric acid in the phosphate-citrate buffering agent, monosodium dihydrogen phosphate and disodium hydrogenphosphate in the phosphate buffering agent, and sodium carbonate and sodium hydrogencarbonate in the carbonate buffering agent, and these were added with the concentrations described in tables 10 to 13. Next, the compound 72, egg yolk lecithin and dimyristoylphosphatidylglycerol were dissolved in soybean oil. These were mixed and roughly emulsified with a homogenizer, and then emulsion compositions were prepared by Sonicator. The obtained emulsion compositions were passed through a membrane filter having a pore size of 5 µm, and filled in 20 mL vial by 20 mL. After processing a nitrogen displacement, the compositions were tightly sealed with a rubber stopper and a plastic cap, and sterilized in an autoclave at 121°C or above for not less than 15 min to give emulsion compositions in which various types of buffering agents are to be added. Each of the pH values of the emulsion compositions was examined before processing in an autoclave and after the performance.

The results are shown in tables 10 to 13. By adding 5mM to 32mM of acetate buffering agent, lactate buffering agent, citrate buffering agent and phosphate-citrate buffering agent, to the emulsion compositions composed with the compound 72, emulsion compositions of pH 4 to 5 and having almost non pH changes according to a high-pressure steam sterilization were obtained.

Also, when the pH value of the emulsion compositions were adjusted to 6 by adding a liquid buffer, although phosphate buffering agent, carbonate buffering agent and citrate buffering agent were used, the pH value significantly decreased by the high-pressure steam sterilization. With the carbonate buffering agent, pH value changed to about 7 before the high-pressure steam sterilization.

**[Table 10]**

| pH value of emulsion composition | types of buffering agent | concentration of buffering agent | high-pressure steam sterilization | pH |
|---|---|---|---|---|
| 4 | acetate buffering agent | 5mM | before processing after processing | 4.1 |
| | | | | 4.1 |
| | | 10mM | before processing after processing | 4.0 |
| | | | | 4.0 |
| | | 15mM | before processing after processing | 4.0 |
| | | | | 4.0 |
| | | 20mM | before processing | 4.0 |
| | | | after processing | 4.0 |
| | | 5mM | before processing | 4.2 |
| | | | after processing | 4.1 |
| | lactate buffering | 12.5mM | before processing | 4.2 |
| | agent | | after processing | 4.1 |
| | | 25mM | before processing | 4.2 |
| | | | after processing | 4.1 |
| | | 5mM | before processing | 4.1 |
| | | | after processing | 4.1 |
| | citrate buffering agent | 10mM | before processing | 4.0 |
| | | | after processing | 4.1 |
| | | 15mM | before processing | 4.0 |
| | | | after processing | 4.1 |
| | | 20mM | before processing | 4.1 |
| | | | after processing | 4.2 |

**[Table 11]**

| pH value of emulsion composition | types of buffering agent | concentration of buffering agent | high-pressure steamsterilization | pH |
|---|---|---|---|---|
| 4.5 | | 5mM | before processing | 4.6 |
| | | | after processing | 4.5 |
| | acetate buffering agent | 10mM | before processing | 4.6 |
| | | | after processing | 4.4 |
| | | 15mM | before processing after processing | 4.5 4.4 |
| | | 20mM | before processing | 4.5 |
| | | | after processing | 4.4 |
| | | 5mM | before processing | 4.5 |
| | | | after processing | 4.4 |
| | lactate buffering | 12.5mM | before processing | 4.5 |
| | agent | | after processing | 4.4 |
| | | 25mM | before processing | 4.5 |
| | | | after processing | 4.4 |
| | phosphate -citrate | 8mM | before processing | 4.7 |
| | buffering | | after processing | 4.3 |
| | agent | 16mM | before processing | 4.6 |
| | | | after processing | 4.3 |
| | | 32mM | before processing | 4.6 |
| | | | after processing | 4.3 |
| | | 5mM | before processing | 4.6 |
| | | | after processing | 4.7 |
| | citrate buffering agent | 10mM | before processing | 4.6 |
| | | | after processing | 4.7 |
| | | 15mM | before processing | 4.5 |
| | | | after processing | 4.7 |
| | | 20mM | before processing | 4.5 |
| | | | after processing | 4.7 |

**[Table 12]**

| pH value of emulsion composition | types of buffering agent | concentration of buffering agent | high-pressure steam sterilization | pH |
|---|---|---|---|---|
| 5 | acetate buffering agent | 5mM | before processing | 5.0 |
| | | | after processing | 4.8 |
| | | 10mM | before processing | 5.0 |
| | | | after processing | 4.8 |
| | | 15mM | before processing | 4.9 |
| | | | after processing | 4.8 |
| | | 20mM | before processing | 5.0 |
| | | | after processing | 4.9 |
| | | 5mM | before processing | 5.1 |
| | | | after processing | 5.1 |
| | citrate buffering agent | 10mM | before processing | 5.1 |
| | | | after processing | 5.2 |
| | | 15mM | before processing | 5.1 |
| | | | after processing | 5.1 |
| | | 20mM | before processing | 5.0 |
| | | | after processing | 5.0 |

**[Table 13]**

| pH value of emulsion composition | types of buffering agent | concentration of buffering agent | high-pressure steam sterilization | pH |
|---|---|---|---|---|
| 6 | citrate buffering agent | 5mM | before processing | 6.3 |
| | | | after processing | 5.6 |
| | | 10mM | before processing | 6.3 |
| | | | after processing | 5.7 |
| | | 15mM | before | 6.3 |
| | | | processing | |
| | | | after processing | 5.7 |
| | | 20mM | before processing | 6.3 |
| | | | after processing | 5.6 |
| | | 5mM | before processing | 6.1 |
| | | | after processing | 4.6 |
| | phosphate buffering agent | 10mM | before processing | 6.1 |
| | | | after processing | 4.7 |
| | | 15mM | before processing | 6.0 |
| | | | after processing | 5.0 |
| | | 20mM | before processing | 6.0 |
| | | | after processing | 5.3 |
| | | 5mM | before processing | 7.0 |
| | | | after processing | 4.3 |
| | carbonate buffering agent | 10mM | before processing | 7.0 |
| | | | after processing | 4.3 |
| | | 15mM | before processing | 7.0 |
| | | | after processing | 5.0 |
| | | 20mM | before processing | 7.1 |
| | | | after processing | 5.6 |

### Example 6

| | Formulation 5 |
|---|---|
| Compound 72 | 500g |
| Soybean Oil | 10000g |
| egg yolk lecithin | 600g |
| glycerine | 1125g |
| dimyristoylphosphatidylglycerol | 100g |
| glacial acetic acid | 33.63g |
| sodium acetate trihydrate | 59.88g |
| distilled water total amount | 50L |

500g of compound 72 was dissolved in 10kg of soybean oil, and 600g of purified egg yolk lecithin and 100g of dimyristoylphosphatidylglycerol were further dissolved at 50 to 55°C. 1125g of glycerine was dissolved in about 10 kg of distilled water, and 33.63g of glacial acetic acid and 59.88g of sodium acetate trihydrate were mixed and dissolved at 50 to 55°C. These were then mixed, distilled water was added to total amount of 50 L, roughly emulsified, and finely emulsified in conditions of 8000 psi pressure and 8-pass by using a high pressure homogenizer. The dissolution and emulsification of compound 72 were performed under a nitrogen gas stream. The obtained emulsion composition was passed through a membrane filter having a pore size of 4.5 µm, and filled in 20 mL vial by 20 mL. After processing a nitrogen displacement, the composition was tightly sealed with a rubber stopper and a plastic cap. Similarly, the obtained emulsion composition was filled in 20 mL vial by 10 mL, in 20 mL vial by 5 mL, in 10 mL vial by 10 mL, and in 5 mL vial by 5 mL, and after processing a nitrogen displacement, each composition was tightly sealed with a rubber stopper and a plastic cap. These compositions were sterilized in an autoclave at 121°C or above for not less than 15 min to give emulsion compositions of formulation 5 having the aforementioned composition and different vial size and filling amount.

### Example 7

pH values and average particle sizes of the emulsion compositions of the formulation 5 obtained in Example 6 were measured before and after autoclave treatment. The particle size was measured by Malvern Mastersizer 2000.

The results are shown in table 14.

In the formulation 5 containing an acetate buffering agent, regardless of the vial size and filling amount, pH values were constant and the average particle sizes did not change before and after the sterilization, and thus the formulation 5 was very stable.

**Table 14**

| | Vial size | Filling amount | High-pressure steam sterilization | pH | Average Particle size (µm) |
|---|---|---|---|---|---|
| Formulation 5 | 20mL | 20mL | before processing | 4.6 | 0.2 |
| | | | after processing | 4.6 | 0.2 |
| | 20mL | 10mL | before processing | 4.6 | 0.2 |
| | | | after processing | 4.5 | 0.2 |
| | 20mL | 5mL | before processing | 4.6 | 0.2 |
| | | | after processing | 4.6 | 0.2 |
| | 10mL | 10mL | before processing | 4.6 | 0.2 |
| | | | after processing | 4.6 | 0.2 |
| | 5mL | 5mL | before processing | 4.6 | 0.2 |
| | | | after processing | 4.6 | 0.2 |

**Example 8**

| | Formulation 6 |
|---|---|
| Compound 72 | 5000g |
| Soybean Oil | 100000g |
| egg yolk lecithin | 6000g |
| glycerine | 11250g |
| dimyristoylphosphatidylglycerol | 1000g |
| glacial acetic acid | 336.3 g |
| sodium acetate trihydrate | 598.8g |
| distilled water total amount | 500L |

5000g of compound 72 was dissolved in 100kg of soybean oil, and 6000g of purified egg yolk lecithin and 1000g of dimyristoylphosphatidylglycerol were further dissolved at 50 to 55°C. 11250g of glycerine was dissolved in distilled water, and 336.3g of glacial acetic acid and 598.8g of sodium acetate trihydrate were mixed and dissolved at 50 to 55°C. These were then mixed, distilled water was added to total amount of 500 L, roughly emulsified, and finely emulsified in conditions of 8000 psi pressure and 6-pass by using a high pressure homogenizer. The dissolution and emulsification of compound 72 were performed under a nitrogen gas stream. The obtained emulsion composition was passed through a membrane filter having a pore size of 4.5 µm, and filled in 10 mL vial by 10 mL and in 10 mL vial by 5 mL. After processing a nitrogen displacement, the compositions were tightly sealed with a rubber stopper and a plastic cap. These compositions were sterilized in an autoclave at 121°C or above for not less than 15 min to give emulsion compositions of formulation 6 having the aforementioned composition and different filling amount.

### Example 9

pH values and average particle sizes of the emulsion compositions of the formulation 6 obtained in Example 8 were measured before and after autoclave treatment. The particle size was measured by Malvern Mastersizer 2000.

The results are shown in table 15.

In the formulation 6 containing an acetate buffering agent, regardless of the filling amount, pH values were constant and the average particle sizes did not change before and after the sterilization, and thus the formulation 6 was very stable.

**Table 15**

| | Vial size | Filling amount | High-pressure steam sterilization | pH | Average Particle size (µm) |
|---|---|---|---|---|---|
| Formulation 6 | 10mL | 10mL | before processing | 4.6 | 0.2 |
| | | | after processing | 4.5 | 0.2 |
| | 10mL | 5mL | before processing | 4.6 | 0.2 |
| | | | after processing | 4.5 | 0.2 |

### Comparative Example 1

In order to prepare total volume of 25ml formulations containing various concentrations of sodium hydroxide as a pH adjuster with a same ratio as the comparative formulation 2, it was respectively dissolved in an aqueous phase composed of glycerine and distilled water so as to make final concentrations of sodium hydroxide of 0, 0.5, 0.75, 1, 1.5 and 2.0 mM. Next, the compound 72, egg yolk lecithin and dimyristoylphosphatidylglycerol were dissolved in the soybean oil. These were mixed and roughly emulsified with a homogenizer, and then emulsion compositions were prepared by Sonicator. The obtained emulsion compositions were passed through a membrane filter having a pore size of 5 µm, and filled in 20 mL vial by 20 mL. After processing a nitrogen displacement, the compositions were tightly sealed with a rubber stopper and a plastic cap, and sterilized in an autoclave at 121°C or above for not less than 15 min to give emulsion compositions in which sodium hydroxide having various concentrations was added as the pH adjustor. Each of the pH values of the emulsion compositions was examined before processing in an autoclave and after the performance.

The results are shown in table 16. The emulsion compositions in which the compound 72 is added, or not added, both have a significantly lowered pH value after sterilization when sodium hydroxide of all concentrations was added.

**[Table 16]**

| Concentration of Sodium hydroxide | High-pressure steam sterilization | pH |
|---|---|---|
| 0 mM | before processing | 5.4 |
| | after processing | 4.6 |
| 0.5 mM | before processing | 6.5 |
| | after processing | 4.5 |
| 0.75 mM | before processing | 6.7 |
| | after processing | 4.5 |
| 1 mM | before processing | 6.7 |
| | after processing | 4.5 |
| 1.5 mM | before processing | 6.7 |
| | after processing | 4.6 |
| 2.0 mM | before processing | 6.9 |
| | after processing | 4.7 |

### Industrial Applicability

As is clear from the above-mentioned description, the emulsion composition of the present invention is adjusted to about pH 3.7 - about pH 5.5 by adding a buffering agent. Therefore, the pH of the emulsion composition and the average particle size of the disperse phase particles therein scarcely vary even after sterilization in an autoclave etc. or after a long-term preservation, and the composition is stable. Consequently, the emulsion composition of the present invention and a compound, a salt thereof or a prodrug thereof, which is the active ingredient of the emulsion composition, exhibit superior stability. Moreover, sterilization of the emulsion composition of the present invention in an autoclave etc. and a long-term preservation thereof do not result in the production of visually observable free oil drops. In other words, the disperse phase particle and water, in which the disperse phase particle has been dispersed, do not show phase separation and are stable.

In addition, since the emulsion composition of the present invention can be adjusted to pH about 3.7 - about 5.5 by adding a buffering agent to give a stable emulsion composition, an optimal pH can be determined depending on various other conditions such as the characteristics of the emulsifier and stability of the compound and the like. Therefore, even when other conditions that can be overcome by adjusting pH to about 3.7 - about 5.5 are present, such as the case where the stability of the emulsion composition is influenced, and the like, an emulsion composition satisfying various conditions can be afforded by adopting the adjustment of pH.

The emulsion composition of the present invention moreover shows long-term preservation stability for 24 months. This exceeds a general period of use of 18 months of emulsion preparations.

This application is based on a patent application No. 2005-131807 filed in Japan, the contents of which are hereby incorporated by reference.

## Claims

1. An emulsion composition comprising
(A) a compound stable in an acidic range, and
(B) a buffer,
wherein the pH is adjusted from about 3.7 to about 5.5.

2. The composition of Claim 1, which further comprises an anionic synthetic phospholipids as an emulsifier.

3. The composition of any of Claim 1 or 2, wherein (A) a compound stable in an acidic range is
(a) a compound of the formula: 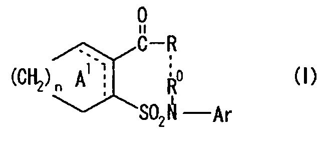 wherein R represents
an optionally substituted aliphatic hydrocarbon group,
an optionally substituted aromatic hydrocarbon group,
an optionally substituted heterocyclic group,
a group represented by the formula -OR¹
where R¹ represents a hydrogen atom or an optionally substituted aliphatic hydrocarbon group,
or a group represented by the formula: 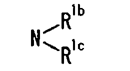 where R^{1b} represents a hydrogen atom or an optionally substituted aliphatic hydrocarbon group, and R^{1c} is same or different from R1b and represents a hydrogen atom or an optionally substituted aliphatic hydrocarbon group,
R° represents a hydrogen atom or an optionally substituted aliphatic hydrocarbon group,
or taken together, R and R° represent a bond,
ring A¹ is a cycloalkene optionally substituted by 1 to 4 substituents selected from the group consisting of
(1) an aliphatic hydrocarbon group optionally having substituents,
(2) an aromatic hydrocarbon group optionally having substituents,
(3) a group represented by the formula -OR¹
where R¹ represents a hydrogen atom or an aliphatic hydrocarbon group optionally having substituents, and
(4) a halogen atom,
Ar represents an optionally substituted aromatic hydrocarbon group, and
a group represented by the formula 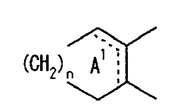 is a group represented by the formula 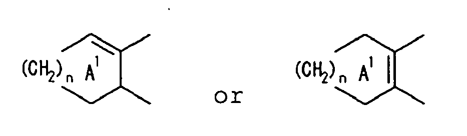 n represents an integer of 1 to 4,
or
(b) a compound of the formula: 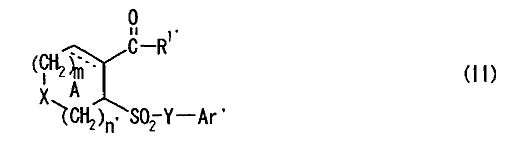
wherein R¹' represents
an optionally substituted aliphatic hydrocarbon group,
an optionally substituted aromatic hydrocarbon group,
an optionally substituted heterocyclic group,
a group represented by the formula: OR^{1a}'
where R^{1a'} represents a hydrogen atom or an optionally substituted aliphatic hydrocarbon group,
or a group represented by the formula: 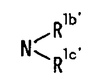 where R^{1b'} and R^{1c'} are the same or different from each other and represent a hydrogen atom or an optionally substituted aliphatic hydrocarbon group,
X represents a methylene group, a nitrogen atom, a 205 sulfur atom, or an oxygen atom,
Y represents an optionally substituted methylene group, or an optionally substituted nitrogen atom,
ring A is a 5 to 8 membered ring optionally further substituted by 1 to 4 substituents selected from the group consisting of
(1) an aliphatic hydrocarbon group optionally having substituents,
(2) an aromatic hydrocarbon group optionally having substituents,
(3) a group represented by the formula: OR^{2'}
where R²' represents a hydrogen atom or an aliphatic hydrocarbon group optionally having substituents, and
(4) a halogen atom,
Ar' represents an optionally substituted aromatic hydrocarbon group, and
a group represented by the formula 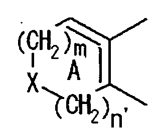 is a group represented by the formula 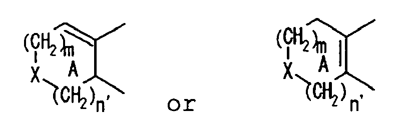 m represents an integer of 0 to 2,
n' represents an integer of 1 to 3, and
sum of m and n' is 4 or less,
provided that when X is a methylene group, Y represents an optionally substituted methylene group,
or
a salt thereof or a prodrug thereof.

4. The composition of Claim 3, wherein the compound represented by the formula (I) is selected from the group consisting of (6R)-6-[N-(2-chloro-4-fluorophenyl)sulfamoyl]cyclohex-1-en-1-carboxylate, d-ethyl 6-[N-(2,4-difluorophenyl)sulfamoyl]cyclohex-1-en-1-carboxylate, ethyl 6-[N-(2-chlorophenyl)sulfamoyl]cyclohex-1-en-1-carboxylate or ethyl 6-[N-(2-chloro-4-methylphenyl)sulfamoyl]cyclohex-1-en-1-carboxylate.

5. The composition of any of Claims 1 and 2, wherein the buffer is 1, 2 or more buffers selected from the group consisting of acetate buffer, lactate buffer, citrate buffer, and phosphate buffer.

6. The composition of Claim 5, wherein the acetate buffer is acetic acid and sodium acetate.

7. The emulsion composition of any of Claims 1 and 2, wherein the buffer concentration is 100mM or less.

8. The composition of Claim 1, which further comprises a component selected from the group consisting of oil, emulsifier, water and a combination thereof.

9. The composition of Claim 8, which is an oil-in-water type.

10. The composition of any of Claim 1 or 2, which comprises a disperse phase particle comprising a compound stable in an acidic range, oil, and emulsifier, and water wherein the disperse phase particle is dispersed.

11. The composition of Claim 10, wherein the disperse phase particle has an average particle size of about 0.025 to about 0.7 µm.

12. The composition of Claim 10, wherein the disperse phase particle and water wherein the disperse phase particle is dispersed are not phase separated and are stable.

13. The composition of Claim 9, which does not contain visibly identifiable free oil-droplet.

14. The composition of Claim 8, wherein the oil is a vegetable oil.

15. The composition of Claim 14, wherein the vegetable oil is soybean oil.

16. The composition of Claim 8, wherein the emulsifier is phospholipid.

17. The composition of Claim 16, wherein the phospholipid is egg yolk lecithin or phosphatidylglycerol.

18. The composition of Claim 2, wherein the anionic synthetic phospholipids is phosphatidylglycerol.

19. The composition of Claim 8, wherein the oil is in a proportion of about 1 to about 30 weight percent of the composition in total.

20. The composition of Claim 8, wherein the emulsifier is in a proportion of about 0.1 to 10 % (W/V) of the composition in total.

21. The composition of any of Claim 17 or 18, wherein phosphatidylglycerol is dimyristoylphosphatidylglycerol.

22. The composition of Claim 8, which further comprises a soybean oil, egg yolk lecithin, glycerine, and water.

23. The composition of any of Claims 1 and 2, which is for an injection.

24. The composition of any of Claims 1 and 2, wherein the compound stable in an acidic region is in a proportion of about 0.001 to about 95 weight percent of the composition in total.

25. A preparation method of an emulsion composition comprising (A) a compound stable in an acidic range and (B) a buffer, which comprises the step of adjusting pH of the emulsion composition to the range of about 3.7 to about 5.5.

26. The preparation method of Claim 25, wherein the emulsion composition further comprises an anionic synthetic phospholipid as an emulsifier.

27. The preparation method of Claim 25, whereby stability of pH of the emulsion composition and particle size of the disperse phase particle during autoclave sterilization is improved.

28. A method for long-term stabilizing an emulsion composition, wherein the emulsion composition comprises (A) a compound stable in an acidic range, and (B) a buffer, the method comprises the step of adjusting pH of the emulsion composition to the range of about 3.7 to about 5.5.

29. The method of Claim 28, which further comprises the step of adding an anionic synthetic phospholipids as an emulsifier to the emulsion composition.
